# EUROPEAN PATENT SPECIFICATION

(11) **EP 0 934 422 B1**
(45) Date of publication and mention of the grant of the patent: **06.12.2006**
(21) Application number: 97940830.9
(22) Date of filing: 05.09.1997
(51) Int. Cl.: C12N 15/86, A61K 48/00, C12N 9/88

(54) **GENE THERAPY FOR CONGESTIVE HEART FAILURE**
GENTHERAPIE FÜR KONGESTIVES HERZVERSAGEN
THERAPIE GENIQUE UTILE POUR TRAITER L'INSUFFISANCE CARDIAQUE GLOBALE

(30) Priority: 05.09.1996 US 708661; 16.06.1997 US 48933 P
(43) Date of publication of application: 11.08.1999
(73) Proprietor: The Regents of the University of California, Oakland, CA 94607-5200 (US); Collateral Therapeutics, San Diego, CA 92130 (US)
(72) Inventor: HAMMOND, H., Kirk, La Jolla, CA 92037 (US); INSEL, Paul, A., San Diego, CA 92122 (US); PING, Peipei, Louisville, KY 40292 (US); POST, Steven, R., San Diego, CA 92117 (US); GAO, Meihua, San Diego, CA 92121 (US)
(74) Representative: Campbell, Patrick John Henry
(86) International application number: PCT/US1997/015610
(87) International publication number: WO 1998/010085

(56) References cited:
- EP-A- 0 543 137
- WO-A-96/08260
- WO-A-97/16169
- WO-A-97/17937
- PING. P. ET AL.: "Over-expression of adenylylcyclase VI (ACVI) increases beta-adrenergic receptor-stimulated cAMP in neonatal rat cardiac myocytes" CIRCULATION, vol. 92, no. 8, 15 October 1995, page 2726 XP002056507
- NABEL E G: "GENE THERAPY FOR CARDIOVASCULAR DISEASE" CIRCULATION, vol. 91, no. 2, 15 January 1995, pages 541-548, XP000568740
- PING, P. ET AL.: "Reduced beta-adrenergic receptor activation decreases G-protein expression and beta-adrenergic receptor kinase activity in porcine heart" THE JOURNAL OF CLINICAL INVESTIGATION, vol. 95, no. 3, March 1995, pages 1271-1280, XP002056508
- SUMMERS, R. J. ET AL.: "Signalling pathways in cardiac failure" CLINICAL AND EXPERIMENTAL PHARMACOLGY AND PHYSIOLOGY, vol. 22, no. 1, 1985, pages 874-876, XP002056509
- KATSUSHIKA, S. ET AL.: "Cloning and characterization of a sixth adenylyl cyclase isoform: type V and VI constitute a subgroup within the mammalian adenylyl cyclase family" PROCEEDINGS OF THE NATIONAL ACADEMY OF SCIENCES OF USA., vol. 89, September 1992, WASHINGTON US, pages 8774-8778, XP002056510
- KRUPINSKI J ET AL: "MOLECULAR DIVERSITY IN THE ADENYLYLCYCLASE FAMILY EVIDENCE FOR EIGHT FROMS OF THE ENZYME AND CLONING OF TYPE VI" JOURNAL OF BIOLOGICAL CHEMISTRY, vol. 267, no. 34, 5 December 1992, pages 24858-24862, XP002007673
- GAO, M. ET AL.: "Increased adrenergic signaling after adenylylcyclase type VI gene transfer in rat cardiac myocytes" CIRCULATION, vol. 6, no. 8 sup, 21 October 1997, page I-294 XP002056511
- OPPERMAN M. ET AL: 'Monoclonal antibodies reveal receptor specificity among G-protein-coupled receptor kinases.' PROC. NATL. ACAD. SCI. vol. 93, July 1996, pages 7649 - 7654
- KYPSON A.P. ET AL: 'Global overexpression of beta-adrenergic receptors in the transplanted rat heart via adenoviral-mediated gene transfer.' SURGICAL FORUM vol. 47, 1996, pages 282 - 284
- MILANO ET AL: 'Enhanced Myocardial Function in Transgenic Mice Overexpressing the beta-2-Adrenergic Receptor' SCIENCE vol. 264, 22 April 1994, pages 582 - 586

### SEQUENCE LISTING

<110> The Regents of the University of California
<120> GENE THERAPY FOR CONGESTIVE HEART FAILURE
<130> N.74554A PJC
<140> EP 97940830.9
   <141> 1997-09-05
<150> PCT/US97/15610
   <151> 1997-09-05
<150> US 08/708,661
   <151> 1996-09-05
<150> US 60/048,933
   <151> 1997-06-16
<160> 7
<170> FastSEQ for Windows Version 4.0
<210> 1
   <211> 314
   <212> DNA
   <213> Homo sapiens
<220>
   <221> misc_feature
   <222> 229
   <223> n = A,T,C or G
<400> 1
   atg tca tgg ttt agt ggc ctc ctg gtc cct aaa gtg gat gaa cgg aaa    48
   aca gcc tgg ggt gaa cgc aat ggg cag aag cgt tcg cgg cgc cgt ggc    96
   act cgg gca ggt ggc ttc tgc acg ccc cgc tat atg agc tgc ctc cgg   144
   gat gca gag cca ccc agc ccc acc cct gcg ggc ccc cct cgg tgc ccc   192
   tgg cag gat gac gcc ttc atc cgg agg ggc ggc cca ngc aag ggc aag   240
   gaa ctg ggg ctg cgg gca gtg gcc ctg ggc ttc gaa gat acc gaa gtg   288
   aca acg aca ccg gcg gga ccg ctg aa                                314
<210> 2
   <211> 104
   <212> PRT
   <213> Homo sapiens
<220>
   <221> VARIANT
   <222> 77
   <223> Xaa = Any Amino Acid
<400> 2
   Met Ser Trp Phe Ser Gly Leu Leu Val Pro Lys Val Asp Glu Arg Lys
    1               5                  10                  15
   Thr Ala Trp Gly Glu Arg Asn Gly Gln Lys Arg Ser Arg Arg Arg Gly
               20                  25                  30
   Thr Arg Ala Gly Gly Phe Cys Thr Pro Arg Tyr Met Ser Cys Leu Arg
           35                  40                  45
   Asp Ala Glu Pro Pro Ser Pro Thr Pro Ala Gly Pro Pro Arg Cys Pro
       50                  55                  60
   Trp Gln Asp Asp Ala Phe Ile Arg Arg Gly Gly Pro Xaa Lys Gly Lys
   65                  70                  75                  80
   Glu Leu Gly Leu Arg Ala Val Ala Leu Gly Phe Glu Asp Thr Glu Val
                   85                  90                  95
   Thr Thr Thr Pro Ala Gly Pro Leu
               100
<210> 3
   <211> 28
   <212> DNA
   <213> Artificial Sequence
<220>
   <223> PCR sense primer
<400> 3
   acgtagaatt cggrgaytgt taytactg 28
<210> 4
   <211> 29
   <212> DNA
   <213> Artificial Sequence
<220>
   <223> PCR antisense primer
<400> 4
   acgttaagct tccasacrtc raaytgcca 29
<210> 5
   <211> 11
   <212> RNA
   <213> Artificial Sequence
<220>
   <223> mRNA destabilizing element
<400> 5
   uuauuuauau a 11
<210> 6
   <211> 1812
   <212> DNA
   <213> Homo sapiens
<400> 6
   gtt aac gtg gtg ctg ggc atc ctg gcg gca gtg cag gtc ggg ggc gct 48
   ttc gca gca gac ccg cgc agc ccc tct gcg ggc ctc tgg tgc cct gtg 96
   ttc ttt gta tac atc gca tac acg ctc ctc ccc atc cgc atg cgg gct 144
   gcc gtc ctc agc ggc ctg ggc ctc tcc acc ttg cat ttg atc ttg gcc 192
   tgg caa ctt aac cgt ggt gat gcc ttc ctc tgg aag cag ctc ggt gcc 240
   aat gtg ctg ctg ttc ctc tgc acc aac gtc att agc atc tgc aca cac 288
   tat cca gca gag gtg tct cag cgc cag gcc ttt cag gag acc cgc agt 336
   tac atc cag gcc cgg ctc cac ctg cag cat gag aat cgg cag cag gag 384
   cgg ctg ctg ctg tcg gta ttg ccc cag cac gtt gcc atg gag atg aaa 432
   gaa gac atc aac aca aaa aaa gaa gac atg ttc cac aag atc tac ata 480
   cag aag cat gac aat gtc agc atc ctg ttt gca gac att gag ggc ttc 528
   acc agc ctg gca tcc cag tgc act gcg cag gag ctg gtc atg acc ctg 576
   aat gag ctc ttt gcc cgg ttt gac aag ctg gct gcg gag aat cac tgc 624
   ctg agg atc aag atc ttg ggg gac tgt tac tac tgt gtg tca ggg ctg 672
   ccg gag gcc cgg gcc gac cat gcc cac tgc tgt gtg gag atg ggg gta 720
   gac atg att gag gcc atc tcg ctg gta cgt gag gtg aca ggt gtg aat 768
   gtg aac atg cgc gtg ggc atc cac agc ggg cgc gtg cac tgc ggc gtc 816
   ctt ggc ttg cgg aaa tgg cag ttc gat gtg tgg tcc aat gat gtg acc 864
   ctg gcc aac cac atg gaa gca gga agc cgg gct ggc cgc atc cac atc 912
   act cgg gca aca ctg cag tac ctg aac ggg gac tac gaa gtg gag cca 960
   ggc cgt ggt ggc aag cgc aac gcg tac ctc aag gag cag cac att gag 1008
   act ttc ctc atc ctg ggc gcc agc cag aaa cgg aaa gag gag aaa ggc 1056
   atg ctg gcc aag ctg cag cgg act cgg gcc aac tcc atg gaa ggg ctg 1104
   atg ccg cga tgg gtt cct gat cgt gcc ttc tcc cgg acc aag gac tcc 1152
   aag gcc ttc cgc cag atg ggc att gat gat tcc agc aaa gac aac cgg 1200
   ggc acc caa gat gcc ctg aac cct gag gat gag gtg gat gag ttc ctg 1248
   agc cgt gcc atc gat gcc cgc agc att gat cag ctg cgg aag gac cat 1296
   gtg cgc cgg ttt ttg ctc acc ttc cag aga gag gat ttt gag aag aag 1344
   tac tcc cgg aag gtg gat ccc cgc ttc gga gcc tac gtt gcc tgt gcc 1392
   ctg ttg gtc ttc tgc ttc atc tgc ttc atc cag ctt cta att ttc cca 1440
   cac tcc acc ctg atg ctt ggg att tat gcc agc atc ttc ctg ctg ctg 1488
   cta atc acc gtg ctg atc tgt gct gtg tac tcc tgt ggt tct ctg ttc 1536
   cct aag gcc ctg caa cgt ctg tcc cgc agc att gtc cgc tca cgg gca 1584
   cat agc acc gca gtt ggc atc ttt tcc gtc ctg ctt gtg ttt act tct 1632
   gcc att gcc aac atg ttc acc tgt aac cac acc ccc ata cgg agc tgt 1680
   gca gcc cgg atg ctg aat tta aca cct gct gac atc act gcc tgc cac 1728
   ctg cag cag ctc aat tac tct ctg ggc ctg gat gct ccc ctg tgt gag 1776
   ggc acc atg ccc acc tgc agc ttt cct gag gtg ttc 1812
<210> 7
   <211> 604
   <212> PRT
   <213> Homo sapiens
<400> 7
   Val Asn Val Val Leu Gly Ile Leu Ala Ala Val Gln Val Gly Gly Ala
    1               5                  10                  15
   Phe Ala Ala Asp Pro Arg Ser Pro Ser Ala Gly Leu Trp Cys Pro Val
               20                  25                  30
   Phe Phe Val Tyr Ile Ala Tyr Thr Leu Leu Pro Ile Arg Met Arg Ala
           35                  40                  45
   Ala Val Leu Ser Gly Leu Gly Leu Ser Thr Leu His Leu Ile Leu Ala
       50                  55                  60
   Trp Gln Leu Asn Arg Gly Asp Ala Phe Leu Trp Lys Gln Leu Gly Ala
   65                  70                  75                      80
   Asn Val Leu Leu Phe Leu Cys Thr Asn Val Ile Ser Ile Cys Thr His
                   85                  90                  95
   Tyr Pro Ala Glu Val Ser Gln Arg Gln Ala Phe Gln Glu Thr Arg Ser
               100                 105                 110
   Tyr Ile Gln Ala Arg Leu His Leu Gln His Glu Asn Arg Gln Gln Glu
           115                 120                 125
   Arg Leu Leu Leu Ser Val Leu Pro Gln His Val Ala Met Glu Met Lys
       130                 135                 140
   Glu Asp Ile Asn Thr Lys Lys Glu Asp Met Phe His Lys Ile Tyr Ile
   145                 150                 155                     160
   Gln Lys His Asp Asn Val Ser Ile Leu Phe Ala Asp Ile Glu Gly Phe
                   165                 170                 175
   Thr Ser Leu Ala Ser Gln Cys Thr Ala Gln Glu Leu Val Met Thr Leu
               180                 185                 190
   Asn Glu Leu Phe Ala Arg Phe Asp Lys Leu Ala Ala Glu Asn His Cys
           195                 200                 205
   Leu Arg Ile Lys Ile Leu Gly Asp Cys Tyr Tyr Cys Val Ser Gly Leu
       210                 215                 220
   Pro Glu Ala Arg Ala Asp His Ala His Cys Cys Val Glu Met Gly Val
   225                 230                     235                     240
   Asp Met Ile Glu Ala Ile Ser Leu Val Arg Glu Val Thr Gly Val Asn
                   245                     250                     255
   Val Asn Met Arg Val Gly Ile His Ser Gly Arg Val His Cys Gly Val
               260                     265                     270
   Leu Gly Leu Arg Lys Trp Gln Phe Asp Val Trp Ser Asn Asp Val Thr
           275                     280                     285
   Leu Ala Asn His Met Glu Ala Gly Ser Arg Ala Gly Arg Ile His Ile
       290                     295                     300
   Thr Arg Ala Thr Leu Gln Tyr Leu Asn Gly Asp Tyr Glu Val Glu Pro
   305                 310                     315                     320
   Gly Arg Gly Gly Lys Arg Asn Ala Tyr Leu Lys Glu Gln His Ile Glu
                   325                     330                     335
   Thr Phe Leu Ile Leu Gly Ala Ser Gln Lys Arg Lys Glu Glu Lys Gly
               340                     345                     350
   Met Leu Ala Lys Leu Gln Arg Thr Arg Ala Asn Ser Met Glu Gly Leu
           355                     360                     365
   Met Pro Arg Trp Val Pro Asp Arg Ala Phe Ser Arg Thr Lys Asp Ser
       370                     375                     380
   Lys Ala Phe Arg Gln Met Gly Ile Asp Asp Ser Ser Lys Asp Asn Arg
   385                 390                     395                     400
   Gly Thr Gln Asp Ala Leu Asn Pro Glu Asp Glu Val Asp Glu Phe Leu
                   405                     410                     415
   Ser Arg Ala Ile Asp Ala Arg Ser Ile Asp Gln Leu Arg Lys Asp His
               420                     425                     430
   Val Arg Arg Phe Leu Leu Thr Phe Gln Arg Glu Asp Phe Glu Lys Lys
           435                     440                     445
   Tyr Ser Arg Lys Val Asp Pro Arg Phe Gly Ala Tyr Val Ala Cys Ala
       450                     455                     460
   Leu Leu Val Phe Cys Phe Ile Cys Phe Ile Gln Leu Leu Ile Phe Pro
   465                 470                     475                     480
   His Ser Thr Leu Met Leu Gly Ile Tyr Ala Ser Ile Phe Leu Leu Leu
                   485                     490                     495
   Leu Ile Thr Val Leu Ile Cys Ala Val Tyr Ser Cys Gly Ser Leu Phe
               500                     505                     510
   Pro Lys Ala Leu Gln Arg Leu Ser Arg Ser Ile Val Arg Ser Arg Ala
           515                     520                     525
   His Ser Thr Ala Val Gly Ile Phe Ser Val Leu Leu Val Phe Thr Ser
       530                     535                     540
   Ala Ile Ala Asn Met Phe Thr Cys Asn His Thr Pro Ile Arg Ser Cys
   545                 550                     555                     560
   Ala Ala Arg Met Leu Asn Leu Thr Pro Ala Asp Ile Thr Ala Cys His
                   565                     570                     575
   Leu Gln Gln Leu Asn Tyr Ser Leu Gly Leu Asp Ala Pro Leu Cys Glu
               580                     585                     590
   Gly Thr Met Pro Thr Cys Ser Phe Pro Glu Val Phe
           595                     600

## Description

### Field of the Invention

The present invention relates to enhancing cardiac function in mammalian hearts. More specifically, the invention relates to techniques and polynucleotide constructs for enhancing myocardial β-adrenergic responsiveness using in *vivo* gene therapy.

### Background

It has been reported that 3-4 million adults in the United States have congestive heart failure (abbreviated "CHF" herein); and the incidence of CHF is increasing (see, e.g., Baughman, K., Cardiology Clinics 13: 27-34, 1995). Annually in US hospitals, CHF is the most frequent non-elective admission and the discharge diagnosis for 500,000 patients. Once symptoms of heart failure are moderately severe, the prognosis is worse than most cancers in that 50% of such patients are dead within 2 years (Braunwald, E. (ed), In: Heart Disease, W.B. Saunders, Philadelphia, page 471-485, 1988). Although medical therapy can initially attenuate the symptoms of heart failure (edema, breathlessness, fluid in the lungs), and in some cases prolong life, the prognosis in this disease, even with medical treatment, is grim (see, e.g., Baughman, K., Cardiology Clinics 13: 27-34, 1995).

CHF is defined as abnormal heart function resulting in inadequate cardiac output for metabolic needs (Braunwald, E. (ed), In: Heart Disease, W.B. Saunders, Philadelphia, page 426,1988). Symptoms include breathlessness, fatigue, weakness, leg swelling, and exercise intolerance. On physical examination, patients with heart failure tend to have elevations in heart and respiratory rates, rales (an indication of fluid in the lungs), edema, jugular venous distension, and, in general, enlarged hearts. The most common cause of CHF is atherosclerosis which causes blockages in the blood vessels (coronary arteries) that provide blood flow to the heart muscle. Ultimately such blockages may cause myocardial infarction (death of heart muscle) with subsequent decline in heart function and resultant heart failure. Other causes of CHF include valvular heart disease, hypertension, viral infections of the heart, alcohol, and diabetes. Some cases of heart failure occur without clear etiology and are called idiopathic.

CHF is also typically accompanied by alterations in one or more aspects of β-adrenergic neurohumoral function; see, e.g., Bristow MR, et al., N Engl J Med 307:205-211,1982; Bristow MR, et al., Circ Res 59:297-309, 1986; Ungerer M, et al., Circulation 87: 454-461, 1993; Feldman AM, et al., J Clin Invest 82:189-197, 1988; Bristow MR, et al., J Clin Invest 92: 2737-2745, 1993; Calderone A, et al., Circ Res 69:332-343. 1991; Marzo KP, et al., Circ Res 69:1546-1556, 1991; Liang C-S, et al., J Clin Invest 84: 1267-1275, 1989; Roth DA, et al., J Clin Invest 91: 939-949, 1993; Hadcock JR and Malbon CC: Proc Natl Acad Sci 85:5021-5025, 1988; Hadcock JR, et al., J Biol Chem 264: 19928-19933, 1989; Mahan, et al., Proc Natl Acad Sci USA 82:129-133, 1985; Hammond HK, et al., Circulation 85:269-280, 1992; Neumann J, et al., Lancet 2: 936-937, 1988; Urasawa K, et al., In: G Proteins: Signal Transduction and Disease, Academic Press, London. 44-85, 1992; Bohm M, Mol Cell Biochem, 147: 147-160, 1995; Eschenhage T, et al., Z Kardiol, 81 (Suppl 4): 33-40, 1992; and Yamamoto J, et al., J Mol Cell, 26: 617-626, 1994. See also the numerous additional references regarding various adenylylcyclase enzymes by, e.g., Fujita M et al., Circulation, 90: (No. 4 Part 2), 1994; Yoshimura M et al., Proc Natl Acad Sci USA, 89:6716-6720, 1992; Krupinski J et al., J Biol Chem, 267:24858-24862, 1992; Ishikawa Y et al., J Biol Chem, 267:13553-13557, 1992; Ishikawa Y et al., J. Clin Invest, 93:2224-2229, 1994; Katsushika S et al., Proc Natl Acad Sci USA, 89:8774-8778, 1992; Wallach J et al., FEBS Lett, 338:257-263, 1994; Watson PA et al., J Biol Chem, 269:28893-28898, 1994; Manolopoulos VG et al., Biochem Biophys Res Commun, 208:323-331, 1995; Yu HJ et al., FEBS Lett, 374:89-94, 1995; and Chen Z et al., J Biol Chem, 270:27525-27530, 1995.

As a result of these studies and others, efforts to treat CHF have focused on the administration of pharmacological agents, such as catecholamines and other β-adrenergic agonists, as means of stimulating β-adrenergic responses in dysfunctional hearts. Such therapeutic approaches have been only partly successful. Furthermore, long-term exposure to catecholamines can be detrimental. In particular, the heart tends to become less responsive to β-adrenergic stimulation, and such unresponsiveness is typically associated with high levels of catecholamines in plasma, a factor generally linked to a poor prognosis.

Present treatments for CHF include pharmacological therapies, coronary revascularization procedures (e.g. coronary artery bypass surgery and angioplasty), and heart transplantation. Pharmacological therapies have been directed toward increasing the force of contraction of the heart (by using inotropic agents such as digitalis and β-adrenergic receptor agonists), reducing fluid accumulation in the lungs and elsewhere (by using diuretics), and reducing the work of the heart (by using agents that decrease systemic vascular resistance such as angiotensin converting enzyme inhibitors). β-adrenergic receptor antagonists have also been tested. While such pharmacological agents can improve symptoms, and potentially prolong life, the prognosis in most cases remains dismal.

Some patients with heart failure due to associated coronary artery disease can benefit, at least temporarily, by revascularization procedures such as coronary artery bypass surgery and angioplasty. Such procedures are of potential benefit when the heart muscle is not dead but may be dysfunctional because of inadequate blood flow. If normal coronary blood flow is restored, viable dysfunctional myocardium may contract more normally, and heart function may improve. However, revascularization rarely restores cardiac function to normal or near-normal levels in patients with CHF, even though mild improvements are sometimes noted.

Finally, heart transplantation can be a suitable option for patients who have no other confounding diseases and are relatively young, but this is an option for only a small number of patients with heart failure, and only at great expense. In summary, CHF has a very poor prognosis and responds poorly to current therapies.

Further complicating the physiological conditions associated with CHF, are various natural adaptations that tend to occur in patients with dysfunctional hearts. Although these natural responses can initially improve heart function, they ultimately result in problems that can exacerbate CHF, confound treatment, and have adverse effects on survival. There are three such adaptive responses commonly observed in CHF: (i) volume retention induced by changes in sodium reabsorption, which expands plasma volume and initially improves cardiac output; (ii) cardiac enlargement (from dilation and hypertrophy) which can increase stroke volume while maintaining relatively normal wall tension; and (iii) increased norepinephrine release from adrenergic nerve terminals impinging on the heart which, by interacting with cardiac β-adrenergic receptors, tends to increase heart rate and force of contraction, thereby increasing cardiac output. However, each of these three natural adaptations tends ultimately to fail for various reasons. In particular, fluid retention tends to result in edema and retained fluid in the lungs that impairs breathing; heart enlargement can lead to deleterious left ventricular remodeling with subsequent severe dilation and increased wall tension, thus exacerbating CHF; and long-term exposure of the heart to norepinephrine tends to make the heart unresponsive to adrenergic stimulation and is linked with poor prognosis.

Controlled use of pharmacological agents, such as β-adrenergic agonists and other modulatory drugs, thus remains one of the major forms of treatment despite its shortfalls, including its potentially adverse effect on survival. Researchers who have analyzed and in some cases cloned DNA sequences encoding individual components involved in the β-adrenergic receptor pathway have proposed using such components to identify new classes of drugs that might prove more useful in treating CHF. For example, Ishikawa et al. cloned DNA encoding two different isoforms of adenylylcyclase (AC_{V} and AC_{VI}) that are known to be predominant in mammalian cardiac tissue, and proposed using the DNA and/or recombinant protein to identify new classes of drugs that might stimulate adrenergic pathways (See, e.g., American Cyanamid, WO 93/05061, 18 March 1993, and EP 0 529 662, 03 March 1993; and Ishikawa US Patent 5,334,521, issued 02 August 1994). In other reports in which cloned components of the adrenergic stimulation pathway were investigated, the authors generated transgenic mice overexpressing certain components (including cardiac β₂-adrenergic receptors, Gs_{α} and G-protein receptor kinase inhibitors) and obtained some data suggesting that β-adrenergic stimulation may be enhanced in transgenic mice (see, e.g., Gaudin C, et al., J Clin Invest 95: 1676-1683, 1995; Koch W J, et al., Science 268: 1350-1353, 1995; and Bond R A, et al., Nature 364: 272-276, 1995). None of these reports showed that cardiac function could be effectively restored in animals with heart failure, nor did they show that adrenergic responsiveness could be enhanced in large animal models that would be considered predictive of success in treating CHF in humans.

Indeed, reflecting on the observed difficulties associated with the clinical use of β-adrenergic agonists (such as dopamine and dobutamine), a recent review concluded that β-adrenergics stimulation appears to be harmful; and that, on the contrary, β-receptor "blockers" or antagonists may be more useful for improving morbidity and mortality rates (see, e.g., Baughman, K., Cardiology Clinics 13: 27-34, 1995). While some agents may improve symptoms, the prognosis for patients receiving such pharmacological agents remains dismal.

Ping *et al.,* (1995) Circulation 92, 8, Supplement I, Abstract 2727 reports that over-expression of adenylylcyclase VI (AC_{VI}) increases β-adrenergic receptor-stimulated cAMP in neonatal rat cardiac myocytes.

WO 96/08260 describes a mutant activated AC2 for use as a therapeutic agent.

EP-A-0543134 describes the cloning and characterisation of a cardiac AC.

The invention described and claimed herein addresses and overcomes problems associated with the prior art by providing techniques by which cardiac function can be effectively enhanced *in vivo* without the administration of β-adrenergic-agonist drugs.

### BRIEF DESCRIPTION OF THE FIGURES

**FIGURE 1A** shows a schematic of the construction of an exemplary replication-defective recombinant adenovirus vector useful for gene transfer into cells and into the heart, as described in Example 5-1 below.
**FIGURE 1B** shows a schematic of a clone used in the construction of an exemplary replication-defective recombinant adenovirus vector useful for gene transfer into cells and into the heart, as described in Example 5-2 below.
**FIGURE 2** shows a schematic of the cell surface β-adrenergic-Gs-adenylylcyclase pathway. It is through this pathway that β-adrenergic stimulation increases intracellular cAMP thereby influencing heart rate responsiveness and force of contraction. The pathway includes a β-adrenergic receptor, a stimulatory GTP-binding protein (Gs) linking receptor occupation with cAMP production, and an adenylylcyclase, as described in more detail below.
**FIGURE 3** shows data from experiments using forskolin-stimulated cAMP production to assess the function of adenylylcyclase in left ventricular membranes from normal pigs and from pigs with severe heart failure, using a model of heart failure with very high fidelity to human clinical dilated heart failure, as described in Example 3.
**FIGURE 4** shows data indicating that AC content sets a limit upon βAR-mediated signal transduction in cardiac myocytes, as described in Example 8-1.
**FIGURE 5** shows data indicating that gene transfer of an AC_{VI} transgene to cultured neonatal rat ventricular myocytes increased the levels of cAMP obtained after stimulation with either isoproterenol (10 µM) or forskolin (3 µM), as described in Example 8-1.
**FIGURE 6** shows data from a Northern analysis indicating the presence of transgene mRNA in cardiac myocytes, as described in Example 8-2.
**FIGURE 7** shows data from a Western analysis indicating the presence of transgene protein in cardiac myocytes, as described in Example 8-2.
**FIGURE 8A** shows data from a forskolin binding study indicating that net GTPγ-stimulated forskolin binding was increased after AC_{VI} gene transfer (data are mean values from three experiments), as described in Example 8-2.
**FIGURE 8B** shows data from a cAMP production study indicating that cardiac myocytes expressing transgene AC_{VI} have increased adrenergic responsiveness not only to forskolin stimulation, reflecting increased amounts of AC, but to isoproterenol, suggesting that newly synthesized AC is functionally coupled and recruitable through β-AR stimulation, a described in Example 8-2. Shown are mean values from three experiments.
**FIGURE 8C** shows the observed relationship between AC_{VI} content and cAMP production, as described in Example 8-2. The graph displays three measure of altered adrenergic signaling (forskolin binding, and isoproterenol- and forskolin-stimulated cAMP production). These data indicate that a proportional increase in AC content and enhanced adrenergic signaling has occurred.
**FIGURE 9** shows the results of an isoproterenol stimulation study as described in Example 8-2. Neonatal rat cardiac myocytes underwent gene transfer using recombinant adenovirus expressing lacZ or AC_{VI}. After gene transfer with AC_{VI} (vs lacZ), there is an obvious increase in cAMP produced through a wide range of isoproterenol concentrations. The EC50 for isoproterenol-stimulated cAMP production was unchanged.
**FIGURE 10** shows data summarizing the effects of *in* vivo gene transfer of AC_{VI} on heart rate in pigs, as described in Example 13. These data demonstrate, for the first time, that *in vivo* gene transfer can effectively increase adrenergic responsiveness in a large mammal heart.
**FIGURE 11** shows results of in *vivo* gene transfer of AC_{VI} on left ventricular (LV) dP/dt in a normal pig, as described in Example 13. These data further demonstrate that *in vivo* gene transfer of an adrenergic signaling element (in this case AC_{VI}) can effectively enhance contractile function of the intact heart in a large animal model that is considered highly predictive of human cardiac function.
**FIGURE 12A** shows nucleotide sequence from human AC_{VI} as described in Example 5-3. The -X- indicates a gap of about 0.5 kb within the sequence shown.
**FIGURE 12B** shows the amino acid sequence which corresponds to the nucleotide sequence shown in FIG. 12A, as described in Example 5-3.

### SUMMARY OF THE INVENTION

The invention provides the use of a vector comprising a gene encoding an adenylylcyclase (AC) operably linked to a promoter, for the manufacture of a medicament for enhancing cardiac function in a mammal with congestive heart failure by delivering said vector to the heart of said mammal. Preferably, the vector is introduced into a blood vessel supplying blood to the myocardium of the heart, so as to deliver the vector to cardiac myocytes; more preferably the vector is introduced into the lumen of a coronary artery, a saphenous vein graft, or an internal mammary artery graft. Most preferably, the vector is introduced into the lumen of both the left and right coronary arteries. Preferably, the mammal is a human.

In preferred embodiments, the vector comprises a gene encoding a β-adrenergic signalling protein (β-ASP) different from said AC operably linked to a promoter. Alternatively, a second vector comprising a gene encoding a β-ASP operably linked to a promoter is introduced into said mammal, said β-ASP being different from said AC.

In one preferred embodiment described herein, the vector comprises a gene encoding a cardiac AC such as AC isoform II, AC isoform V or AC isoform VI, more preferably AC isoform VI.

In preferred embodiments, the vector comprises a gene encoding the AC operably linked to a heterologous constitutive promoter or a heterologous inducible promoter. A preferred heterologous constitutive promoter is a CMV promoter which also includes an enhancer. In other preferred embodiments described herein, the promoter is a tissue-specific promoter, preferably a cardiac-specific promoter, more preferably, a ventricular myocyte-specific promoter. Preferred examples of ventricular myocyte-specific promoters include a ventricular myosin light chain 2 promoter and a ventricular myosin heavy chain promoter. The gene encoding the AC can also be operably linked to a heterologous enhancer, such as the CMV enhancer. Preferably, the gene encoding the AC is also operably linked to a polyadenylation signal.

In preferred embodiments, the vector is a viral vector or a lipid-based vector, preferably a viral vector. The vector can be a targeted vector, especially a targeted vector that preferentially binds to ventricular myocytes. Presently preferred viral vectors are derived from adenovirus (Ad) or adeno-associated virus (AAV). Both human and non-human viral vectors can be used but preferably the recombinant viral vector is replication-defective in humans. Where the vector is an adenovirus, it preferably comprises a polynucleotide having a promoter operably linked to a gene encoding an adenylylcyclase, and is replication-defective in humans. Presently preferred replication-defective adenoviral vector have deletions that remove the E1A and E1B genes, or have deletions that remove the E1A, E1B and E4 genes. Preferably about 10⁷ to 10¹³ adenovirus vector particles, more preferably about 10⁹ to 10¹² vector particles, are introduced into a blood vessel, preferably a blood vessel supplying the myocardium as described above. For AAV vectors, the vector preferably comprises a polynucleotide having a promoter operably linked to a gene encoding an adenylylcyclase and, preferably, the gene encoding an AC is flanked by AAV inverted terminal repeats (ITRs). Preferably, the AAV vector is replication-defective in humans. Presently preferred replication-defective AAV vectors have deletions affecting one or more AAV replication or encapsidation sequences. Alternatively, the vector can be a lipid-based vector comprising a gene encoding an adenylylcyclase as described herein. In performing the invention, the following may be used:

A recombinant replication-defective viral particle comprising a gene encoding an adenylylcyclase operably linked to a promoter. Preferably, the promoter is a heterologous constitutive or inducible promoter. The vector can also comprise a gene encoding another β-ASP. Preferred viral vectors are derived from adenovirus (Ad) or adeno-associated virus (AAV). Both human and non-human viral vectors can be used but preferably the recombinant viral vector is replication-defective in humans. Where the vector is an adenovirus, it preferably comprises a polynucleotide having a promoter operably linked to a gene encoding an adenylylcyclase, and is replication-defective in humans. Presently preferred replication-defective adenoviral vector have deletions that remove the E1A and E1B genes, or have deletions that remove the E1A, E1B and E4 genes. For AAV vectors, the vector preferably comprises a polynucleotide having a promoter operably linked to a gene encoding an adenylylcyclase and, preferably, the gene encoding the AC is flanked by AAV inverted terminal repeats (ITRs). Preferably, the AAV vector is replication-defective in humans. Presently preferred replication-defective AAV vectors have deletions affecting one or more AAV replication or encapsidation sequences. Other vectors of the present invention include lipid-based vectors (such as liposomes) comprising a gene encoding an adenylylcyclase, as described herein.

A mammalian cell transfected with a recombinant replication-defective viral particle or other vector according to one of the preceding embodiments.

A filtered injectable adenovirus particle preparation comprising: (i) a recombinant replication-defective adenovirus particle as described above, and
(ii) a carrier. The carrier is preferably a pharmaceutically-acceptable carrier. Preferably the adenovirus vector has been filtered through a 0.1-0.5 micron filter.

A method of generating a recombinant replication-defective viral particle as described above, comprising the following steps in the order listed:
(i) introducing first and second plasmids into a replication-permissive mammalian cell expressing one or more adenovirus genes conferring replication competence, wherein said first plasmid comprises a gene encoding an adenylylcyclase operably linked to a promoter and further comprises a replication-defective adenovirus genome, and wherein said second plasmid comprises a replication-proficient adenovirus genome and further comprises an additional polynucleotide sequence making the second plasmid too large to be encapsidated in an adenovirus particle, whereby rescue recombination takes place between the first plasmid and the second plasmid to generate a recombinant adenoviral genome comprising the gene encoding the AC but lacking one or more adenoviral replication genes, said recombinant adenoviral genome being sufficiently small to be encapsidated in an adenovirus particle;
(ii) identifying successful recombinant viral vectors in cell culture; and
(iii) propagating a resulting recombinant viral particle in replication-permissive mammalian cells expressing the missing adenoviral replication genes to generate a recombinant replication-defective viral particle.

The introducing step can be accomplished by co-transfection of the first and second plasmids into the permissive mammalian cell. The method can also comprise, prior to said step of introducing first and second plasmids, the step of cloning a gene encoding an AC into a plasmid containing a promoter and partial adenovirus sequences of the left end of a replication-defective adenovirus genome such that the gene encoding the AC is operably linked to said promoter. Preferably the method further comprises, after said propagation step, the step of purifying the propagated viral particles, and preferably also includes filtering the purified viral particles through a 0.1-0.5 micron filter. An exemplary first plasmid as described above is plasmid pAC1 or plasmid ACCMVPLPA comprising a gene encoding an AC. The identification step described above preferably comprises the steps of: (i) monitoring transfected cells for evidence of cytopathic effect; (ii) isolating viral nucleic acid from the cell supernatant of cultures of the transfected cells showing a cytopathic effecttreating the cell supernatant from cell cultures showing a cytopathic effect with a proteinase (such as proteinase K), followed by phenol/chloroform extraction and ethanol precipitation; (iii) identifying successful recombinants with PCR using primers complementary to the promoter operably linked to the AC gene and primers complementary to adenovirus sequences; and (iv) purifying the recombinant viral particles by plaque purification (preferably for at least two rounds). Viral nucleic acid can be isolated by treating the cell culture supernatant suspected of containing recombinant viral particles with a proteinase (such as proteinase K), followed by phenol/chloroform extraction of the proteinase-treated supernatant to remove proteins, and finally, ethanol precipitation of the lysate to obtain viral DNA.

The purification step as described above preferably comprises the steps of: (i) propagating the resulting recombinants in cells transformed with the replication competence conferring genes to titers in the 10¹⁰-10¹² viral particles range; and (ii) purifying the propagated recombinants (preferably by double CsCl gradient ultracentrifugation).

A recombinant pro-viral plasmid comprising a gene encoding an AC operably linked to a promoter and further comprising a replication-defective viral genome. Preferably, the AC is adenylylcyclase isoform VI. Exemplary replication-defective viral genomes include an adenovirus genome and an AAV genome. Where the recombinant replication-defective viral genome is an adenovirus genome, the adenovirus may be either a human or a non-human mammalian adenovirus (preferably non-human mammalian), but in either case is preferably replication-defective in humans. Preferably, the recombinant replication-defective adenovirus genome has deletions removing the E1A and E1B genes, or deletions removing the E1A, E1B and E4 genes. Where the recombinant replication-defective viral genome is an AAV genome, the AAV genome preferably has deletions affecting one or more AAV replication or encapsidation sequences.

A cell comprising a recombinant pro-viral plasmid according to one of the preceding embodiments.

### DETAILED DESCRIPTION OF PREFERRED EMBODIMENTS

### Definitions

A "polynucleotide" refers to a polymeric form of nucleotides of any length, either ribonucleotides or deoxyribonucleotides, or analogs thereof. This term refers to the primary structure of the molecule, and thus includes double- and single-stranded DNA, as well as double- and single-stranded RNA. It also includes modified polynucleotides such as methylated and/or capped polynucleotides.

"Recombinant," as applied to a polynucleotide, means that the polynucleotide is the product of various combinations of cloning, restriction and/or ligation steps, and other procedures that result in a construct that is distinct from a polynucleotide found in nature.

A "gene" refers to a polynucleotide or portion of a polynucleotide comprising a sequence that encodes a protein. For most situations, it is desirable for the gene to also comprise a promoter operably linked to the coding sequence in order to effectively promote transcription. Enhancers, repressors and other regulatory sequences may also be included in order to modulate activity of the gene, as is well known in the art. (See, e.g., the references cited below).

The terms "polypeptide," "peptide," and "protein" are used interchangeably to refer to polymers of amino acids of any length. These terms also include proteins that are post-translationally modified through reactions that include glycosylation, acetylation and phosphorylation.

A "heterologous" component refers to a component that is introduced into or produced within a different entity from that in which it is naturally located. For example, a polynucleotide derived from one organism and introduced by genetic engineering techniques into a different organism is a heterologous polynucleotide which, if expressed, can encode a heterologous polypeptide. Similarly, a promoter or enhancer that is removed from its native coding sequence and operably linked to a different coding sequence is a heterologous promoter or enhancer.

A "promoter," as used herein, refers to a polynucleotide sequence that controls transcription of a gene or coding sequence to which it is operably linked. A large number of promoters, including constitutive, inducible and repressible promoters, from a variety of different sources, are well known in the art and are available as or within cloned polynucleotide sequences (from, e.g., depositories such as the ATCC as well as other commercial or individual sources).

An "enhancer," as used herein, refers to a polynucleotide sequence that enhances transcription of a gene or coding sequence to which it is operably linked. A large number of enhancers, from a variety of different sources are well known in the art and available as or within cloned polynucleotide sequences (from, e.g., depositories such as the ATCC as well as other commercial or individual sources). A number of polynucleotides comprising promoter sequences (such as the commonly-used CMV promoter) also comprise enhancer sequences.

"Operably linked" refers to a juxtaposition, wherein the components so described are in a relationship permitting them to function in their intended manner. A promoter is operably linked to a coding sequence if the promoter controls transcription of the coding sequence. Although an operably linked promoter is generally located upstream of the coding sequence, it is not necessarily contiguous with it. An enhancer is operably linked to a coding sequence if the enhancer increases transcription of the coding sequence. Operably linked enhancers can be located upstream, within or downstream of coding sequences. A polyadenylation sequence is operably linked to a coding sequence if it is located at the downstream end of the coding sequence such that transcription proceeds through the coding sequence into the polyadenylation sequence.

A "replicon" refers to a polynucleotide comprising an origin of replication which allows for replication of the polynucleotide in an appropriate host cell. Examples include replicons of a target cell into which a heterologous nucleic acid might be integrated (e.g., nuclear and mitochondrial chromosomes), as well as extrachromosomal replicons (such as replicating plasmids and episomes).

"Gene delivery," "gene transfer," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of mediating transfer of genes to mammalian cells, as is known in the art and described herein.

*"In vivo"* gene delivery, gene transfer, gene therapy and the like as used herein, are terms referring to the introduction of a vector comprising an exogenous polynucleotide directly into the body of an organism, such as a human or non-human mammal, whereby the exogenous polynucleotide is introduced to a cell of such organism *in vivo.*

A "vector" (sometimes referred to as gene delivery or gene transfer "vehicle") refers to a macromolecule or complex of molecules comprising a polynucleotide to be delivered to a host cell, either *in vitro* or *in vivo.* The polynucleotide to be delivered may comprise a coding sequence of interest in gene therapy. Vectors include, for example, viral vectors (such as adenoviruses ("Ad"), adeno-associated viruses (AAV), and retroviruses), liposomes and other lipid-containing complexes, and other macromolecular complexes capable of mediating delivery of a polynucleotide to a host cell. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. A large variety of such vectors are known in the art and are generally available (see, e.g., the various references cited below).

A "recombinant viral vector" refers to a viral vector comprising one or more heterologous genes or sequences. Since many viral vectors exhibit size-constraints associated with packaging, the heterologous genes or sequences are typically introduced by replacing one or more portions of the viral genome. Such viruses may become replication-defective, requiring the deleted function(s) to be provided *in trans* during viral replication and encapsidation (by using, e.g., a helper virus or a packaging cell line carrying genes necessary for replication and/or encapsidation) (see, e.g., the references and illustrations below). Modified viral vectors in which a polynucleotide to be delivered is carried on the outside of the viral particle have also been described (see, e.g., Curiel, DT, et al. PNAS 88: 8850-8854, 1991).

Viral "packaging" as used herein refers to a series of intracellular events that results in the synthesis and assembly of a viral vector. Packaging typically involves the replication of the "pro-viral genome", or a recombinant pro-vector typically referred to as a "vector plasmid" (which is a recombinant polynucleotide than can be packaged in an manner analogous to a viral genome, typically as a result of being flanked by appropriate viral "packaging sequences"), followed by encapsidation or other coating of the nucleic acid. Thus, when a suitable vector plasmid is introduced into a packaging cell line under appropriate conditions, it can be replicated and assembled into a viral particle. Viral "rep" and "cap" genes, found in many viral genomes, are genes encoding replication and encapsidation proteins, respectively. A "replication-defective" or "replication-incompetent" viral vector refers to a viral vector in which one or more functions necessary for replication and/or packaging are missing or altered, rendering the viral vector incapable of initiating viral replication following uptake by a host cell. To produce stocks of such replication-defective viral vectors, the virus or pro-viral nucleic acid can be introduced into a "packaging cell line" that has been modified to contain genes encoding the missing functions which can be supplied *in trans*). For example, such packaging genes can be stably integrated into a replicon of the packaging cell line or they can be introduced by transfection with a "packaging plasmid" or helper virus carrying genes encoding the missing functions.

A "detectable marker gene" is a gene that allows cells carrying the gene to be specifically detected (e.g., distinguished from cells which do not carry the marker gene). A large variety of such marker genes are known in the art. Preferred examples thereof include detectable marker genes which encode proteins appearing on cellular surfaces, thereby facilitating simplified and rapid detection and/or cellular sorting. By way of illustration, the lacZ gene encoding beta-galactosidase can be used as a detectable marker, allowing cells transduced with a vector carrying the lacZ gene to be detected by staining, as described below.

A "selectable marker gene" is a gene that allows cells carrying the gene to be specifically selected for or against, in the presence of a corresponding selective agent. By way of illustration, an antibiotic resistance gene can be used as a positive selectable marker gene that allows a host cell to be positively selected for in the presence of the corresponding antibiotic. Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e. positive/negative) markers (see, e.g., WO 92/08796, published 29 May 1992, and WO 94/28143, published 8 December 1994). Such marker genes can provide an added measure of control that can be advantageous in gene therapy contexts.

"β-adrenergic signaling," as used herein, refers to β-adrenergic receptor-mediated signaling which is mediated via β-adrenergic receptors ("β-ARs") present on cellular surfaces. Of particular relevance in the context of the present invention are receptors present on the surface of β-adrenergic-stimulated cells in the myocardium of mammalian heart tissue. As described below, β-adrenergic signaling within myocardial tissue is initially mediated by agonist binding to β-AR, followed by Gₛ-mediated signal transduction to adenylylcyclase (AC). Activated AC then catalyzes the synthesis of cyclic AMP (cAMP), and increased intracellular concentrations of cAMP mediate increased cytosolic calcium transients which enhance both the rate and force of cardiac contraction (referred to as positive chronotrophy and positive inotrophy, respectively). Various β-adrenergic signaling proteins, and other factors affecting β-adrenergic signaling, are described in the art and are further illustrated herein.

A "β-adrenergic signaling protein" (sometimes abbreviated "β-ASP" herein) or "β-adrenergic signaling element" refers to a protein that is capable of enhancing β-adrenergic receptor-mediated signaling when expressed in mammalian tissue, preferably (for purposes of the present invention) when expressed in mammalian myocardial tissue. β-adrenergic signaling proteins thus include "β-adrenergic signal transducer" proteins that mediate or transduce β-adrenergic signaling, preferably in mammalian myocardial cells, as well as proteins which can either stimulate such transducer proteins or which can inactivate or compete with inhibitors of such transducer proteins (thereby indirectly enhencing signal transduction). A variety of such proteins that are associated with β-adrenergic receptor-mediated signaling in mammalian cardiac tissue have been identified (see, e.g., the various references regarding β-adrenergic responsiveness cited above) and are illustrated herein. Examples of β-ASPs are known to play a role in β-adrenergic receptor-mediated signal transduction in mammalian heart tissue, such as the various proteins associated with the "βAR-Gs-AC" pathway, comprising a β-adrenergic receptor ("βAR"), a Gₛ protein transducer and an adenylylcyclase ("AC") effector, as well as proteins enhancing the activity of such βAR-Gs-AC proteins, as described in more detail herein and in the cited art. Recent data have demonstrated that Gₛ protein is generally present at a much higher molar proportion than either βAR or AC. Particular examples of β-ASPs include: β-adrenergic receptors (such as β₁-adrenergic receptors or β₂-adrenergic receptors, more preferably β₁-adrenergic receptors), adenylylcyclases (preferably a cardiac AC such as AC_{V} or AC_{VI}, more preferably AC_{VI}); as well as inhibitors of the function of G-protein receptor kinases (which are generally referred to herein as "GRK" inhibitors).

"β-adrenergic receptors" (abbreviated "β-AR" or "βAR") are the cell-surface receptors involved in β-adrenergic receptor-mediated signaling via the βAR-Gs-AC pathway. Within the myocardium of a mammalian heart, βARs are the principal receptors for norepinephrine (the sympathetic neurotransmitter) and for epinephrine (the adrenal hormone). Human myocardium contains both β₁-adrenergic receptors and β₂-adrenergic receptors, but β₁-ARs are predominant and are most closely associated with the altered β-adrenergic signaling that is observed with heart failure, as described below.

"Gₛ protein" is a GTP-binding regulatory protein that effectively couples activation of a variety of cell-surface receptors (including β-adrenergic receptors) to the activation of adenylylcyclase, as described in the art and herein.

"Adenylylcyclase" (EC 4.6.1.1, also referred to as "adenylcyclase", "adenylate cyclase", and "cAMP synthetase") is an enzyme that catalyzes the conversion of adenosine triphosphate (ATP) to 3':5'-cyclic adenosine monophosphate (cAMP). Adenylylcyclase (abbreviated herein as "AC") is known to exist in a number of different isoforms that are found in varying levels in most all mammalian tissues. The most preferred adenylylcyclases useful in the present invention are "cardiac adenylylcyclases" which are isoforms found to be predominant in mammalian heart tissue, particularly in cardiac myocytes; as described in more detail below.

"G-protein receptor kinases" (abbreviated "GRK", but also referred to in the art as "β-adrenergic receptor kinases" or "βARK"), are kinase proteins that catalyze phosphorylation of G-protein-coupled receptor proteins including β-adrenergic receptors ("βARs"). Phosphorylation of βARs by GRK proteins leads to uncoupling of the receptors and a concomitant decrease in responsiveness to β-adrenergic signaling.

"GRK inhibitors," as used herein refer to proteins that inhibit the function of G-protein receptor kinases. Such inhibitors of GRK include modified GRK proteins in which receptor-binding activity has been uncoupled from kinase activity. Exemplary GRK inhibitors thus include modified GRKs that have been truncated (typically by deletions beginning at the amino-terminus) to remove kinase function while retaining the ability to bind to G-protein-coupled receptor proteins such as βARs. Such truncated GRK proteins can thus effectively compete with or prevent normal GRK from binding to βAR but do not cause subsequent inhibition of receptor activity (since they lack kinase activity).

"Vasculature" or "vascular" are terms referring to the system of vessels carrying blood (as well as lymph fluids) throughout the mammalian body.

"Blood vessel" refers to any of the vessels of the mammalian vascular system, including arteries, arterioles, capillaries, venules, veins, sinuses, and vasa vasorum.

"Artery" refers to a blood vessel through which blood passes away from the heart. Coronary arteries supply the tissues of the heart itself, while other arteries supply the remaining organs of the body. The general structure of an artery consists of a lumen surrounded by a multi-layered arterial wall.

An "individual" as used herein refers to a large mammal, most preferably a human.

"Treatment" or "therapy" as used herein refers to administering, to an individual patient, agents that are capable of eliciting a prophylactic, curative or other beneficial effect in the individual.

"Gene therapy" as used herein refers to administering, to an individual patient, vectors comprising a therapeutic gene.

A "therapeutic polynucleotide" or "therapeutic gene" refers to a nucleotide sequence that is capable, when transferred to an individual, of eliciting a prophylactic, curative or other beneficial effect in the individual.

### References

The practice of the present invention will employ, unless otherwise indicated, conventional techniques of molecular biology and the like, which are within the skill of the art. Such techniques are explained fully in the literature. See e.g., Molecular Cloning: A Laboratory Manual, (J. Sambrook et al., Cold Spring Harbor Laboratory, Cold Spring Harbor, N.Y., 1989); Current Protocols in Molecular Biology (F. Ausubel et al. eds., 1987 and updated); Essential Molecular Biology (T. Brown ed., IRL Press 1991); Gene Expression Technology (Goeddel ed., Academic Press 1991); Methods for Cloning and Analysis of Eukaryotic Genes (A. Bothwell et al. eds., Bartlett Publ. 1990); Gene Transfer and Expression (M. Kriegler, Stockton Press 1990); Recombinant DNA Methodology (R. Wu et al. eds., Academic Press 1989); PCR: A Practical Approach (M. McPherson et al., IRL Press at Oxford University Press 1991); Cell Culture for Biochemists (R. Adams ed., Elsevier Science Publishers 1990); Gene Transfer Vectors for Mammalian Cells (J. Miller & M. Calos eds., 1987); Mammalian Cell Biotechnology (M. Butler ed., 1991); Animal Cell Culture (J. Pollard et al. eds., Humana Press 1990); Culture of Animal Cells, 2nd Ed. (R. Freshney et al. eds., Alan R. Liss 1987); Flow Cytometry and Sorting (M. Melamed et al. eds., Wiley-Liss 1990); the series Methods in Enzymology (Academic Press, Inc.); Techniques in Immunocytochemistry, (G.Bullock & P. Petrusz eds., Academic Press 1982, 1983, 1985, 1989); Handbook of Experimental Immunology, (D. Weir & C. Blackwell, eds.); Cellular and Molecular Immunology (A. Abbas et al., W.B. Saunders Co. 1991, 1994); Current Protocols in Immunology (J. Coligan et al. eds. 1991); the series Annual Review of Immunology; the series Advances in Immunology; Oligonucleotide Synthesis (M. Gait ed., 1984); and Animal Cell Culture (R. Freshney ed., IRL Press 1987).

Additional references describing delivery and logistics of surgery which may be used in the present invention include the following: Topol, EJ (ed.), The Textbook of Interventional Cardiology, 2nd Ed. (W.B. Saunders Co. 1994); Rutherford, RB₃ Vascular Surgery, 3rd Ed. (W.B. Saunders Co. 1989); Wyngaarden JB et al. (eds.), The Cecil Textbook of Medicine, 19th Ed. (W.B. Saunders, 1992); and Sabiston, D, The Textbook of Surgery, 14th Ed. (W.B. Saunders Co. 1991).

Additional references describing cell types found in the blood vessels, and the structure of the vasculature which may be useful in the present invention include the following: W. Bloom & D. Fawcett, A Textbook of Histology, 10th Ed., (W.B. Saunders Co. 1975).

Various publications have postulated on the uses of gene transfer for the treatment or prevention of disease, including heart disease. See, e.g., Methods in Molecular Biology, Vol. 7: Gene Transfer and Expression Protocols, Murray, E. (ed.), Humana Press, Clifton, N.J. (1991); Mazur et al., Molecular and Cellular Pharmacology, 21:104-111, 1994; French, Herz 18:222-229, 1993; Williams, American Journal of Medical Sciences 306:129-136, 1993; and Schneider and French, Circulation 88:1937-1942, 1993.

Sources and structural/functional features of vectors and of various AC proteins that could be used in the present invention are provided in the various reports as cited throughout this specification, and are described in more detail below.

### Description of Various Preferred Embodiments

Various preferred aspects of the present invention are summarized below and further described and illustrated in the subsequent detailed descriptions and examples.

One preferred aspect of the present invention is to treat CHF, in which one or more AC proteins is synthesized *in vivo* in a patient by targeting the myocardium with a vector construct containing a gene encoding an AC. The preferred aspects employ vector constructs that result in localized expression of the AC that is relatively restricted to the myocardium of the patient. The presently preferred adenylylcyclases ("AC"s) include cardiac AC such as AC_{II}, AC_{V} or AC_{VI}, more preferably AC_{VI}.

Preferred vectors for use in the present invention include viral vectors, lipid-based vectors and other vectors that are capable of delivering DNA to non-dividing cells *in vivo.* Presently preferred are viral vectors, particularly replication-defective viral vectors (including, for example replication-defective adenovirus vectors and adeno-associated virus (AAV) vectors). For ease of production and use in the present invention, replication-defective adenovirus vectors are presently most preferred.

The presently preferred means of in *vivo* delivery (especially for vector constructs that are not otherwise targeted for delivery and/or expression that is restricted to the myocardium), is by injection of the vector into a blood vessel directly supplying the myocardium, preferably by injection into a coronary artery. Such injection is preferably achieved by catheter introduced substantially (typically at least about 1 cm) within the ostium of one or both coronary arteries or one or more saphenous veins or internal mammary artery grafts or other conduits delivering blood to the myocardium.

By injecting the vector stock, preferably containing no wild-type virus, deeply into the lumen of one or both coronary arteries (or grafts and other vascular conduits), preferably into both the right and left coronary arteries (or grafts and other vascular conduits), and preferably in an amount of 10⁷-10¹³ viral particles as determined by optical densitometry (more preferably 10⁹-10¹¹ viral particles), it is possible to locally transfect a desired number of cells, especially cardiac myocytes, with genes that encode proteins that increase β-adrenergic signal transduction in the affected myocardium, thereby maximizing therapeutic efficacy of gene transfer, and minimizing undesirable effects at extracardiac sites and the possibility of an inflammatory response to viral proteins. Vector constructs that are specifically targeted to the myocardium, such as vectors incorporating myocardial-specific binding or uptake components; and/or which incorporate AC transgenes that are under the control of myocardial-specific transcriptional regulatory sequences (e.g., ventricular myocyte-specific promoters) can be used in place of or, preferably, in addition to such directed injection techniques as a means of further restricting expression to the myocardium, especially the ventricular myocytes. For vectors that can elicit an immune response, it is preferable to inject the vector directly into a blood vessel supplying the myocardium as described above, although the additional techniques for restricting the potential for extracardiac expression can also be employed.

As described in detail below, we have shown that the use of such techniques with vectors carrying AC transgenes can effectively enhance endogenous β-adrenergic responsiveness and function within the myocardium of a large mammal heart, without any observed effect on non-cardiac tissues and without generating any substantial immune reaction.

Also described is a filtered, injectable adenovirus vector preparation, comprising a recombinant adenovirus vector, preferably in a final viral titer of 10⁷-10¹⁴ viral particles, said vector containing no wild-type virus and comprising a partial adenovirus sequence from which one or more required adenovirus genes conferring replication competence, for example, the E1A/E1B genes have been deleted, and a transgene coding for an AC such as AC_{Vl}, ACᵥ, or other adenylylcyclase, driven by a promoter flanked by the partial adenovirus sequence; and a pharmaceutically acceptable carrier.

Also described are methods for the generation of recombinant viral stocks capable of effecting expression of an AC*in vivo* in the myocardium, comprising the steps of cloning a transgene coding for an AC (such as AC_{VI}, AC_{V}, or other adenylylcyclase) into a plasmid containing a promoter and a polylinker flanked by partial adenovirus sequences of an adenovirus genome from which one or more adenovirus genes required for replication competence (generically referred to as viral replication or "rep" genes), such as the E1A/E1B genes of the human adenovirus 5 genome, have been deleted; co-transfecting said plasmid into mammalian cells transformed with the missing replication genes, along with a plasmid which contains a complete adenovirus genome and an additional insert making the plasmid too large to be encapsidated, whereby rescue recombination takes place between the transgene-inserted plasmid and the plasmid having the entire adenovirus genome so as to create a recombinant genome containing the transgene without the deleted viral replication genes, said recombinant genome being sufficiently small to be encapsidated; identifying successful recombinants in cell cultures; propagating the resulting recombinant in mammalian cells comprising or transformed with the viral replication genes; and purifying the propagated recombinants so as to contain the recombinant vector, without wild-type virus therein, and preferably passing the purified vector through a filter, preferably 0.1 - 0.5 micron filter, more preferably a 0.3 micron filter, to obtain purified filtered recombinant virus stock.

These and other preferred aspects are described and illustrated below.

### Transgenes Encoding β-Adrenergic Signaling Elements

The present invention employs genes encoding protein or peptide elements that increase β-adrenergic signaling and are therefore capable of enhancing responsiveness to endogenous β-adrenergic stimulation within dysfunctional regions of a mammalian heart. Such proteins are referred to herein as "β-adrenergic signaling proteins" (or "β-ASPs"). The term β-ASP refers to a protein that is capable of enhancing β-adrenergic signaling when expressed in mammalian tissue, preferably (for purposes of the present invention) when expressed in mammalian myocardial tissue.

β-adrenergic signaling proteins include β-adrenergic signal transducer proteins that mediate or transduce β-adrenergic signaling, preferably in mammalian myocardial cells, as well as proteins which can either stimulate such transducer proteins or which can inactivate or compete with inhibitors of such transducer proteins (thereby indirectly enhancing signal transduction). A variety of such proteins that are associated with β-adrenergic signaling in mammalian cardiac tissue have been identified (see, e.g., the various references regarding β-adrenergic responsiveness cited above) and are illustrated herein.

The invention uses an AC gene. Preferred additional β-ASPs for use in the present invention are those that are known to play a role in β-adrenergic signal transduction in mammalian heart tissue, such as the various proteins associated with the "βAR-Gs-AC" pathway, comprising a β-adrenergic receptor ("βAR"), a Gₛ protein transducer and an adenylylcyclase ("AC'') effector, as described in more detail herein and in the cited art. Recent data have demonstrated that Gₛ protein is generally present at a much higher molar proportion than either βAR or AC.

β-adrenergic signaling within myocardial tissue is initially mediated by agonist binding to βAR, followed by Gₛ-mediated signal transduction to AC. Activated AC then catalyzes the synthesis of cyclic AMP, and increased intracellular concentrations of cAMP mediate increased cytosolic calcium transients which enhance both the rate and force of cardiac contraction (referred to as positive "chronotrophy" and positive "inotrophy," respectively).

Examples of particularly preferred additional β-ASPs for use in the present invention thus include: β-adrenergic receptors (such as β₁-adrenergic receptors or β₂-adrenergic receptors, preferably β₁-adrenergic receptors), as well as inhibitors of the function of G-protein receptor kinases (which are generally referred to herein as "GRK" inhibitors).

β-adrenergic receptors (abbreviated "β-AR" or "βAR") are cell-surface receptors involved in β-adrenergic signaling via the βAR-Gs-AC pathway. Within the myocardium of a mammalian heart, βARs are the principal receptors for norepinephrine (the sympathetic neurotransmitter) and for epinephrine (the adrenal hormone). Human myocardium contains both β₁-adrenergic receptors and β₂-adrenergic receptors, but β₁-ARs are predominant and are most closely associated with the altered β-adrenergic signaling that is observed with heart failure.

Gₛ protein is a GTP-binding regulatory protein that effectively couples activation of a variety of cell-surface receptors (including β-adrenergic receptors) to the activation of adenylylcyclase, as described in the art and herein.

Adenylylcyclase (also referred to as "adenylylcyclase," and abbreviated "AC") is an enzyme that catalyzes the conversion of adenosine triphosphate (ATP) to 3':5'-cyclic adenosine monophosphate (cAMP). Adenylylcyclase is known to exist in a number of different isoforms that are found in varying levels in most all mammalian tissues. The most preferred adenylylcyclases used in the present invention are "cardiac adenylylcyclases" which are isoforms found to be predominant in mammalian heart tissue, particularly in cardiac myocytes; as described in more detail below.

G-protein receptor kinases (abbreviated "GRK", but also referred to in the art as "β-adrenergic receptor kinases" or "βARK"), are kinase proteins that catalyze phosphorylation of G-protein-coupled receptor proteins including β-adrenergic receptors ("βARs"). Phosphorylation of βARs by GRK proteins leads to inactivation of the receptors and a concomitant decrease in responsiveness to β-adrenergic signaling.

GRK inhibitors, as used herein, refer to peptide inhibitors of the function of G-protein receptor kinases. Peptide inhibitors of GRK include modified GRK proteins in which receptor-binding activity has been uncoupled from kinase activity. Exemplary GRK inhibitors thus include modified GRKs that have been truncated (typically by deletions beginning at the amino-terminus) to remove kinase function while retaining the ability to bind to G-protein-coupled receptor proteins such as βARs. Such truncated GRK proteins can thus effectively compete with or prevent normal GRK from binding to βAR but without causing subsequent inhibition of receptor activity.

Genes encoding such β-adrenergic signaling proteins, including preferred genes encoding βARs, AC isoforms and inhibitors of GRK proteins are known in the art and generally available (see, e.g., the references cited above regarding β-adrenergic signaling components). In addition, since these components tend to be relatively highly conserved, new homologs (or isoforms) of known genes can generally be readily obtained by screening a cDNA or genomic library of interest (e.g., a tissue-specific cDNA library), using techniques that are now quite well known in the art (see, e.g., the molecular biology references cited herein).

The most preferred adenylylcyclases of the present invention are "cardiac adenylylcyclases" which are isoforms found to be predominant in mammalian heart tissue, particularly in cardiac myocytes. Presently preferred cardiac ACs include AC isoform V (abbreviated "AC_{V}") and AC isoform VI (abbreviated "AC_{VI}"), with AC_{VI} being presently most preferred for reasons described herein. Although the various AC isoforms are distinct in terms of DNA and protein sequence, and are typically expressed in a tissue-specific manner, certain of the isoforms are closely homologous to each other and the mammalian isoforms generally have a common topographical feature comprising transmembrane spanning regions that are associated with large cytoplasmic loops. In addition, the amino acid composition of the cytoplasmic loops tends to be conserved among isoforms. Typically, cloned DNA encoding such adenylylcyclases will already be available as plasmids, although polynucleotides encoding the enzymes can also be obtained using polymerase chain reaction (PCR) methodology, as described in the art (see, e.g., PCR: A Practical Approach (M. McPherson et al., IRL Press at Oxford University Press 1991)). The detection, purification, and characterization of adenylylcyclases, including assays for identifying and characterizing new adenylylcyclases effective in a given cell type, have also been described in a number of publications (see, e.g., the references cited herein by Ishikawa et al. and Krupinski et al., regarding AC isoforms).

As described and illustrated in more detail below, we have successfully employed gene therapy techniques to deliver vectors encoding AC into the myocardium of a large animal model that has been determined to be predictive of heart function in humans. We have also shown that gene delivery of the AC resulted in enhanced cardiac function in the animals tested, indicating that the invention is likely to provide effective alternatives to present treatments for congestive heart failure.

### Vectors for Gene Delivery in vivo

In general, the gene of interest is transferred to the heart, including cardiac myocytes, *in vivo* and directs production of the encoded protein. Preferably such production is relatively constitutive. A variety of different gene transfer vectors, including viral as well as non-viral systems, can be employed to deliver transgenes for use in the present invention (see, e.g., the references cited above). As illustrated below, we have found that the helper-independent replication-defective human adenovirus 5 system can be used effectively transfect a large percentage of myocardial cells *in vivo* by a single intracoronary injection. We have also shown that such a delivery technique can be used to effectively target vectors to the myocardium of a large mammal heart. Additional means of targeting vectors to particular cells or tissue types are described below and in the art.

In various illustrations described below, we have used recombinant adenovirus vectors based on the human adenovirus 5 (as described by McGrory WJ, et al., Virology 163: 614-617, 1988) which are missing essential early genes from the adenovirus genome (usually E1A/E1B), and are therefore unable to replicate unless grown in permissive cell lines that provide the missing gene products *in trans.* In place of the missing adenovirus genomic sequences, a transgene of interest can be cloned and expressed in tissue/cells infected with the replication-defective adenovirus. Although adenovirus-based gene transfer does not generally result in stable integration of the transgene into the host genome (less than 0.1 % adenovirus-mediated transfections result in transgene incorporation into host DNA), adenovirus vectors can be propagated in high titer and transfect non-replicating cells; and, although the transgene is not passed to daughter cells, this is suitable for gene transfer to adult cardiac myocytes, which do not actively divide. Retrovirus vectors provide stable gene transfer, and high titers are now obtainable via retrovirus pseudotyping (Burns, et al., Proc Natl Acad Sci (USA) 90: 8033-8037, 1993), but current retrovirus vectors are generally unable to efficiently transduce nonreplicating cells.

An advantage associated with nondividing cells such as myocytes is that the viral vector is not readily "diluted out" by host cell division. To further enhance duration of transgene expression in the heart, however, it is also possible to employ various second generation adenovirus vectors that have both E1 and E4 deletions, which can be used in conjunction with cyclophosphamide administration (See, e.g., Dai et al., Proc. Nat'l Acad Sci. (USA) 92: 1401-1405, 1995). To further increase the extent of initial gene transfer, multiple infusions, or infusion in an isolated coronary circuit can also be employed.

Human 293 cells, which are human embryonic kidney cells transformed with adenovirus E1A/E1B genes, typify useful permissive cell lines for the production of such replication-defective vectors. However, other cell lines which allow replication-defective adenovirus vectors to propagate therein can also be used, such as HeLa cells.

References describing a variety of other gene delivery vectors are known in the art, some of which are cited herein. Such other vectors include, for example, other viral vectors (such as adeno-associated viruses (AAV), liposomes and other lipid-containing complexes, and other macromolecular complexes capable of mediating delivery of a polynucleotide to a host cell. As described above and in the cited references, vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Selectable markers can be positive, negative or bifunctional. Positive selectable markers allow selection for cells carrying the marker, whereas negative selectable markers allow cells carrying the marker to be selectively eliminated. A variety of such marker genes have been described, including bifunctional (i.e. positive/negative) markers (see, e.g., Lupton, S., WO 92/08796, published 29 May 1992; and Lupton, S., WO 94/28143, published 8 December 1994). Such marker genes can provide an added measure of control that can be advantageous in gene therapy contexts. A large variety of such vectors are known in the art and are generally available (see, e.g., the various references cited above).

Additional references describing adenovirus vectors and other viral vectors which could be used in the methods of the present invention include the following: Horwitz, M.S., Adenoviridae and Their Replication, in Fields, B., et al. (eds.) Virology, Vol. 2, Raven Press New York, pp. 1679-1721, 1990); Graham, F., et al., pp. 109-128 in Methods in Molecular Biology, Vol. 7: Gene Transfer and Expression Protocols, Murray, E. (ed.), Humana Press, Clifton, N.J. (1991); Miller, N., et al., FASEB Journal 9: 190-199, 1995; Schreier, H, Pharmaceutica Acta Helvetiae 68: 145-159, 1994; Schneider and French, Circulation **88**:1937-1942, 1993; Curiel D.T., et al., Human Gene Therapy 3: 147-154, 1992; Graham, F.L., et al., WO 95/00655 (5 January 1995); Falck-Pedersen, E.S., WO 95/16772 (22 June 1995); Denefle, P. et al., WO 95/23867 (8 September 1995); Haddada, H. et al., WO 94/26914 (24 November 1994); Perricaudet, M. et al., WO 95/02697 (26 January 1995); Zhang, W., et al., WO 95/25071 (12 October 1995). A variety of adenovirus plasmids are also available from commercial sources, including, e.g., Microbix Biosystems of Toronto, Ontario (see, e.g., Microbix Product Information Sheet: Plasmids for Adenovirus Vector Construction, 1996).

Additional references describing AAV vectors which could be used in the methods of the present invention include the following: Carter, B., Handbook of Parvoviruses, vol. I, pp. 169-228, 1990; Berns, Virology, pp. 1743-1764 (Raven Press 1990); Carter, B., Curr. Opin. Biotechnol., 3: 533-539, 1992; Muzyczka, N., Current Topics in Microbiology and Immunology, 158: 92-129, 1992; Flotte, T.R., et al., Am. J. Respir. Cell Mol. Biol. 7:349-356, 1992; Chatterjee et al., Ann. NY Acad. Sci., 770: 79-90, 1995; Flotte, T.R., et al., WO 95/13365 (18 May 1995); Trempe, J.P., et al., WO 95/13392 (18 May 1995); Kotin, R., Human Gene Therapy, 5: 793-801, 1994; Flotte, T.R., et al., Gene Therapy 2:357-362, 1995; Allen, J.M., WO 96/17947 (13 June 1996); and Du et al., Gene Therapy 3: 254-261, 1996.

Additional references describing non-viral vectors which could be used in the methods of the present invention include the following: Ledley, FD, Human Gene Therapy 6: 1129-1144, 1995; Miller, N., et al., FASEB Journal 9: 190-199, 1995; Chonn, A., et al., Curr. Opin. in Biotech. 6: 698-708, 1995; Schofield, JP, et al., British Med. Bull. 51: 56-71, 1995; Brigham, K.L., et al., J. Liposome Res. 3: 31-49, 1993; Brigham, K.L., WO 91/06309 (16 May 1991); Felgner, P.L., et al., WO 91/17424 (14 November 1991); Solodin et al., Biochemistry 34: 13537-13544, 1995; WO 93/19768 (14 October 1993); Debs et al., WO 93/25673; Felgner, P.L., et al., U.S. Patent 5,264,618 (November 23, 1993); Epand, R.M., et al., U.S. Patent 5,283,185 (February 1, 1994); Gebeyehu et al., U.S. Patent 5,334,761 (August 2, 1994); Felgner, P.L., et al., U.S. Patent 5,459,127 (October 17, 1995); Overell, R.W., et al., WO 95/28494 (26 October 1995); Jessee, WO 95/02698 (26 January 1995); Haces and Ciccarone, WO 95/17373 (29 June 1995); Lin et al., WO 96/01840 (25 January 1996).

### Construction of Recombinant Viral Vectors

For purposes of illustrating vector-mediated gene delivery of AC to the myocardium, we chose a basic (i.e. "first generation") adenovirus vector that can be constructed by the rescue recombination technique as described in McGrory WJ, et al., Virology **163:**614-617, 1988. Briefly, the transgene of interest is cloned into a shuttle vector that contains a promoter, polylinker and partial flanking adenovirus sequences from which E1A/E1B genes have been deleted.

Illustrative shuttle vectors include, e.g., plasmid"pAC1" (Virology 163:614-617, 1988) (or an analog) which encodes portions of the left end of the human adenovirus 5 genome but lacks the early protein region comprising E1A and E1B sequences that are essential for viral replication; and plasmid "ACCMVPLPA" (J Biol Chem 267:25129-25134, 1992) which contains a polylinker, CMV promoter and SV40 polyadenylation signal flanked by partial adenovirus sequences from which the E1A/E1B genes have been deleted. The use of plasmids such as pAC 1 or ACCMVPLA can thus facilitate the cloning process.

The shuttle vector can then be co-transfected, along with a plasmid comprising the entire human adenovirus 5 genome (but with a length too large to be encapsidated), into suitable host cells such as human 293 cells. Co-transfection can be, conducted by calcium phosphate precipitation or lipofection (see, e.g., Biotechniques 15:868-872, 1993).

As an illustrative plasmid for co-transfection, plasmid "JM17" encodes the entire human adenovirus 5 genome plus portions of the vector pBR322 including the gene for ampicillin resistance (4.3 kb) (Giordano, et al. Nature Medicine 2: 534-539, 1996). Although JM17 encodes all of the adenovirus proteins necessary to make mature viral particles, it is too large to be encapsidated (40 kb versus 36 kb for wild type).

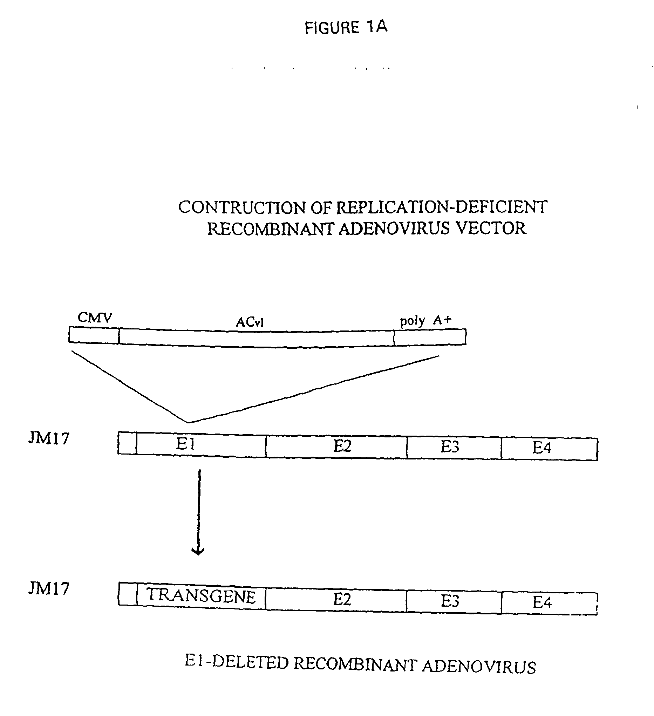

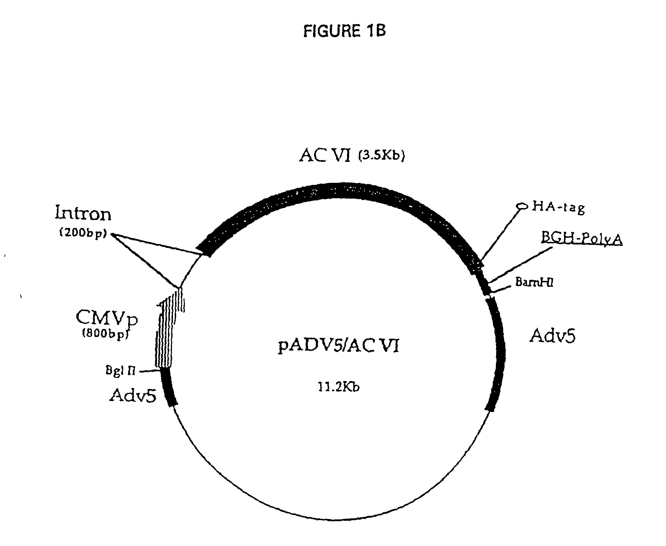

In a small subset of co-transfected cells, "rescue recombination" occurs between the transgene-containing shuttle vector (such as plasmid pACl) and the plasmid having the entire adenovirus 5 genome (such as plasmid pJM1.7) which generates a recombinant genome that contains the transgene of interest in place of the deleted E1A/E1B sequences, and that secondarily loses the additional sequence (such as pBR322 sequences) during recombination, thereby being small enough to be encapsidated (see, e.g., Giordano, et al. Nature Medicine 2: 534-539, 1996). An illustration of such a vector is presented in Figure 1. The CMV driven β-galactosidase gene in adenovirus HCMVSP11acZ (Nature Medicine 2: 534-539, 1996) can be used to evaluate the efficiency of gene transfer using X-gal treatment.

Illustrative examples demonstrating the preparation and use of such vectors are provided below. Advantages of using adenovirus vectors include the ability to effect high efficiency gene transfer (as many as 50% of target organ cells transfected *in vivo*), the ease of obtaining high titer viral stocks and the ability of these vectors to effect gene transfer into cells such as cardiac myocytes which do not divide.

A variety of other vectors suitable for *in vivo* gene therapy can also be readily employed to deliver AC transgenes in accordance with the present invention. Such other vectors include, by way of illustration, other viral vectors such as adeno-associated virus (AAV) vectors; non-viral protein-based delivery platforms); as well as lipid-based vectors (including, e.g., cationic liposomes and analogous gene delivery complexes). The preparation and use of these and other vectors are described in the art (see, e.g., the references regarding gene delivery vectors cited above).

### Targeted AC Vector Constructs

The present invention contemplates the use of cell targeting not only by delivery of the transgene into the coronary artery, for example, but also by use of targeted vector constructs having features that tend to target gene delivery and/or gene expression to particular host cells or host cell types (such as the myocardium). Such targeted vector constructs would thus include targeted delivery vectors and/or targeted vectors, as described in more detail below and in the published art. Restricting delivery and/or expression can be beneficial as a means of further focusing the potential effects of gene therapy. The potential usefulness of further restricting delivery/expression depends in large part on the type of vector being used and the method and place of introduction of such vector. As described herein, delivery of viral vectors via intracoronary injection to the myocardium has been observed to provide, in itself, highly targeted gene delivery (see the Examples below). In addition, using vectors that do not result in transgene integration into a replicon of the host cell (such as adenovirus and numerous other vectors), cardiac myocytes are expected to exhibit relatively long transgene expression since the cells do not undergo rapid turnover. In contrast, expression in more rapidly dividing cells would tend to be decreased by cell division and turnover. However, other means of limiting delivery and/or expression can also be employed, in addition to or in place of the illustrated delivery method, as described herein.

Targeted delivery vectors include, for example, vectors (such as viruses, non-viral protein-based vectors and lipid-based vectors) having surface components (such as a member of a ligand-receptor pair, the other half of which is found on a host cell to be targeted) or other features that mediate preferential binding and/or gene delivery to particular host cells or host cell types. As is known in the art, a number of vectors of both viral and non-viral origin have inherent properties facilitating such preferential binding and/or have been modified to effect preferential targeting (see, e.g., Miller, N., et al., FASEB Journal 9: 190-199, 1995; Chonn, A., et al., Curr. Opin. in Biotech. 6: 698-708, 1995; Schofield, JP, et al., British Med. Bull. 51: 56-71, 1995; Schreier, H, Pharmaceutica Acta Helvetiae 68: 145-159, 1994; Ledley, FD, Human Gene Therapy 6: 1129-1144, 1995; Conary, J.T., et al., WO 95/34647 (21 December 1995); Overell, R.W., et al., WO 95/28494 (26 October 1995); and Truong, V.L. et al., WO 96/00295 (4 January 1996)).

Targeted vectors include vectors (such as viruses, non-viral protein-based vectors and lipid-based vectors) in which delivery results in transgene expression AC is relatively limited to particular host cells or host cell types. By way of illustration, AC transgenes to be delivered according to the present invention can be operably linked to heterologous tissue-specific promoters thereby restricting expression to cells in that particular tissue.

For example, tissue-specific transcriptional control sequences derived from a gene encoding left ventricular myosin light chain-2 (MLC_{2V}) or myosin heavy chain (MHC) can be fused to an AC transgene (such as the AC_{V1} gene) within a vector such as the adenovirus constructs described above. Expression of the transgene can therefore be relatively restricted to ventricular cardiac myocytes. The efficacy of gene expression and degree of specificity provided by MLC_{2V} and MHC promoters with lacZ have been determined (using a recombinant adenovirus system such as that exemplified herein); and cardiac-specific expression has been reported (see, e.g., Lee, et al., J Biol Chem 267:15875-15885,1992).

Since the MLC_{2V} promoter comprises only about 250 bp, it will fit easily within even size-restricted delivery vectors such as the adenovirus-5 packaging system exemplified herein. The myosin heavy chain promoter, known to be a vigorous promoter of transcription, provides another alternative cardiac-specific promoter and comprises less than 300 bp. Other promoters, such as the troponin-C promoter, while highly efficacious and sufficiently small, do not provide such tissue specificity.

### Propagation and Purification of Viral Vectors

Recombinant viral vectors, such as adenoviral vectors, can be plaque purified according to standard methods. By way of illustration, recombinant adenoviral viral vectors can be propagated in human 293 cells (which provide E1A and E1B functions *in trans*) to titers in the preferred range of about 10¹⁰-10¹² viral particles/ml.

Propagation and purification techniques have been described for a variety of viral vectors that can be used in conjunction with the present invention. Adenoviral vectors are exemplified herein but other viral vectors such as AAV can also be employed. For adenovirus, cells can be infected at about 80% confluence and harvested 48 hours later. After 3 freeze-thaw cycles the cellular debris can be collected by centrifugation and the virus purified by CsCl gradient ultracentrifugation (double CsCl gradient ultracentrifugation is preferred).

Prior to *in vivo* injection, the viral stocks can be desalted by gel filtration through Sepharose columns such as G25 Sephadex. The product can then be filtered through a 30 micron filter, thereby reducing the potential for deleterious effects associated with intracoronary injection of unfiltered virus. The resulting viral stock preferably has a final viral titer that is at least about 10¹⁰-10¹² viral particles/ml.

Preferably, the recombinant adenovirus is highly purified, and is substantially free of wild-type (potentially replicative) virus. For these reasons, propagation and purification can be conducted to exclude contaminants and wild-type virus by, for example, identifying successful recombinants with PCR using appropriate primers, conducting two rounds of plaque purification, and double CsCl gradient ultracentrifugation. Additionally, we have found that the problems associated with cardiac arrhythmias that can be induced by adenovirus vector injections into patients can be essentially avoided by filtration of the recombinant adenovirus through an appropriately-sized filter prior to intracoronary injection. This strategy also appears to substantially improve gene transfer and expression.

### Delivery of Vectors Carrying one or More AC Transgenes

The means and compositions which are used to deliver the vectors carrying AC transgenes depend on the particular vector employed as is well known in the art. Typically, however, a vector can be in the form of an injectable preparation containing pharmaceutically acceptable carrier/diluent such as saline, for example.

For viral vectors (such as adenovirus), the final titer of the virus in the injectable preparation is preferably in the range of about 10⁷-10¹³ viral particles which allows for effective gene transfer. Other pharmaceutical carriers, formulations and dosages are described below.

Vectors comprising AC transgenes can be delivered to the myocardium by direct intracoronary (or graft vessel) injection using standard percutaneous catheter based methods under fluoroscopic guidance, in an amount sufficient for the transgene to be expressed and to provide a therapeutic benefit. Such an injection is preferably made deeply into the lumen (about 1 cm within the arterial lumen) of the coronary arteries (or graft vessel), and preferably is made in both coronary arteries (to provide general distribution to all areas of the heart).

By injecting the material directly into the lumen of the coronary artery by coronary catheters, it is possible to target the gene rather effectively, and to minimize loss of the recombinant vectors to the proximal aorta during injection. We have found that gene expression when delivered in this manner does not occur in hepatocytes and that viral RNA cannot be found in the urine at any time after intracoronary injection. In addition, using PCR, we find no evidence of extracardiac gene expression in the eye, liver, or skeletal muscle two weeks after intracoronary delivery. Any variety of coronary catheter can be used in the present invention. In addition, other techniques known to those having ordinary skill in the art can be used for transfer of genes to the heart.

### Animal Model of Congestive Heart Failure

Important prerequisites for developing any cardiac gene therapy technique to be applicable to humans are: (a) constitution of a large animal model that is applicable to clinical congestive heart failure and which can provide useful data regarding mechanisms for altered β-adrenergic signaling in the setting of heart failure, and (b) accurate evaluation of the effects of gene transfer. None of the prior art has effectively described and/or demonstrated a means for treating congestive heart failure using *in vivo* gene therapy.

For this invention, we have employed a porcine model of heart failure that mimics human clinical congestive heart failure in a number of important ways, including the clinical abnormalities associated with β-adrenergic signaling. In our porcine model, sustaine ' rapid ventricular pacing in these large mammals (225 beats/min) results in left ventricular chamber enlargement, depressed systolic function, and hemodynamic abnormalities, all of which mimic clinical dilated heart failure in humans (see, e.g., Roth DA, et al., J Clin Invest 91: 939-949, 1993). Furthermore, the detailed analysis of altered β-adrenergic signaling in the porcine model heart, particularly plasma and myocardial catecholamine levels, β-adrenergic receptor down-regulation and uncoupling, and alterations in adenylylcyclase function, likewise mimic conditions associated with heart failure in humans (Roth DA, et al., J Clin Invest 91: 939-949, 1993).

The fundamental findings from studies conducted on myocardium from these animal models with heart failure vis-A-vis the current invention include the following. First, there is a 75% reduction in left ventricular β₁-adrenergic receptor number, with a similar decrease in β₁-adrenergic receptor mRNA; whereas β₂ -adrenergic receptor number (and mRNA) do not change. This mirrors what is seen in human heart failure (see, e.g., Bristow MR, et al., J Clin Invest 92: 2737-2745, 1993). Second, left ventricular β-adrenergic receptors are uncoupled from Gs, and there is increased function and expression of G-protein receptor kinase. These findings are also present in failed human hearts (see, e.g., Ungerer M, et al., Circulation, 87: 454-461, 1993; Ungerer M, et al., Circ Res, 74: 206-213, 1994). Third, there is a reduction in the function of left ventricular AC, that is associated with mRNA for AC_{VI} (Roth DA, et al., J Clin Invest, 91: 939-949, 1993; see also Ishikawa Y, et al., J Clin Invest, 93: 2224-2229, 1994).

Recent studies also show reduced forskolin-stimulated cAMP production in homogenates of failing left ventricle, suggesting impaired AC function (Bristow, M.R.et al., Mol. Pharm. 35, 295-303, 1989; Ishikawa, Y., et al., J. Clin. Invest. **93**, 2224-2229, 1994; Kiuchi K., et al. J. Clin. Invest. **91**, 907-914, 1993; Marzo, K.P., et al., Circ. Res. 69,1546-1556, 1991; Roth, D.A., et al., J. Clin. Invest. **91**, 939-949, 1993). A problem with the assessment of the catalytic subunit of AC is that there are currently only imperfect means to assess either its concentration or its function. The diterpene forskolin is an activator of AC and, accordingly, forskolin-stimulated cAMP production has been the most commonly employed method for assaying its biological activity. However, since full response to forskolin involves interaction of Gs₁ and AC, and since activation of Gsα inhibits forskolin response (Darfler, F.J., et al., J. Biol. Chem. **257,** 11901-11907, 1982), forskolin stimulation falls short of providing a precise measure of AC function. It is widely recognized that AC tends to be quite labile and is generally unstable out of its normal cellular environment. Antibodies to AC are not widely available, and the protein is expressed in low abundance, exacerbating the problems of quantitation and functional assessment of this pivotal transducing element. Because of these problems, little is known about the precise alterations in quantity and function of AC in pathophysiological settings. Regulation of cellular function by AC is further complicated by the recent evidence that multiple isoforms of AC exist, at least two of which have been demonstrated to be expressed in the heart (Ishikawa, Y., et al., J. Clin. Invest. **93,** 2224-2229, 1994; Iyengar R. FASEB J. 7, 768-775, 1993; Katsusshika, S., et al., Proc. Natl. Acad. Sci. (U.S.A.) **89,** 8774-8778, 1992; Krupinski, J., et al., J. Biol. Chem. **267,**24858-24862, 1992**;** Tang, W.J. and A.G. Gilman. Cell 70, 869-872, 1992; Taussig, R., et al., J. Biol. Chem. **269,** 6093-6100, 1994). Studies from our laboratories have employed two approaches to provide quantitative information regarding AC expression. First, forskolin binding is used to quantitate AC function (Alousi A, et al. FASEB Journal 5:2300-2303, 1991). Second, RNase protection assays are used to provide quantitative assessment of mRNA levels for AC isofonns (Ping P, et al., Circulation 90: I-I-580, 1994; Ping P and Hammond HK, Am J Physiol 267: H2079-H2085, 1994).

Using degenerate PCR primers for AC isoforms, we have isolated twenty-eight positive clones. Subsequent sequence analyses established, based upon previously reported sequences of AC isoforms, that at least three AC isoforms are expressed in porcine left ventricle. Previous reports regarding AC isoform expression in mammalian heart using whole rat heart homogenates and PCR identified eight AC isoforms (Katsusshika, S., et al., Proc. Natl. Acad. Sci. (U.S.A.) **89,** 8774-8778, 1992); although the cell types expressing the various AC isoforms were not identified. Subsequent studies using Northern blotting (with poly-(A)-selection) have been able to identify only two isoforms, AC_{V} and AC_{VI} in dog heart (Ishikawa, Y., et al., J. Clin. Invest. **93,** 2224-2229, 1994). Ishikawa, et al. (Ishikawa, Y., et al., J. Clin. Invest. 93, 2224-2229, 1994) confirmed that AC_{V} and AC_{VI} isoforms could be identified in RNA extracted from isolated cardiac myocytes.

We have found, with respect to the presently-employed porcine model, that AC isoforms II, V, and VI are present not only in RNA extracted from whole heart, but also in RNA extracted from a pure population of adult porcine left ventricular cardiac myocytes.

A previous study showed that severe heart failure was associated with downregulation of AC isoforms V and VI mRNA (Ishikawa, Y., et al., J. Clin. Invest. 93, 2224-2229, 1994). We used an animal model that mimics clinical CHF to see if AC isoform mRNA downregulation in heart failure is uniform among isoforms. Although our findings are in agreement with some aspects of previous reports, including an early uncoupling of myocardial βAR (Ishikawa, Y., et al., J. Clin. Invest. **93**, 2224-2229, 1994; Kiuchi K., et al. J. Clin. Invest. **91**, 907-914, 1993), there are also differences. First, we identified a third AC isoform of cardiac myocyte origin (AC_{H}), and established, through quantitative measurements, the level of expression of these three isoforms in left ventricle. Second, in contrast to Ishikawa, et al. (Ishikawa, Y., et al., J. Clin. Invest. **93**, 2224-2229, 1994), we found that AC isoform downregulation is isoform-specific. In particular, reduced AC_{VI} expression appears to be associated with impaired β-adrenergic responsiveness in CHF.

In summary, in the normal heart, the amount of AC appears to set a limit on β-adrenergic responsiveness. In heart failure, where AC downregulation further impairs transmembrane signaling, this would be expected to be an even more important limitation affecting transmembrane β-adrenergic signaling.

Our studies with pigs, described and illustrated in more detail below, demonstrate that over-expression of AC, by *in vivo* delivery to the myocardium, can enhance cardiac function in a large animal model predictive of humans. There are no previous publications describing and/or demonstrating such techniques for use in the treatment of heart failure. Our studies also provided additional confirmation that the CHF phenotype in the porcine pacing model is very similar to that observed in clinical CHF in humans.

Our demonstrated ability (using the methods of the present invention) to significantly increase adrenergic responsiveness in the porcine pacing model of CHF thus provides a critical advance, not only for understanding the molecular mechanisms of cardiac adrenergic signaling but also for actually treating the clinical condition.

As described in the examples below, we used normal pigs to initially examine the efficacy of our gene delivery techniques and to provide data to show that intracoronary gene delivery of a recombinant adenovirus expressing AC_{VI} could positively impact cardiac function. In these initial studies, a recombinant adenovirus expressing lacZ (as a reporter gene used to document successful gene transfer and expression) was injected into the coronary arteries of 5 pigs (using approximately 0.5 x 10¹¹ viral particles). Two weeks later, the animals were killed and tissue samples were examined.

PCR was used to detect adenovirus DNA in myocardium from animals that had received gene transfer. Effective gene transfer was documented in the hearts of the pigs, and was not found in other tissues. As further confirmation of the success of these techniques, myocardial samples from lacZ-infected animals were found to exhibit substantial β-galactosidase activity on histological inspection.

To illustrate cardiac gene therapy using a β-ASP transgene, pigs were exposed to *in vivo* gene delivery of DNA encoding an AC_{VI} isoform. In studies conducted on three animals, physiological measures of β-adrenergic responsiveness were obtained before and 5-10 days after intracoronary injection of a recombinant adenovirus expressing the β-ASP transgene (encoding AC_{VI}).

Our results revealed that heart rate responsiveness to β-adrenergic stimulation was significantly increased after gene transfer according to the present invention.

In addition, one of the pigs was further examined (both before and after gene transfer), to monitor potential changes in left ventricular dP/dt, which is a measure of the rate of rise of pressure development, and a further indicator of cardiac function. The results, described below, indicated that cardiac gene therapy according to the present invention also resulted in a substantial increase in left ventricular dP/dt. These data thus provided further confirmation that the techniques of the present invention can be used to enhance cardiac function in a large animal model that mimics CHF in humans.

Our data demonstrated that a β-ASP, AC, can be effectively delivered by *in vivo* gene therapy to a large animal heart and that β-adrenergic responsiveness and cardiac function can be increased using such a method. Additional preferred β-ASP can be delivered in an analogous manner. By way of illustration, in the following description and in the examples, we describe additional types of β-ASPs, including a β-adrenergic receptor (β₁-AR) and a GRK inhibitor. β₁-AR is functionally upstream of AC in the βAR-G₅-AC pathway, and like AC has been found to be down-regulated in association with heart failure.

With further regard to the predictiveness of the pacing-induced model of heart failure to clinical heart failure in humans, it is important to note that although the two end conditions are not brought about in the same manner (i.e. in terms of etiology and rate of development), the resulting states of heart failure necessitating treatment are quite closely related. Thus, not only are both systems characterized by dilated, poorly contracting hearts, and multi-chamber enlargement, but, most importantly, the two systems exhibit strikingly similar abnormalities associated with β-adrenergic signaling (see, e.g., Bristow MR, et al., N Engl J Med, 307: 205-211, 1982; Bristow MR, et al., Mol Pharm, 35: 295-303, 1989; Bristow MR, et al., J Clin Invest, 92: 2737-2745, 1993; Ishikawa Y, et al., J Clin Invest, 93: 2224-2229, 1994; Kiuchi K, et al., J Clin Invest, 91: 907-914, 1993; Marzo KP, et al., Circ Res, 69: 1546-1556, 1991; Ping P, et al., Am J Physiol, 267: H2079-H2085, 1994; Ping P, et al., J Clin Invest, 95: 1271-1280, 1995; Roth DA, et al., J Clin Invest, 91: 939-949, 1993; Ungerer M, et al., Circulation, 87: 454-461, 1993; Ungerer M, et al., Circ Res, 74: 206-213, 1994).

The data described below also document, for the first time, that myocardial GRK₅ protein and mRNA contents are susceptible to upregulation in mild and severe heart failure. Without wishing to be bound by theory, our data support the idea that increased expression of GRK₅ may be responsible for the increased GRK activity observed in these clinical conditions. In any case, the current data strongly support the hypothesis that increased left ventricular GRK expression contributes to reduced adrenergic signaling at an early stage during the development of heart failure. The role of GRK in heart failure, and the use of GRK inhibitors as a β-ASP for gene delivery are described in more detail below.

Without wishing to be bound by theory, our data support the idea that increased GRK expression is an early change in heart failure that predates alterations in AC isoform expression; and that impaired hormonal stimulation of AC, associated with βAR uncoupling, may result from increased βAR phosphorylation by GRK, possibly resulting from increased GRK₅ expression.

The enhancement of β-adrenergic responsiveness according to the present invention is expected to be beneficial for enhancing cardiac function in the numerous disease situations in which heart failure is associated with reductions in β-adrenergic signaling. In that regard, it is important to distinguish etiology from putative intervention points, particularly in situations such as this in which a molecular pathway leads from upstream signaling events to downstream effector events, and in which signaling components tend to be decreased (in number or activity) without actually being eliminated (thereby making the dysfunction and potential treatment more quantitative in nature). Thus, interventionary treatment such as that described herein can be directed at the principal molecular site of impact or potentially at a downstream site that tends to obviate or "by-pass" the principal limitation. By way of illustration, although an effective reduction in the level of a β-adrenergic receptor (β-AR) may be, and preferably is, treated directly by increasing the level of that same protein; it may also be compensated for indirectly, for example by increasing the activity of the residual β-AR proteins or relieving inhibition of such proteins (e.g. using GRK inhibitors), by increasing the activity of an analogous β-AR, and/or by increasing the availability of downstream signal transducers (e.g. AC) to make it more likely that initial signaling events result in downstream stimulation. Indeed, although our analyses described above support the idea that β-AR number and/or activity are significantly affected in severe heart failure, our results involving gene therapy *in vivo* according to the present invention (as described below) demonstrate that even intervention to increase a downstream component such as AC can substantially enhance cardiac function. Therapies directed at upstream deficiencies (employing, e.g., the delivery of β-AR and/or GRK inhibitors) would also be expected to provide substantial benefit in terms of β-adrenergic signaling and cardiac function. As described below, it will be possible to supply one or more such β-ASP transgenes as means of enhancing β-adrenergic responsiveness and cardiac function in the context of congestive heart failure.

### Therapeutic Applications

Our data demonstrate that gene transfer of an AC into the myocardium can be used to enhance responsiveness of the heart to endogenous β-adrenergic stimulation. Our technique, which we show to be successfully applicable in a large mammal model used to mimic clinical congestive heart failure in humans, would be expected to be beneficial for the treatment of CHF in humans, thereby providing a much-needed alternative to present treatments such as the administration of exogenous β-adrenergic stimulants that tend to limit long-term survival.

As described herein, a number of different vectors can be employed to deliver the AC transgene *in vivo* according to the present invention. By way of illustration, the replication-defective recombinant adenovirus vectors exemplified resulted in highly efficient gene transfer *in vivo* without cytopathic effect or inflammation in the areas of gene expression.

Compositions or products for use in the invention may conveniently be provided in the form of formulations suitable for administration into the blood stream (e.g. in an intracoronary artery). A suitable administration format may best be determined by a medical practitioner for each patient individually, according to standard procedures. Suitable pharmaceutically acceptable carriers and their formulation are described in standard formulations treatises, e.g., Remington's Pharmaceuticals Sciences by E.W. Martin. See also Wang, Y.J. and Hanson, M.A. "Parental Formulations of Proteins and Peptides: Stability and Stabilizers," Journals of Parental Sciences and Technology, Technical Report No. 10, Supp. 42:2S (1988). Vectors of the present invention should preferably be formulated in solution at neutral pH, for example, about pH 6.5 to about pH 8.5, more preferably from about pH 7 to 8, with an excipient to bring the solution to about isotonicity, for example, 4.5% mannitol or 0.9% sodium chloride, pH buffered with art-known buffer solutions, such as sodium phosphate, that are generally regarded as safe, together with an accepted preservative such as metacresol 0.1% to 0.75%, more preferably from 0.15% to 0.4% metacresol. Obtaining a desired isotonicity can be accomplished using sodium chloride or other pharmaceutically acceptable agents such as dextrose, boric acid, sodium tartrate, propylene glycol, polyols (such as mannitol and sorbitol), or other inorganic or organic solutes. Sodium chloride is preferred particularly for buffers containing sodium ions. If desired, solutions of the above compositions can also be prepared to enhance shelf life and stability. Therapeutically useful compositions of the invention can be prepared by mixing the ingredients following generally accepted procedures. For example, the selected components can be mixed to produce a concentrated mixture which may then be adjusted to the final concentration and viscosity by the addition of water and/or a buffer to control pH or an additional solute to control tonicity.

For use by the physician, the compositions can be provided in dosage form containing an amount of a vector of the invention which will be effective in one or multiple doses to induce AC transgene delivery/expression at a desired level. As will be recognized by those in the field, an effective amount of therapeutic agent will vary with many factors moulding the age and weight of the patient, the patient's physical condition, and the level of enhancement of cardiac function desired, and other factors.

For viral vectors, the effective does of the compounds of this invention will typically be in the range of at least about 10⁷ viral particles, preferably about 10⁹ viral particles, and more preferably about 10¹¹ viral particles. The number of viral particles may, but preferably does not exceed 10¹⁴. As noted, the exact dose to be administered is determined by the attending clinician, but is preferably in I ml phosphate buffered saline.

The presently most preferred mode of administration in the case of heart disease is by intracoronary injection to one or both coronary arteries (or to one or more saphenous vein or internal mammary artery grafts or other conduits) using an appropriate coronary catheter. A variety of catheters and delivery routes can be used to achieve intracoronary delivery, as is known in the art. For example, a variety of general purpose catheters, as well as modified catheters, suitable for use in the present invention are available from commercial suppliers such as Advanced Cardiovascular Systems (ACS), Target Therapeutics and Cordis. Also, where delivery to the myocardium is achieved by injection directly into a coronary artery (which is presently most preferred), a number of approaches can be used to introduce a catheter into the coronary artery, as is known in the art. By way of illustration, a catheter can be conveniently introduced into a femoral artery and threaded retrograde through the iliac artery and abdominal aorta and into a coronary artery. Alternatively, a catheter can be first introduced into a brachial or carotid artery and threaded retrograde to a coronary artery. Detailed descriptions of these and other techniques can be found in the art (see, e.g., the references cited above, including: Topol, EJ (ed.), The Textbook of Interventional Cardiology, 2nd Ed. (W.B. Saunders Co. 1994); Rutherford, RB, Vascular Surgery, 3rd Ed. (W.B. Saunders Co. 1989); Wyngaarden JB et al. (eds.), The Cecil Textbook of Medicine, 19th Ed. (W.B. Saunders, 1992); and Sabiston, D, The Textbook of Surgery, 14th Ed. (W.B. Saunders Co. 1991)).

The following Examples are provided to further assist those of ordinary skill in the art. Such examples are intended to be illustrative and therefore should not be regarded as limiting the invention. A number of exemplary modifications and variations are described in this application and others will become apparent to those of skill in this art. Such variations are considered to fall within the scope of the invention as described and claimed herein.

### EXAMPLES

### EXAMPLE 1: General Methods

### Example 1-1: Animals and Surgical Procedures

Animal use was in accordance with NIH guidelines. Sixteen Yorkshire pigs (*Sus scrofa*) weighing 42±5 kg were used for this study, and were anesthetized with ketamine (50 mg/kg, im) and atropine sulfate (0.1 mg/kg, im) followed by sodium amytal (100 mg/kg, iv). After endotracheal intubation, halothane (0.5-1.5%) was delivered by a pressure-cycled ventilator throughout the procedure. At left thoracotomy, catheters were placed in the aorta, pulmonary artery, and left atrium. A Konigsberg micromanometer was placed into the left ventricular apex and an epicardial unipolar lead was placed 1.0 cm below the atrioventricular groove in the lateral wall of the left ventricle. The power generator (Spectrax 5985; Medtronic Incorporated, Minneapolis, MN) was inserted into a subcutaneous pocket in the abdomen. The pericardium was loosely approximated and the chest closed.

Ten days after thoracotomy, baseline measures of hemodynamics and left ventricular function were made. Ventricular pacing then was initiated (225 bpm). Five of these animals underwent assessment of hemodynamic and ventricular function 96 hours after initiation of pacing and then were killed. Six animals underwent weekly studies of hemodynamics and ventricular function and were killed twenty-eight days after initiation of pacing. The five remaining animals (controls) were not paced and were killed thirty-eight days after instrumentation.

### Example 1-2: Hemodynamic Studies

Hemodynamic data were obtained from conscious, unsedated animals after the pacemaker had been inactivated for 1 hr and animals were in a basal state. Pressures were obtained from the left atrium and aorta. Left ventricular dP/dt was obtained from the high fidelity left ventricular pressure.

### Example 1-3: Echocardiographic Studies

Two-dimensional and M-mode images were obtained using a Hewlett Packard Sonos 1500 imaging system. Images were obtained from a right parastemal approach at the mid-papillary muscle level and recorded on VHS tape. Measurements were made using criteria from the American Society of Echocardiography (Sahn DJ, et al., Circulation 58: 1072-1083, 1978). End-diastolic dimension (EDD) and end-systolic dimension (ESD) were measured on at least 5 beats and averaged. End-diastolic dimension was obtained at the onset of the QRS complex. End-systolic dimension was taken at the instant of maximum lateral position of the interventricular septum, or at the end of the T wave. Left ventricular systolic function was assessed using fractional shortening [(EDD-ESD)/EDD] X 100. The coefficient of variation for end-diastolic dimension on repeated measurements was <5%. All of these measurements were obtained with pacemakers inactivated.

### Example 1-4: Terminal Thoracotomy

After four days (n=5) or twenty-eight days (n=6) of continuous pacing (or a similar post-operative duration without pacing for the five control animals), pigs were anesthetized, and midline sternotomies made. Hearts were removed, rinsed in sterile saline (4°C), and the coronary arteries perfused with sterile saline (4°C). Transmural samples of the left ventricular free wall were taken mid-way from base to apex, near the midportion of the left anterior descending coronary artery. Myocardial samples were then frozen (-80°C). Time from heart removal to placing samples in liquid nitrogen was 5-10 min.

### Example 1-5: Membrane Assessment and Preparation

Frozen transmural samples (-80°C) were powdered in a stainless steel mortar and pestle (also -80°C), placed in Tris buffer, glass-glass homogenized, and contractile proteins extracted (0.5 M KCl, 20 min, 4°C). The pellet of a 45,000xg centrifugation was resuspended in buffer. Protein concentrations were determined by the method of Bradford (Bradford MM, Anal. Biochem, 72: 248-254,1976); the protein yield (mg protein/mg wet weight) was assessed in all preparations. We have previously shown that the activity of p-nitrophenyl-phosphatase (Bers DM, Biochem Biophys Acta, 555: 131-146, 1979), a sarcolemmal membrane-associated enzyme used to assess membrane protein yield per mg crude membrane homogenate, is not altered in this model (Roth DA, et al., J. Clin Invest, 91: 939-949, 1993).

### Example 1-6: Adenylylcyclase Assays

Methods for measuring AC activity were modified from Salomon (Salomon Y, et al., Anal Biochem, 58: 541-548, 1974) as previously reported (Hammond HK, et al., Circulation 85: 269-280, 1992; Hammond HK, et al., Circulation 8:, 666-679, 1992). The following agents were used to stimulate cAMP production (final concentrations): isoproterenol (10 µM), GTP (10 µM), Gpp[NH]p (100 µM), forskolin (100 µM). We found that cAMP production under these conditions was linear with respect to time and protein concentration, and that 3-isobutyl, 2-methylxanthine (1.0 mM), adenosine deaminase (5 U/ml), or both, had no effect on basal or maximally stimulated cAMP production. Previous experiments established that AC activity does not distribute to the supernatant of a 45,000 x g centrifugation in our membrane preparation (Hammond HK, et al., Circulation 85: 269-280,1992).

### Example 1-7: β-Adrenergic Receptor Binding Studies

As previously described (Hammond HK, et al., Circulation 8:, 666-679, 1992) βARs were identified using the radioligand [¹²⁵I]-iodocyanapindolol. Agonist affinity was determined by performing competitive binding assays using (-)isoproterenol (Hammond HK, et al., Circulation 8:, 666-679, 1992).

### Example 1-8: Assessment of Gsα and Giα₂ Content by Immunoblotting

Assessment of αs and αi subunits of Gs and Gi, respectively, was conducted using standard SDS-PAGE and immunoblotting techniques as previously described (Roth DA, et al., FEBS Lett, 29: 46-50, 1992). Briefly, 100 µg ofprotein from each supernatant and pellet fraction of a 45,000xg centrifugation of crude myocardial homogenate derived from appropriate transmural samples was electrophoresed on a 10% denaturing gel for 4 hours at 30 mA. Proteins were electroblotted onto nitrocellulose membranes (Amersham, U.K.) for 14 hours, 70 volts, 4°C. Transfer efficiency was recorded by photocopies of membranes dyed with reversible Ponceau staining, and gel retention checked with Coomasie Blue staining. Background blocking was accomplished by incubating membranes in Tris-buffered saline (TBS, pH 7.5) with 2% non-fat dry milk, 2 hours, 25°C. Purified primary polyclonal antibodies (NEN, Boston MA, rabbit Anti-G-proteins: RM/1 Gsα; AS/7, transducin, Giα₁, Giα₂) were diluted 1:600 in 15 ml of TBS with 0.05% Tween-20 (TTBS, pH 7.5) and 1% non-fat dry milk, and membranes incubated for 14 hours, 4°C. Autoradiographic detection of bands was performed by incubating membranes in 75 ml TTBS with I % non-fat dry milk and 15 x 10⁶ cpm ¹²⁵I-Protein A (NEN, Boston, MA) for 2 hours, 25°C followed by thorough sequential washes in TTBS, and placing against X-ray film (Kodak X-OMAT AR) for 5 days, -70°C. The 45 and 40 kDa bands for Gsα and Gsα₂ were removed from the membranes with background controls for gamma counting.

### Example 1-9: Assessment of GRK₂ and GRK₅ Content by Immunoblotting

Assessment of left ventricular GRK₂ and GRK₅ content was conducted using standard SDS-PAGE and immunoblotting techniques as previously reported (Ping P, et al., J. Clin Invest, 95: 1271-1280, 1995; Roth DA, et al., FEBS Lett, 29: 46-50, 1992). An antibody specific for purified bovine GRK₂ and purified bovine GRK₂ were provided by Dr. Jeffrey L. Benovic. An antibody specific for GRK₅ was obtained from Santa Cruz Biotechnology (Santa Cruz, CA). Transmural left ventricular samples were placed in a lysis buffer containing 20 mM Tris HCl (pH 7.5), 2 mM EDTA, 10 µg/ml benzamidine, 10 µg/ml leupeptin, 100 µg/ml PMSF and 5 µg/ml pepstatin A. Powdered samples were homogenized then centrifuged and resuspended by sonication in lysis buffer. Eighty µg of protein from each left ventricular sample was mixed with Laemmli buffer and boiled, then electrophoresed on a 10% denaturing gel. Proteins were transferred to PVDF paper (Immobolin-P, Millipore); transfer efficiency was determined by Ponceau staining. The membrane was blocked for 2 hr in Tris-buffered saline containing 0.1% Tween-20 and 5% non-fat dry milk and developed by conventional methods using GRK₂ or GRK₅ antiserum followed by exposure to horseradish peroxidase-lined anti-rabbit immunoglobulin (1:1000 in TBS). The blots were developed by the ECL method and bands were visualized after exposing blots to X-ray film. Densities of bands co-migrating with purified bovine GRK₂ were quantified by densitometric scanning; for GRK₅, we quantified the GRK₅-specific band migrating at approximately (68 kD).

### Example 1-10: G-Protein Receptor Kinase Activity

GRK enzymatic activity was determined using light-dependent phosphorylation of rhodopsin (Benovic JL, Methods Enzymology, 200: 351-363, 1991) as we have previously reported (Ping P, et al., J. Clin Invest, 95: 1271-1280, 1995). No GRK subtype-specific activity assay is available so phosphorylation of rhodopsin reflects activity of GRK₂ (βARK₁) and GRK₅, the predominant GRK isoforms in the heart (Inglese J, et al.,.J Biol Chem, 268: 23735-23738, 1993). We purified rhodopsin from rod outer segments obtained from dark-adapted calf retina. Light-dependent phosphorylation of purified rhodopsin was first tested by using recombinant GRK₂ (a gift from Dr. J. Benovic). One gram of left ventricle was homogenized in 9 ml lysis buffer (50 mM Tris-HCL, pH 7.5, 5 mM EDTA, 10 µg/ml benzamindine, 20 µg/ml leupeptin, 40 µg/ml PMSF and 5 µg/ml pepstin A), then centrifuged at 45,000 x g for 30 minutes. The pellet was resuspended in 4 ml of lysis buffer with 250 mM NaCl (used to dissociate membrane-associated GRK) and homogenized again in a power-driven glass rotor (4°C). The pellet suspension was then re-centrifuged and ion exchange columns (Amicon) were used to remove NaCl in the supernatant of the pellet suspension. Both the supernatant and the supernatant of the pellet suspension then underwent DEAE-Sephacel column purification to eliminate endogenous kinases that could contaminate GRK-dependent phosphorylation (Ping P, et al., J Clin Invest 95: 1271-1280, 1995; Ungerer M, et al., Circulation, 87: 454-461, 1993). Both supernatant and pellet fractions were independently column-purified.

GRK-dependent phosphorylation was measured by incubating 100 µg protein from either fraction with 250 pmol rhodopsin in buffer containing 18 mM Tris HCl, 1.8 mM EDTA, 4.8 mM MgCl₂, 73 µM ATP, and 2.9 cpm/fmol [³²P]-ATP. The GRK-dependent phosphorylation reaction was confirmed by adding protein kinase A inhibitor (1 µM) and heparin (10 µg/ml) into the reaction. Protein concentration for both pellet and supernatant were determined before and after DEAE-Sephacel purification and the final enzyme activity was expressed as pmol phosphate/min/mg of protein as well as per gram of tissue. We have previously shown that 45,000xg centrifugation (30 min) provides a supernatant which contains less than 1% of the total cellular activity of p-nitrophenylphosphatase (a sarcolemmal membrane-associated enzyme), suggesting excellent separation of cytosolic from membrane components (Roth DA, et al., FEBS Lett, 29: 46-50, 1992).

### Example 1-11: RNA Extraction

Total RNA was extracted from left ventricle using a modification of the acid guanidinium thiocyanate-phenol-chloroform extraction method (Chomczynski P, et al., Anal Biochem, 162: 156-159, 1987) as previously described (Ping P, et al., Am J Physiol, 267: H2079-H2085, 1994; Ping P, et al., J Clin Invest 95: 1271-1280, 1995). Tissue samples were homogenized in 4.0 M guanidinium buffer, extracted twice with acidic phenol-chloroform, and precipitated with isopropanol. The final pellet was washed with 70% ethanol, dissolved in diethylpyrocarbonate-treated water and stored at -80°C. The integrity and purity of the RNA were assessed by gel electrophoresis and the ultraviolet absorbance ratio (260 nm, 280 nm); the sample was rejected if the ratio was less than 1.5, or if visual inspection of the gel photograph suggested degradation.

### Example 1-12: Polymerase Chain Reaction Cloning

AC isoforms in porcine left ventricle were isolated by the polymerase chain reaction (PCR). Porcine heart RNA was isolated and reverse-transcribed into cDNA using AMV (avian myeloblastosis virus) reverse transcriptase (Life Science Incorporated, St. Petersburg, Florida). Degenerate primers spanning a total of 207 bp of the putative nucleotide binding region of the AC gene family (Krupinski J, et al., J Biol Chem, 267: 24858-24862, 1992) were used to amplify the porcine heart cDNA AC genes. The primer sequences used included:
(1) 5'-ACGTAGAATTCGG(AG)GA(CT)TGTTA(CT)TACTG-3' (sense)
(2) 5'-ACGTTAAGCTTCCA(GC)AC(AG)TC(AG)AA(CT)TGCCA-3' (antisense)

Complementary DNA derived from 5 µg of total RNA was amplified with 4 µM PCR primers in 10 mM Tris-HCl and 50 mM KCl reaction buffer (pH 8.3). Taq DNA polymerase (2.5 units) was used (Gibco BRL). The amplification reaction was run for 30 cycles at 95°C (2 min), 52°C (2 min), and 72°C (2 min), followed by extension at 72°C for 10 min.

### Example 1-13: Construction of Porcine Riboprobes

The PCR fragments obtained from the above reaction were subcloned into pGEM 4Z vectors (Promega) and sequenced. Among the twenty-eight clones sequenced, sixteen type VI, eleven type V, and one type II AC clones were identified based on the published sequences from the rat (Krupinski J, et al., J Biol Chem, 267: 24858-24862, 1992). Porcine and rat AC types II and VI have identical predicted amino acid sequences. Porcine AC type V differs from rat AC type V only at a single amino acid. These three porcine.' C isoforms share 90% homology with the rat at the nucleotide level (Krupinski J, et al., J Biol Chem, 267: 24858-24862, 1992).

The three sequenced AC isoform plasmids were linearized with either HindIII to generate the control RNA or with EcoRl to generate the antisense riboprobes for AC types II, V, and VI. In vitro transcription was then performed using either SP6 or T7 polymerase (Promega) to generate the control RNA (214 bp) or antisense riboprobes (219 bp) for subsequent RNase protection assays. Riboprobes synthesized in vitro contain the complementary sequences of both the mRNA and the pGEM 4Z vector, and are therefore longer than the protected band fragment from the porcine RNA sample (mRNA only). The protected band from porcine heart is sized at 207 bp. The longer length of the control RNA, also derived from the extra sequence from the pGEM 4Z vector, protects the in vitro, synthesized control mRNA from contamination by sample RNA.

### Example 1-14: RNase Protection Assay

RNase protection assays methods were performed as described previously (Ping P, et al., Am J Physiol, 267: H2079-H2085, 1994; Ping P, et al., J Clin Invest 95: 1271-1280, 1995). *In vitro* transcription was carried out to synthesize [³²P]-labeled riboprobes with specific activities ranging from 1 x 10⁸ to 5 x 10⁹ cpm/µg, using the gene constructs described above. Total RNA (20 µg) from tissue and various amounts of *in vitro* synthesized sense strand control RNA were hybridized with 2-8 x 10⁴ cpm probe (in 5-8 fold excess of mRNA as determined in preliminary experiments) in 20 T1 of 80% formamide, 40 mM Hepes (pH 7.6), 400 mM NaCl, and 1.0 mM EDTA for 12-16 hours (45°C). Digestion buffer (300 T1) containing 300 mM NaCl, 10 mM Tris-HCL (pH 7.4), 5 mM EDTA, and 20 µg RNase A and 3 units of T1 RNase per µg total RNA was then added and incubated for 30 minutes (37°C). After treatment with proteinase K and extraction with phenol-chloroform, the RNAse resistant hybrids were precipitated and run on a 6% polyacrylamide urea gel. The AC_{II}, AC_{V}, and AC_{VI} mRNA signals were quantitated by counting the excised gel band with a β-counter. After counting, the cpm of control RNA could be expressed as cpm/µg of control RNA. These data then were used to quantitate mRNA levels in myocardial tissue (pmol specific mRNA/g total RNA). The cardiac content of AC_{II}, AC_{V}, and AC_{VI} mRNA were calculated from the ratio of their signal to the signal from their sense strand control RNA in the same hybridization reaction. A mammalian 18S riboprobe (400 cpm; plasmid construct from Ambion) was used together with AC_{II}, AC_{V}, and AC_{VI} riboprobes in the hybridizations to assess the loading and hybridization conditions for each tissue sample. The high yield and very low specific activity of the 18S riboprobe (5-8 x 10⁴ cpm/µg; Megascript, Ambion) was obtained to assure accurate measurement of the 18S transcript from 20 µg of total RNA. RNase resistance (<1%) and riboprobe specificity were confirmed by complete digestion of single stranded antisense riboprobe plus 40 µg of transfer RNA (yeast) with RNase A and T1.

### Example 1-15: Northern Blot Analysis of GRK₂

A mouse GRK₂ (βARK₁) cDNA fragment provided by Dr. P.A. Insel was used to assess GRK₂ mRNA content in left ventricular samples. Twenty micrograms of total RNA was gel denatured and blotted onto nylon membranes. The Northern blot was hybridized with a [³²P]-labeled random primer GRK₂ cDNA fragment (bp 1134-1688) for 24 h at 42°C in a buffer containing 5X SSPE, 10X Denhardt's solution, 100 µg/ml salmon sperm DNA, 50% formamide, and 2% SDS. The blot was washed with 2X SSC/0.1% SDS at 27°C for 15 min.

### Example 1-16: Northern Blot Analysis of GRK₅

A human GRK₅ cDNA probe (Marzo KP, et al., Circ Res, 69: 1546-1556, 1991) provided by Dr. J. Benovic was used to assess GRK₅ mRNA content in left ventricular samples. Twenty micrograms of total RNA was gel denatured and blotted onto nylon membrane. The Northern blot was hybridized with a [³²P]-labeled random primer GRK₅ cDNA fragment (bp 383-1540) for 24 h at 42°C in a buffer containing 5X SSPE, 10X Denhardt's solution, 100 µg/ml salmon sperm DNA, 50% formamide, and 2% SDS. The blot was washed with 2X SSC/0.1% SDS at 27°C for 30 min, followed by a high stringency wash with 0.12X SSC/0.1% SDS at 42°C for 30 min,

### Example 1-17: Isolated Cardiac Myocytes

Adult porcine cardiac myocytes were obtained by collagenase perfusion of the coronary artery; and cell viability, purity and yield established as previously described (Spinale FG, et al., Circ. Res. 69:1058-1067,1991). Cardiac myocytes, devoid of other cell line contaminants, were frozen and subsequently used for RNA extraction. RT-PCR using the AC isoform primers described above was performed. AC isoforms were identified by hybridization (Southern blot) with specific AC_{II}, AC_{VI}, and AC_{VI} isoform probes. These probes were cloned and sequenced from porcine heart cDNA as described above. Probe specificity was tested and no cross hybridization occurred between AC_{II}, AC_{V}, and AC_{VI} isoform probes.

### Example 1-19: Statistical Analysis

For indication of statistical significance, data are generally expressed as mean ±1 SD. Specific measurements were compared using repeated measures analysis of variance; *post hoc* tests were performed using Student's t-test, with the Bonferroni correction for multiple comparisons between group means. The null hypothesis was rejected when p< 0.05.

### EXAMPLE 2: A Large Animal Model of Congestive Heart Failure

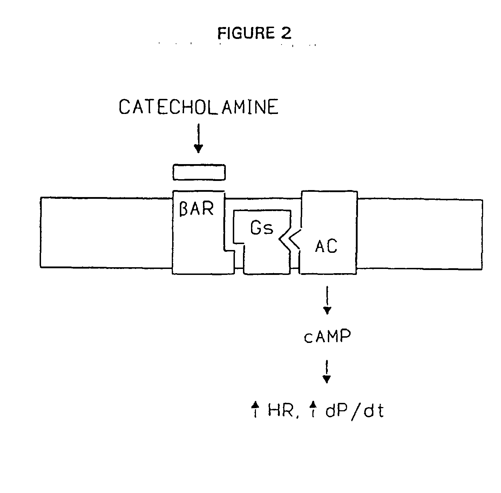

The means by which cardiac myocytes respond to biological signals from the internal environment is through strategically placed cell surface receptors. Principal among these receptors is the β-adrenergic receptor. The transduction pathway by which a hormone or neurotransmitter interacting with a β-adrenergic receptor on the cell surface alters intracellular behavior is known as the β-adrenergic-Gs-adenylylcyclase (or "βAR:Gs:AC") pathway as shown in FIGURE 2. It is through this pathway that β-adrenergic stimulation increases intracellular cAMP thereby influencing heart rate responsiveness and force of contraction. The pathway includes three principle components: the β-adrenergic receptor (βAR), the stimulatory GTP-binding protein (Gs) and adenylylcyclase (AC). The molar stoichiometry of components within the βAR:Gs:AC pathway in rat ventricular myocytes, and presumably cardiac myocytes of other mammals, is believed to be about 1:70:1 respectively (see, e.g., Alousi, et al., FASEB J 5:2300,1991). β-adrenergic signaling within myocardial tissue is initially mediated by agonist binding to βAR, followed by G₅-mediated signal transduction to AC. Activated AC then catalyzes the synthesis of cyclic AMP, and increased intracellular concentrations of cAMP mediate increased cytosolic calcium transients which enhance both the rate and force of cardiac contraction (referred to as positive "chronotrophy" and positive "inotrophy," respectively.

We used a large animal model predictive of humans to examine the possibility that one or more components of the βAR:Gs:AC pathway (as shown in FIGURE 2) might effectively limit transmembrane β-adrenergic signaling in cardiac myocytes, and that elevating expression of one or more of the proteins in the pathway might lead to enhanced responsiveness to endogenous β-adrenergic signaling. As exemplary β-ASPs, we have initially focused attention on AC, β-AR, and GRK inhibitors (which indirectly enhance β-AR activity), as described in more detail below.

### Example 2-1: Hemodynamics and Left Ventricular Function

As shown in Table 1, rapid ventricular pacing resulted in increased basal heart rate and mean left atrial pressure four days after the initiation of pacing. Data were obtained from sixteen animals (pacemakers inactivated); values represent mean ± 1 SD. Groups included normal animals that were not paced (Control, n=5), animals with mild heart failure induced by four days of pacing (4d; n=5), and animals with severe heart failure induced by twenty-eight days of pacing (28d, n=6). LAP, mean left atrial pressure; MAP, mean arterial pressure; EDD, end-diastolic pressure; FS%, % fractional shortening. Analysis of variance (repeated measures) was used to determine whether duration of pacing affected a specific variable.

After four days, animals also showed increased end-diastolic dimension, reduced fractional shortening, and diminished left ventricular peak positive dP/dt. These data indicate mild deterioration of left ventricular function four days after the initiation of continuous rapid pacing.

Cardiac conditions worsened considerably after twenty-eight days of pacing (Table 1), providing a model for heart failure in a large animal considered predictive of humans. Additional evidence establishing the predictiveness of this porcine heart failure model for CHF in humans is described herein and in the art (see, e.g., Roth DA et al., J Clin Invest 91:939-949, 1993, and related references as cited above).

**TABLE 1**

| **HEMODYNAMICS AND LEFT VENTRICULAR FUNCTION** | | | | |
|---|---|---|---|---|
| | **CONTROL** | **4d** | **28d** | **p (ANOVA)** |
| **Heart Rate (bpm)** | 100±6 | 131±2^{b} | 157±15^{c,e} | 0.0001 |
| **LAP (mmHg)** | 14±1 | 22±3^{a} | 36±6^{c,e} | 0.0001 |
| **MAP (mmHg)** | 106±5 | 97±13 | 102±14 | 0.48 |
| **EDD (mm)** | 42±2 | 49±3 | 58±6^{c,d} | 0.0002 |
| **FS (%)** | 41±6 | 32±2^{a} | 13±4^{c,f} | 0.0001 |
| **LV dP/dT (mmHg/sec)** | 3043±387 | 2036±582^{b} | 1072±123^{c,d} | 0.0001 |

| | | | | |
|---|---|---|---|---|
| Data were obtained from sixteen animals (pacemakers inactivated); values represent mean ± 1 SD. Groups included normal animals that were not paced (Control, n=5), animals with mild heart failure induced by four days of pacing (4d; n=5), and animals with severe heart failure induced by twenty-eight days of pacing (28d, n=6). LAP, mean left atrial pressure; MAP, mean arterial pressure; EDD, end-diastolic pressure; FS%, % fractional shortening. Analysis of variance (repeated measures) was used to determine whether duration of pacing affected a specific variable. *Post hoc* testing was performed using Student's t-test with the Bonferroni correction: ^{a}p<0.05; ^{b}p<0.01; ^{c}p<0.001 (vs control); ^{d}p<0.05; ^{e}p<0.01; ^{f}p<0.001 (4d vs 28d). | | | | |

### Example 2-2: Necropsy

In animals paced for four days, ascites was present (mean amount: 150 ml; range 0-500 ml). Liver to body weight ratios, compared to previously reported weight-matched control pigs (Roth DA, et al., J. Clin Invest, 91: 939-949, 1993), increased after four days of pacing (Control: 18±3 g/kg, n=15; 4d: 25±2 g/kg, n=5; p<0.0001). Therefore, four days of pacing caused a modest increase in systemic congestion. Left ventricular to body weight ratios, compared to previously reported weight-matched controls (Roth DA, et al., J. Clin Invest, 91: 939-949, 1993) did not increase (Control: 2.7±0.5 g/kg, n=15; 4d: 3.0±0.4 g/kg, n=5; p=0.24).

After twenty-eight days of pacing, liver to body weight ratios were increased twofold (p<0.0001) and left ventricle to body weight ratios were unchanged, as previously reported (Roth DA, et al., J. Clin Invest, 91: 939-949, 1993).

### EXAMPLE 3: The Role of Adenylylcyclase as a β-Adrenergic Signaling Protein in a Large Animal Model of Congestive Heart Failure

The following data indicate that the amount of the exemplary β-ASP adenylylcyclase does in fact impose a limit on β-adrenergic signaling through this pathway in large animal models that are expected to be highly predictive of cardiac function and dysfunction in humans.

### Example 3-1: Adenylylcyclase Activity (cAMP production) After Cardiac Pacing in Pigs

We examined cAMP production to assess the function of adenylylcyclase in left ventricular membranes from normal pigs and from pigs with severe heart failure, using a model of heart failure with very high fidelity to human clinical dilated heart failure (as described by Roth DA, et al., J Clin Invest 91: 939-949, 1993).

After only four days of pacing, isoproterenol-stimulated cAMP production was somewhat reduced (p<0.05) in left ventricular membranes. Forskolin-stimulated cAMP production, an indicator of AC activation independent of β-AR-mediated stimulation, was not significantly affected at this time.

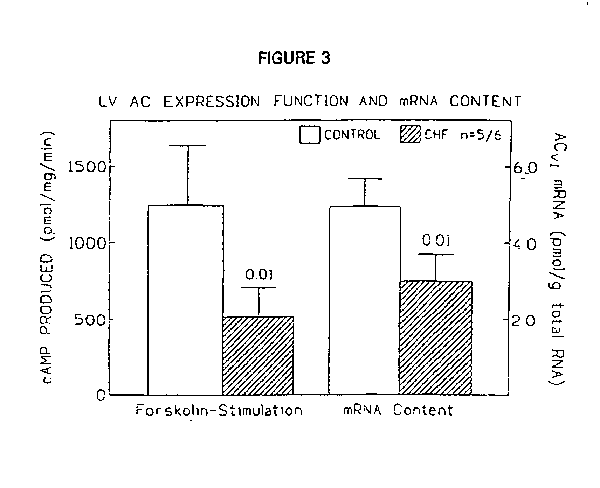

In contrast, twenty-eight days of pacing resulted in substantial reductions in all measures of AC activity. Our results as shown in FIGURE 3 (indicating forskolin-stimulated cAMP production), demonstrated that there was in fact a substantial reduction in activity of the β-ASP adenylylcyclase in association with severe heart failure in these animals.

### Example 3-2: Identification of AC Isoforms in Cardiac Myocyte Samples

RT-PCR was used to amplify a DNA fragment corresponding to the expected size of AC from cardiac myocyte samples obtained from adult pigs. The DNA fragment was absent when reverse transcriptase was omitted from the reaction.

After hybridization with various AC-specific probes, Southern blots revealed that adult porcine myocytes express AC isoforms II, V, and VI.

### Example 3-3: AC mRNA Expression in the Porcine Model

We examined changes in the mRNA levels of AC isoforms (II, V and VI) in normal pigs and in pigs exhibiting heart failure.

After only four days of pacing, there were no significant changes in the mRNA content of the three AC isoforms.

In contrast, as shown in FIGURE 3, after 28 days of pacing in the heart failure model, AC_{VI} mRNA was significantly downregulated (p=0.002), whereas AC_{II} and AC_{V} were relatively unchanged. It remains possible that changes in the latter AC isoforms may have occurred at the protein level without substantial changes in mRNA for those isoforms.

Our observations that forskolin-stimulated cAMP production was relatively unchanged until severe heart failure was present indirectly support the idea that AC protein expression was relatively normal during the initial stages of heart dysfunction. However, our data clearly indicate substantial reductions in all measures of AC activity, and down-regulation of AC_{VI} protein and mRNA, in association with the onset of more severe heart failure.

### EXAMPLE 4: The Role of β-AR and GRK Inhibitors as β-Adrenereic Signaling Proteins in a Large Animal Model of Congestive Heart Failure

As described in the following examples, we also examined the role of β-adrenergic receptor proteins and GRK activity in β-adrenergic signaling in the porcine heart failure model.

### Example 4-1: Left Ventricular β-Adrenergic Receptors and G-Protein Content

Despite alterations in left ventricular function and circulatory congestion, four days of pacing was not associated with significant changes in left ventricular β-AR number, or changes in the stimulatory (Gsa) or inhibitory (Giα₂) GTP-binding proteins. However, four days of pacing did result in a reduced proportion of left ventricular β-ARs exhibiting high affinity agonist binding (p<0.01), suggesting an uncoupling of the β-AR from Gsα.

After twenty-eight days of pacing in the heart failure model, we observed substantial downregulation of left ventricular β-AR number, at a time when both Gsα and Giα₂ are also downregulated (Roth DA, et al., J. Clin Invest, 91: 939-949, 1993).

### Example 4-2: G-Protein Receptor Kinase Activity

Both cytosolic and membrane fractions of left ventricular homogenates were column-purified and light-dependent phosphorylation of rhodopsin was used to measure GRK activity.

After four days of pacing, animals exhibited increases in left ventricular GRK activity that did not appear to undergo further increases after an additional twenty-four days of pacing (Table 2). A substantial portion (40%) of myocardial GRK activity was associated with the sarcolemma and, although total GRK activity increased in conjunction with the onset of left ventricular dysfunction, the cellular distribution of GRK activity was not altered.

**TABLE2**

| **LEFT VENTRICULAR G-PROTEIN RECEPTOR KINASE ACTIVITY** | | | | |
|---|---|---|---|---|
| | **CONTROL** | **4d** | **28d** | **p(ANOVA)** |
| **CYTOSOL** | 21±4 | 30±8^{a} | 28±2^{a} | 0.04 |
| **MEMBRANE** | 15±2 | 18±2 | 21±3^{b} | 0.005 |
| **TOTAL** | 35±5 | 48±9^{b} | 49±2^{b} | 0.01 |
| **% IN CYTOSOL** | 59±3 | 63±12 | 57±7 | 0.52 |

| | | | | |
|---|---|---|---|---|
| G-protein receptor kinase activity in left ventricular membranes. Data from sixteen animals; groups included normal animals that were not paced (Control, n=5), animals with mild heart failure induced by four days of pacing (4d; n=5) , and animals with severe heart failure induced by twenty-eight days of pacing (28d, n=6). Values are mean ± 1 SD and represent ³²P incorporated (fmol/mg/min). Analysis of variance (repeated measures) was used to determine whether duration of pacing affected a specific variable. *Post hoc* testing was performed by Student's t-test with the Bonferroni correction: ^{a}p≤0.05; ^{b}p<0.01 (vs control). | | | | |

### Example 4-3: G-Protein Receptor Kinase₂ Protein Content

A band estimated to be 80 kD, co-migrating with purified bovine GRK₂, was identified with the GRK₂ antibody. LV total GRK₂ protein content was unchanged by mild heart failure (4d pacing) but tended to decrease in LV from animals with severe heart failure (Control: 1.37±0.17 du/µg; 4d: 1.58±0.34 du/µg; 28d: 1.02±0.07 du/µg; p<0.02 by ANOVA). However, post hoc analysis revealed that the only group mean comparisons that were statistically significant was 4d vs 28d (p<0.03). GRK₂ concentration was reduced in the supernatant (p<0.04), but not the pellet fraction of the LV homogenate in severe heart failure.

### Example 4-4: G-Protein Receptor Kinase₅ Protein Content

A band migrating at 68 kD, identifying GRK₅, was detected using the GRK₅ antibody. LV total GRK₅ protein content was increased by mild heart failure (4d pacing) and increased further in severe heart failure (Control: 0.62±0.16 du/µg; 4d: 0.97t0.16 du/µg; 28d: 1.33±0.25 du/µg; p=0.0004 by ANOVA; Control vs 4d, p<0.04; Control vs 28d, p<0.001).

### Example 4-5: G-Protein Receptor Kinase₂ mRNA Content

An mRNA species estimated to be 3.8 kb was identified with the GRK₂ probe. An additional species of 2.4 kb was also noted. LV GRK₂ mRNA content was unchanged by mild heart failure (4d pacing) but was reduced in LV from animals with severe heart failure (Control: 67±11 du; 4d: 66±16 du; 28d: 45±13 du; p=0.02 by ANOVA; Control vs 28d, p=0.02; 4d vs 28d, p<0.03). We were unable to detect GRK₃ (βARK₂) expression either with PCR or by Northern blotting.

### Example 4-6: G-Protein Receptor Kinase₅ mRNA Content

An mRNA species estimated to be 3.0 kb was identified with the GRK₅ probe. LV GRK₅ mRNA content was upregulated within 4d of the initiation of pacing, a finding that persisted for 28d (Control: 54±4 du; 4d: 110±28 du; 28d: 96±17 du; p=0.001 by ANOVA; Control vs 4d: p=0.003; Control vs 28d: p=0.01).

### Example 4-7: β-AR and GRK Inhibitors as β-Adrenergic Signaling Proteins (β-ASPs)

Results obtained in our large animal model of heart failure confirmed that reduced β-AR responsiveness in heart failure is associated with selective down-regulation of myocardial β₁-AR and β₁-AR mRNA levels; β₂-AR expression and mRNA content do not change (see, e.g., Bristow MR et al., J Clin Invest 92:2737-2745, 1993; Ping P, et al., Am J Physiol, 267: H2079-H2085, 1994; Ungerer M, et al., Circulation, 87: 454-461, 1993); and that remaining β-ARs are uncoupled from Gs (the stimulatory GTP-binding protein which links receptor activation with AC stimulation), as reflected by a reduction in high affinity agonist binding (see, e.g., Bristow MR, et al., Mol Pharm, 35: 295-303, 1989; Bristow MR, et al., J Clin Invest, 92: 2737-2745, 1993). Mechanisms for β-AR uncoupling in CHF have not been firmly established. GRK, primarily studied in cells with β₂-ARs, phosphorylates the β₂-AR after adrenergic activation (Hausdorff WP, et al., FASEB J, 4: 2881-2889, 1990; Inglese J, et al., J Biol Chem, 268: 23735-23738, 1993), and may play a role in β-AR desensitization in the setting of sustained sympathetic activation, as occurs in CHF. After β₂-AR stimulation by agonist, GRK₂ translocates from cytosol to sarcolemma, phosphorylates the β₂-AR, and thereby uncouples the β₂-AR and Gs, thus attenuating the signal (Hausdorff WP, et al., FASEB J, 4: 2881-2889,1990; Inglese J, et al., J Biol Chem, 268: 23735-23738, 1993). A role for GRK-mediated phosphorylation of the β₁-AR has recently been demonstrated, a phosphorylation that can be mediated both by GRK₂ and GRK₅ (Freedman NJ, et al., J Biol Chem, 270: 17953-17961, 1995; Koch WJ, et al., Science, 268: 1350-1353, 1995). In addition, chronic reduction of β₁-AR activation (bisoprolol treatment) results in downregulation of GRK activity and enhanced adrenergic signaling, suggesting that the extent of adrenergic activation may influence GRK expression (Ping P, et al., J Clin Invest 95: 1271-1280, 1995). Recent studies have suggested that GRK activity is also increased in the soluble fraction of left ventricular homogenates of failing human hearts (Salomon Y, et al., Anal Biochem, 58: 541-548, 1974; Ungerer M, et al., Circ Res, 74: 206-213, 1994).

Our findings with respect to GRK activity in the porcine heart failure model are also similar to those reported by Ungerer et al (Ungerer M, et al., Circulation, 87: 454-461, 1993), in that left ventricular GRK activity is increased in failing left ventricle. However, there are several aspects of our data which are quite new with respect to GRK expression in the setting of heart failure. First, unlike previous reports, we measured GRK activity in both soluble and particulate fractions. While heart failure does not affect the sarcolemmal/cytosolic distribution of GRK activity, severe heart failure is associated with increased myocardial GRK activity in both fractions. A second new finding is that the increase in total GRK activity was an early event in heart failure, occurring prior to alterations in β-AR number, G-protein content, or AC isoform expression or catalyst activity. To the extent that increased GRK activity may serve to phosphorylate and uncouple Gs and the β-AR, we also have demonstrated a potential biochemical correlate of increased GRK activity. In conjunction with decreased numbers of β-ARs showing high affinity agonist binding, we found reduced hormonal stimulation of cAMP that correlated temporally with increased total GRK activity. Third, total GRK₅ protein and mRNA content were increased, also at this early time point, and these elevations persisted in severe heart failure. These data support the idea that increased myocardial GRK expression predates other changes in adrenergic signaling in heart failure, and thus appears to be an important early event in the pathogenesis. A previous study found increased GRK activity and increased GRK₂ mRNA (using PCR) in explanted failed human left ventricles (Ungerer M, et al., Circulation, 87: 454-461, 1993). However, we found reduced LV GRK₂ mRNA levels by Northern blotting. The discrepancy regarding LV GRK₂ mRNA levels in the present study vs the human studies may reflect different methods employed to assess mRNA, different models employed, or the difficulty in obtaining true control material in the human studies. We also note that GRK₅ was not examined in the cited report. While we do not rule out a role for GRK₂ in severe heart failure, our data support the idea that GRK₅ may play a more important role.

Increased GRK activity was detected in the soluble fraction of LV membranes from animals with mild heart failure, while GRK₂ and GRK₅ protein content were not significantly increased in the soluble fraction. This may indicate a disparity between enzymatic activity and protein content, or may reflect methodological differences in protein distribution in the membrane preparations used for the enzymatic assay vs immunoblotting studies.

The present study documents, for the first time, that myocardial GRK₅ protein and mRNA contents are susceptible to upregulation in mild and severe heart failure. Without wishing to be bound by theory, our data support the idea that increased expression of GRK₅ may be responsible for the increased GRK activity observed in these clinical conditions. In any case, the current data strongly support the hypothesis that increased left ventricular GRK expression contributes to reduced adrenergic signaling at an early stage during the development of heart failure.

### EXAMPLE 5: Construction of a Vector for Gene Delivery of Adenylylcyclase

As an illustration of the construction of a gene delivery vector for use in the present invention, we have prepared constructs using a helper-independent replication-defective viral vector as described above. In particular, in this example, we demonstrate the generation of replication-defective adenoviral constructs based on the human adenovirus-5 system (see, e.g., McGrory WJ, et al., Virology 163: 614-617,1988).

We initially selected an adenylylcyclase protein, in particular adenylylcyclase isoform VI (AC_{VI}). We therefore constructed vectors comprising either AC_{VI} or lacZ (as an illustrative detectable marker gene which encodes β-galactosidase). The system used to generate recombinant adenoviruses (based on these "first-generation" vectors) imposes packaging constraints that are believed to increase as the size of the transgene insert exceeds about 5 kb. In the case of control elements such as a CMV promoter and an SV40 polyadenylation signal (which together comprise approximately 1 kb), the transgene itself (i.e. without additional control elements) would therefore preferably be less than about 4kb. Although smaller transgenes are therefore preferred, we have also shown that substantially larger transgenes can nevertheless be employed, even in these "first generation" vectors. For example, in a first exemplary AC transgene, described in Example 5-1, we used essentially the entire transcribed region from a murine adenylylcyclase gene (approximately 5748 bp), together with a heterologous CMV promoter (approximately 790 bp) and an SV40 polyadenylation signal (approximately 230 bp). As described in detail below, we have shown that such transgenes (including more than 6.7 kb with control elements) can nevertheless be incorporated and effectively used with these first generation vectors.

However, in view of the known packaging constraints of these particular vectors, and as another exemplary AC transgene, we constructed an altered adenylylcyclase gene in which an untranslated region of the transcript was deleted to generate a shorter AC transgene. In the illustrative embodiment described below in Example 5-2, the 3'-untranslated region from the AC_{VI} gene was removed, and the resulting construct was incorporated into a vector for delivery of the transgene to the heart, as described herein. As will be apparent, the desirability of truncating the transgene (in the case of a size-constrained viral vector such as Ad or AAV) depends in part on the length of the native gene, as well as the choice of control elements used. In the case of Ad, where the total insert size exceeds about 5kb, the transgene can be truncated, preferably in the 3'-untranslated region, to result in a shorter insert. As is also apparent, the preferred extent of truncation depends on the insert size, but is typically at least about 100 bp, more preferably at least about 500 bp, still more preferably at least about 1000 bp (particularly if the total insert size is still greater than about 5 kb). We have shown that the entire 3'-untranslated region can be readily deleted from the AC_{VI} isoform, resulting in a substantial shorter transgene. First generation AAV vectors are also size constrained and efficiency decreases with inserts significantly greater than about 5kb. For these and other such vectors, however, "second generation" derivatives can provide additional space for transgene packaging. Other viral vectors, as well as various non-viral vectors, can be used to accommodate substantially larger inserts.

Example 5-3 describes the identification of sequences encoding a human adenylylcyclase gene. As described herein, such human AC transgenes can be obtained by screening of human DNA libraries (using probes from homologous mammalian genes), and such human AC transgenes can be usefully employed in the context of the present invention. In particular, where the therapeutic target is a human heart, it is expected that the use of human AC transgenes can provide additional advantages (including potentially closer coordination with other components of the β-adrenergic signaling pathway, as well as further minimizing the possibility of a host response to non-human proteins). The isolation and sequencing of an exemplary human AC transgene is described below in Example 5-3.

### Example 5-1: Generation of an AC transgene using a full length AC_{VI} cDNA

For generation of a first exemplary AC transgene, we used a cDNA encoding murine AC_{VI} (a Ca(2+)-inhibitable AC from murine NCB-20 cells as reported by Yoshimura M and Cooper DM, Proc Natl Acad Sci (USA) 89: 6716-6720, 1992, referred to in the paper as "pAC-V", now known as "pAC-VI"; see also Krupinski, J., et al., J. Biol. Chem. 267: 24858-24862, 1992). The full length AC_{VI} cDNA was cloned into the polylinker of plasmid ACCMVPLPA (J Biol Chem 267: 25129-25134, 1992) which contains the CMV promoter and the SV40 polyadenylation signal flanked by partial adenovirus sequences from which the E1A and E1B genes, which are essential for viral replication, had been deleted. The resulting plasmid was co-transfected (by lipofection) into human 293 cells with plasmid JM17 (Giordano, et al. Nature Medicine 2: 534-539, 1996) which contains the entire human adenovirus 5 genome as well as an additional 4.3 kb insert (thereby making pJM17 too large to be encapsidated).

Homologous rescue recombination resulted in the generation of recombinant adenovirus vectors containing the transgene (AC_{VI} or lacZ) in the absence of E1A/E1B sequences (as illustrated in FIGURE 1). Although these recombinants were nonreplicative in normal mammalian cells, they can be propagated in human 293 cells which had been transformed with E1A/E1B (and therefore provided these essential replication gene products *in trans*).

Transfected human 293 cells were monitored for evidence of cytopathic effect which usually occurred 10-14 days after transfection. To identify successful recombinants, cell supernatant from plates showing a cytopathic effect was treated with proteinase K (50 mg/ml with 0.5% sodium dodecyl sulfate and 20 mM EDTA) at 56 degrees C for 60 minutes, followed by phenol/chloroform extraction and ethanol precipitation. Successful recombinant viral vectors were then identified by PCR using primers complementary to the CMV promoter and SV40 polyadenylation sequences to amplify the insert, and primers designed to concomitantly amplify adenovirus sequences (as in Biotechniques 15:868-872, 1993).

Successful recombinant viral particles were then subjected to two rounds of plaque purification. Viral stocks were further propagated in human 293 cells to titers ranging between 10¹⁰ and 10¹² viral particles, and were purified by double CsCl gradient centrifugation prior to use following standard procedures. Briefly, cells were infected at 80% confluence and harvested at 36-48 hours; and, after freeze-thaw cycles, the cellular debris was collected by standard centrifugation and the virus further purified by double CsCl gradient ultracentrifugation (discontinuous 1.33/1.45 CsCl gradient; cesium prepared in 5 mM Tris, 1 mM EDTA (pH 7.8); 90,000 x g (2 hr), 105,000 x g (18 hr)).

Prior to *in vivo* injection, the viral stocks were typically desalted by gel filtration through Sepharose columns such as G25 Sephadex. The resulting viral stock had a final viral titer in the 10¹⁰-10¹² viral particles range. The viral preparation was found to be highly purified, with essentially no replicative virus present (as determined by an absence of cytopathic effect after transfection of the vector into human host 293 cells).

### Example 5-2: Generation of a second AC transgene using a truncated adenylylcyclase gene

As another illustrative AC transgene, we constructed an altered adenylylcyclase gene in which an untranslated region of the normal transcript was removed to generate a shorter β-ASP transgene. In particular, we essentially removed the 3'-untranslated region from an AC_{VI} construct, and incorporated the resulting truncated transgene into a viral vector for gene delivery.

### Plasmid Construction and Recombinant Adenovirus Production. A murine

AC_{VI} cDNA without the 3'-untranslated region was constructed as follows. A subfragment containing the 3' portion of AC_{VI} with an Xho I site at its 5' end was generated using two PCR primers: AC_{VI}PX, which anneals to AC_{VI} cDNA at bases 2500-2521; and AC_{VI}p3'HA which contains sequences from the 3' end of AC_{VI} and sequences from human influenza hemoagglutinin. The PCR product was digested with restriction enzymes Xho I and Xba I which were designed in the primers at the 3' end. The 5' portion of the AC_{VI} fragment (base pair -92 to +2500) was obtained by Eco RI and Xho I digestion of the full length cDNA (as described above) and was isolated on an agarose gel. Two AC_{VI} fragments were then subcloned into an Eco RI-Xba I digested adenovirus vector (pAd5CI, a gift of Dr. Swang Huang, The Scripps Research Institute, La Jolla, CA) by three molecule ligation. The pAd5/CI vector contains a cytomegalovirus immediate-early enhancer/promoter region (CMV promoter), a chimeric intron, and a multicloning site derived from pCI plasmid DNA (Promega, Madison, WI). It also contains a bovine growth hormone polyadenylation (poly A) sequence and partial human adenovirus-5 sequences. Introduction of the two AC_{VI} fragments into the EcoRI-XbaI digested plasmid, as noted above, resulted in the generation of plasmid pADV5/AC_{VI} which is depicted in FIGURE 1B (abbreviations: AC VI -- Adenylylcyclase VI; CMVpCytomegalovirus immediate-early enhancer/promoter region; HA-tag -- Hemagglutinin protein of influenza virus - tag; BGH-poly A -- Bovine growth hormone-poly A; Adv5Adenovirus 5).

Following procedures as described above, the AC_{VI}-containing vector was cotransfected (calcium phosphate) into a human embryonal kidney cell line (H293) with pJM17 which contains the adenovirus genome except the E1 region. After recombination, plaques were selected and expanded in H293 cells. H293 cells have been transformed with adenovirus E1, and therefore provide this viral transcription factor *in trans.* Expression of AC_{VI} was examined by RT-PCR and Western blot analysis. Virus was purified by cesium chloride ultracentrifugation and desalted by column filtration through Sephadex G-25 equilibrated with PBS as described above. The viral concentration was determined by optical densitometry at OD₂₆₀. Plaque-forming units (pfu) were assayed by plaque titration using H293 cells overlaid with agarose-DMEM medium.

**Identification of AC**_{**VI**} **Expressing Clones.** To identify adenovirus clones that expressed AC_{VI}, sixteen clones were screened by RT-PCR using a pair of specific primers (AC_{VI}PX and AC_{VI}p3'HA) which hybridize to transgene AC_{VI} but not endogenous AC_{VI} in H293 cells. The 512-bp RT-PCR product was confirmed by digestion with the restriction enzyme Apa I to produce 312-bp and 200-bp fragments. Three of sixteen clones expressed AC_{VI} mRNA. Expressed as fold increase in cAMP production, all three clones showed increased cAMP production in response to stimulation by isoproterenol (Clone 1, 22-fold increase; Clone 2, 15-fold increase; Clone 3, 12-fold increase) and forskolin (Clone 1, 16-fold increase; Clone 2, 11-fold increase; Clone 3, 13-fold increase). Clone 1 was selected for additional analyses using cardiac myocytes as described in Example 8-2.

### Example 5-3: Generation of a third AC transgene

As discussed above, AC genes exist in many mammalian tissues, with different isoforms typically predominating in certain tissue types. There tends to be a predominance of isoforms II, V and VI in cardiac tissues. Such genes also tend to exhibit a fairly significant degree of sequence conservation between different mammals, particularly with respect to the isoforms found in a specific tissue such as the heart. DNA hybridization and associated molecular biological techniques can thus be employed to identify additional AC transgenes for use in the context of the present invention.

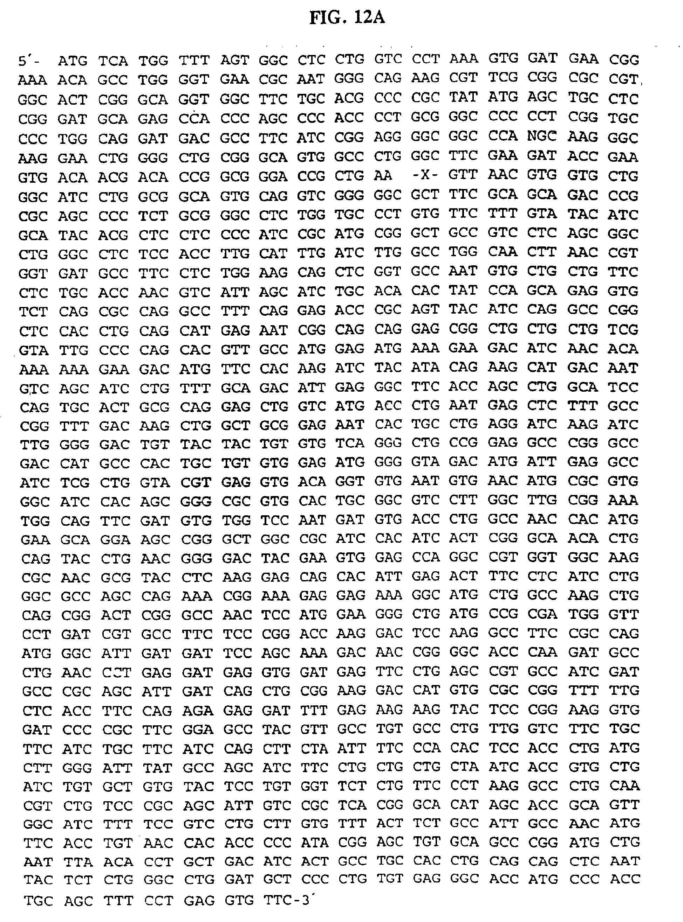

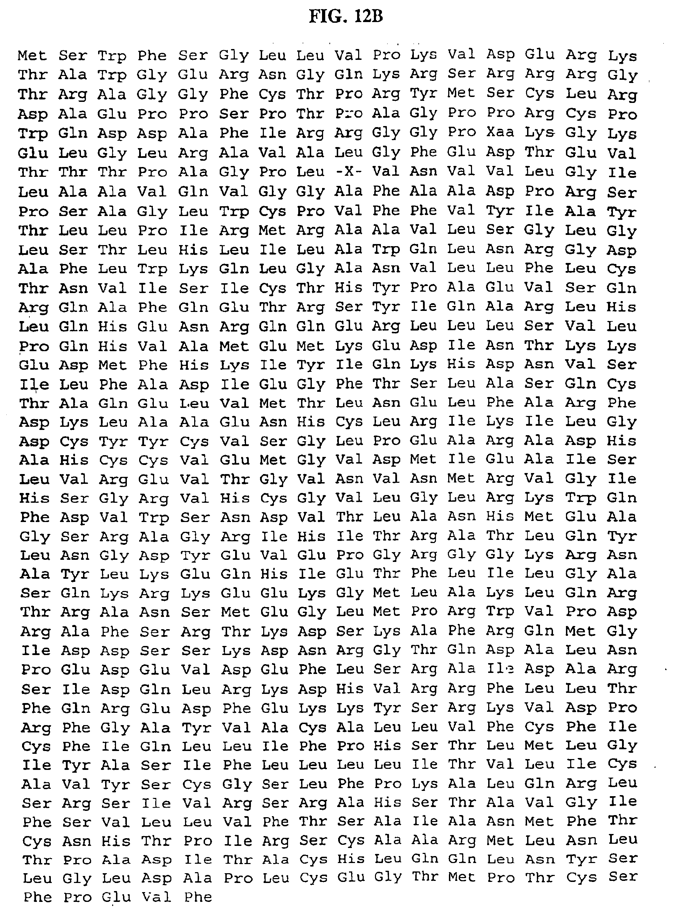

By way of illustration, we have identified clones in a human heart cDNA library that were homologous to a murine AC_{VI} cDNA fragment as a means of obtaining the human AC_{VI} gene. Identification of the human sequence will thus permit the use of the corresponding human AC transgene according to the methods described herein. Briefly, a human heart cDNA library (commercially available from Clontech, #HL3026b) was screened with an SphI fragment of about 1.9kb from the murine AC_{VI} cDNA using standard molecular biological techniques as described in the references cited above. Six positive clones were identified in the primary screen and confirmed in secondary and tertiary screens. Three of these clones (designated clones 1, 4 and 5) were sub-cloned into a vector for sequencing. We employed the "Bluescript" vector pBS-SK (commercially available from Stratagene). The first round of sequencing was carried out using T3 and T7 primers, and then internal primers were employed for subsequent sequencing. All three of the clones contained sequences that were highly homologous to AC_{VI} genes of other species including the mouse. These clones, and sub-fragments thereof, were used to identify overlapping clones containing the remaining sequence. From the overlapping clones we obtained the nucleotide sequence shown in Figure 12A, which corresponds to more than 2 kb of the presumed 3.4kb coding sequence of human AC_{VI}. The -X- indicates a gap of about 0.5 kb within the sequence shown. Figure 12B shows the amino acid sequence corresponding to the nucleotide sequence depicted in Figure 12A. From the sequence information provided in Figure 12A, the complete nucleotide sequence encoding the full length human AC_{VI} and variants thereof can be readily obtained using standard recombinant DNA methodology.

Polynucleotides comprising closely related sequences can likewise be obtained, using techniques such as hybridization, as is known in the art. Such sequences would include, for example, those exhibiting at least least 95%, even more preferably at least 99% sequence identity with a nucleotide sequence comprising that shown in Figure 12A. Isolated polynucleotides that hybridize at high stringency to a polynucleotide having the nucleotide sequence of Figure 12A can thus be readily obtained based on standard molecular biological techniques. These polynucleotides can also be used to obtain isolated polypeptides encoded by the polynucleotides. As used in this context, an "isolated polypeptide" or protein is a polypeptide or protein which has been substantially separated from any cellular contaminants and components naturally associated with the protein in vivo. The phrase embraces a polypeptide which has been removed from its naturally occurring environment, and includes recombinant polypeptide and chemically synthesized analogues or analogues biologically synthesized by heterologous systems. An "isolated polynucleotide" is similarly defined. The variants will include allelic variants. An "allelic variant" in the context of a nucleic acid or a gene is an alternative form (allele) of a gene that exists in more than one form in the population. At the polypeptide level, "allelic variants" generally differ from one another by only one, or at most, a few amino acid substitutions. There can also be synthetic or "unnatural" variants of a gene that are generated by recombinant biological techniques. Preferably, the amino acid residue positions which are not identical in the variant differ by conservative amino acid substitutions. "Conservative amino acid substitutions" refers to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine.

The AC_{VI} polynucleotides encoding variants can also comprise silent nucleotide subtitutions. By "silent" subtitution is meant that the substituted nucleotide does not result in an amino acid change at the protein level. Even more substantial nucleotide changes can be introduced into regions that do not affect the enzymatic activity of the encoded adenycyclase polypeptide. Many such polynucleotides will encode polypeptides that maintain adenycyclase enzymatic activity, which can be tested by routine methods as known in the art. "High stringency" conditions for polynucleotide hybridization are described, e.g., in J. Sambrook et al., supra, and typically refer to conditions in which the salt concentration and the temperature are increased such that only sequences having substantial overall sequence identity (typically in excess of 90%, more preferably in excess of 95%) over stretches of greater than about 100 nucleotides remain hybridized.

As will be appreciated by those of skill in the art, small fragments of the human AC_{VI} polynucleotide sequence (including fragments on the order of about 15-50 nucleotides) can be used as primers or probes to identify and isolate isoforms or variants of the native polypeptides.

Isolated polypeptides encoded by the polynucleotides of the preceding embodiments include, for example, polypeptides comprising a sequence in which at least about 300 amino acid residues is at least 95% (preferably greater than 99%) identical with a sequence of comparable length within Figure 12B.

### EXAMPLE 6: Construction of a Vector for Gene Delivery of a β-Adrenergic Receptor Protein (β₁-AR) as a Second Type of β-ASP

As described above, additional β-adrenergic signaling proteins for use in the present invention include β-adrenergic receptor proteins, particularly β₁-adrenergic receptors (β₁-AR), and GRK inhibitors (which can indirectly enhance β-AR activity as described above).

Gene delivery vectors comprising transgenes encoding such additional β-ASPs can be readily generated using techniques such as those described in the preceding example.

By way of illustration, we have constructed a recombinant replication-defective adenovirus vector expressing a human β-adrenergic receptor. As an exemplary β-AR, we used a full length cDNA encoding human β₁-AR (about 1.8 kb) as described and sequenced in Frielle, et al., PNAS (USA) 84: 7920-7942, 1987).

Briefly, the β₁-AR cDNA fragment (which had been cloned into the EcoRI site of pSP65) was inserted into an E1-deleted recombinant human adenovirus-5 vector using the techniques described in the preceding example, thereby generating a recombinant vector for the delivery of a gene encoding a second preferred β-ASP (i.e. a β-adrenergic receptor protein).

### EXAMPLE 7: Illustrative Construction of a Vector for Gene Delivery of a GRK Inhibitor as a Third Type of β-ASP

Yet another illustrative example of an additional β-adrenergic signaling protein for use in the present invention is a G-protein receptor kinase inhibitor (GRK inhibitor), which can be used to indirectly enhance β-AR activity and therefore β -adrenergic responsiveness. Gene delivery vectors comprising transgenes encoding such a β-ASP can be readily generated using techniques such as those described in the preceding examples.

Since the functional kinase domains of various GRK proteins have been identified (and corresponding domains in related GRK proteins can be identified by homology), and the mutation need only impair kinase functionality (which is testable using standard techniques), a variety of GRK inhibitors can be readily prepared. By way of illustration, a GRK inhibitor can be constructed as described for the "β-ARK1-rninigene" (Koch, et al., Science 268: 1350-1353, 1995), or an analogous construct (in which a GRK is mutated to effectively delete or impair kinase function without disrupting receptor binding activity) can be used.

Briefly, the DNA fragment encoding the GRK inhibitor can be inserted into an E1-deleted recombinant human adenovirus-5 vector using the techniques described in the preceding examples, or using another vector as known in the art.

### EXAMPLE 8: Rapid Screening of Vector Constructs for β-ASP Gene Transfer and Expression Using Neonatal Rat Ventricular Myocytes in Cell Culture

β-ASP gene transfer vectors can initially be tested by examining the ability of the vectors to deliver β-adrenergic signaling proteins to ventricular cells maintained in cell culture. Such cell culture studies can thus be useful in screening putative gene transfer vectors (having, e.g., particular combinations of β-ASP transgenes and promoters) for the ability to deliver expressible transgenes to cells of particular types, such as exemplified herein.

The first round of such screening can be conveniently accomplished using a standard detectable marker gene (such as lacZ) so that gene delivery and gene expression can be readily and rapidly quantified, as illustrated below.

Vectors that effectively deliver and cause expression of the first round "test" transgene (e.g. a detectable marker gene) can then be subjected to a second screening round using a β-ASP according to the present invention.

By way of illustration of such vector screening techniques, neonatal rat ventricular myocytes were prepared with a collagenase-containing perfusate according to standard methods. Rod-shaped cells were cultured on laminin-coated plates and at 48 hours were infected with a vector comprising a detectable marker gene (viz. an adenovirus vector comprising a lacZ gene, as described in the examples above) at a multiplicity of infection of 1:1 (plaque forming units: cell). After a further 36 hour period, the cells were fixed with glutaraldehyde and incubated with X-gal.

Examination of staining revealed that essentially all exposed cardiac myocytes expressed the product of the lacZ transgene (i.e. β-galactosidase) after infection with the recombinant adenovirus vector in cell culture.

### Example 8-1: Delivery of a β-ASP transgene to cardiac myocytes derived from mammalian ventricles

Experiments were then performed to examine the ability of the gene transfer vectors to deliver and express a β-adrenergic signaling protein in the ventricular myocytes. Forty-eight hours after culture, myocytes were infected with recombinant vectors, as in Example 5, comprising either a β-ASP gene (AC_{VI}) or a detectable marker gene (lacZ).

Twenty-four hours after gene transfer, cells were incubated with and without isoproterenol (10 µM) in the presence of ³H-forskolin, to assess the degree of forskolin binding as a measure of AC protein content. In additional studies, AC_{VI} mRNA content was assessed in Northern blots.

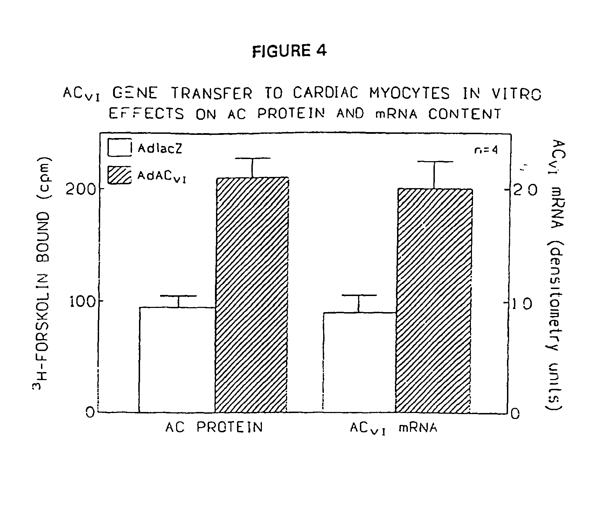

As shown in FIGURE 4, cells exposed to vectors carrying the β-ASP transgene AC_{VI} had a 2-fold increase in AC protein content and mRNA, confirming successful gene transfer and expression of AC_{VI} in cardiac myocytes *in vitro* using viral vectors.

Additional experiments were conducted on cultured neonatal rat ventricular myocytes to examine cAMP production in myocytes following delivery of the β-ASP transgene AC_{VI}. Forty-eight hours after culture, myocytes underwent infection with recombinant adenovirus expressing either AC_{VI} or lacZ. Twenty-four hours after gene transfer, cells were incubated with isoproterenol (10 µM) or forskolin (3 µM) and cAMP content was measured.

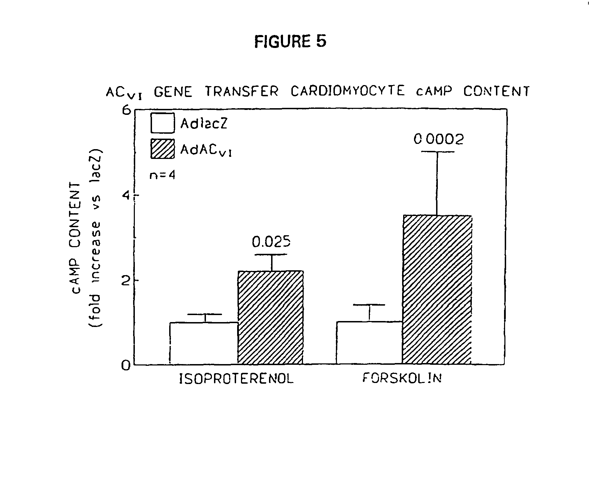

As shown in FIGURE 5, cells exposed to vectors carrying the β-ASP transgene AC_{VI} exhibited a 2-fold increase in isoproterenol-stimulated cAMP content, and an 3-fold increase in forskolin-stimulated cAMP content.

These rapid-screening techniques are also applicable to the testing of other vectors, as described herein, including other vectors (e.g. other viral vectors such as AAV as well as non-viral vectors including lipid-based vectors and various non-viral delivery platforms), vectors in which transgenes are linked to different transcriptional control sequences (such as ventricular-specific promoters), as well as vectors encoding other β-ASP transgenes, as described and illustrated herein.

### Example 8-2: Delivery of a truncated AC transgene to cardiac myocytes derived from mammalian ventricles

As an additional illustration, we delivered a truncated AC transgene (constructed as described in Example 5-2) to cardiac myocytes that had been derived from the ventricles of a mammalian heart, and assessed expression of the transgene and the encoded protein in the treated cardiac cells.

We also analyzed the physiological effects of transgene delivery to the ventricular myocytes (as measured by alterations in forskolin binding and cAMP production), and examined β-adrenergic receptor binding in the cells (using radioligand binding assays).

**Cardiac Myocyte Preparation and Gene Transfer**. Hearts from 1 to 2 day old Sprague-Dawley rats were removed, atria and great vessels discarded, and ventricles trisected. Myocardium was digested with collagenase II (Worthington) and pancreatin (GibcoBRL Life Technology, Gaithersburg, MD); and the myocardial cell suspension was centrifuged through Percoll step gradients to separate cardiac myocytes from other cells. Cells then were plated (4 x 10⁴ cells/cm²) in plates precoated with gelatin, and incubated for 24h. Cells were then washed with serum-free media and maintained in 2% fetal bovine serum for 24h (as described in Knowlton, KU et al, J Biol Chem 266: 7759-7768, 1991). Adenovirus-mediated gene transfer was performed 3d after initial isolation by adding recombinant adenovirus expressing AC_{VI} or lacZ (10 pfu/cell), and incubating for 20h in DMEM containing 2% fetal bovine serum. Adenovirus was removed and the cells were maintained for 24h and then used for study. The extent of gene transfer was evaluated by X-gal staining of cells after gene transfer with lacZ. Preliminary studies established that at virus titers of 1,10, and 100 pfu per cell, 95-100% of cardiac myocytes expressed lacZ with no evidence of cytotoxicity. We selected 10 pfu/cell for our studies. Protein content per plate was similar after gene transfer with lacZ and AC_{VI}.

**RT-PCR.** RT-PCR was used to identify AC_{VI} mRNA. The reverse transcription reaction was performed (SuperScript II, GibcoBRL Life Technology). Briefly, 1 µg of mRNA was mixed with 100 ng of the primer AC_{VI}3'pHA in 11 µl, heated (70°C, 10m) and quickly chilled on ice. Four microliters of 5 x first strain buffer, 2 µl of 0.1 M DTT, and 1 µl of 10 mM dNTP were added and the reaction mixture allowed to equilibrate (37°C, 2m). Finally, 1 µl (200 units) of SuperScript II Rnase H reverse transcriptase was added; reaction duration was 1 h (37°C).

**Northern Blot Analysis**. Total RNA was isolated from cardiac myocytes 48h after gene transfer using Trizon reagent (GIBCOBRL Life Technology). Twenty micrograms of denatured total RNA was electrophoresed in 1 x MOPS/EDTA buffer on a 1.0% agarose gel. The RNA was transferred to a nylon membrane in 20 x SCC solution. RNA was immobilized (80°C, 2h); and the membrane hybridized with randomly labeled ³²P-dCTP murine AC_{VI} cDNA probe or glyceraldehyde-3-phosphate dehydrogenase (GADPH). Hybridization was carried out in Hood buffer (50% formamide, 5 x SSC, 20 mM NaHPO4, pH6.7, 7% SDS, 1% PEG 15,000-20,000, and 0.5% non-fat milk) at 42°C for 16h. The membrane was washed once with 2 x SSC-0.5% SDS for 30m at RT, and 2-3 times with 0.1 x SSC-0.1% SDS for 30min. each (60-65°C) and exposed to X-ray film.

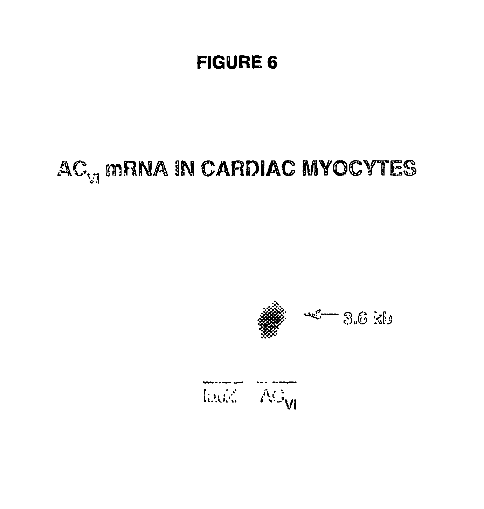

The resulting Northern blots showed a band compatible with AC_{VI} mRNA in RNA isolated from cardiac myocytes that had received AC_{VI} gene transfer (FIGURE 6). The low abundance endogenous rat AC_{VI} mRNA was detectable after prolonged exposure in cells that had received lacZ gene transfer. Equal RNA loading was documented by GADPH controls. These data document robust transgene AC_{VI} mRNA expression after gene transfer.

**Western Blot Analysis**. Cell lysates were prepared from virus infected myocytes by using NP-40 lysis buffer (20 mM Hepes pH7.0, 120 mM HCl, 1 mM DTT, 5 mM magnesium acetate, 10% glycerol, 0.5% NP-40, and proteinase inhibitors: 10 µg/ml each of leupeptin, aprotinin, and pepstatin, and 1 mg/ml of pefabloc) for 10m on ice. The samples were centrifuged in a microfuge at full speed for 15m at 4°C. The pellet was resuspended in 1 x SDS buffer. Samples were boiled (5m) and cell lysates loaded onto a 7.5% SDS-polyacrylamide gel (SDS-PAGE). Protein was electrophoretically transferred (1h, 100V, 4°C) to nitrocellulose membranes in Tris-glycine buffer (25 mM Tris-HCl pH 8.3, 150 mM glycine, and 10% methanol). The membranes were treated with blocking buffer consisting of 5% nonfat dry milk in Tris-saline buffer (0.9% NaCl and 10 mM Tris-HCl, pH 7.5). For detection of AC_{VI} protein, membranes were incubated with anti-ACV/VI antibody (Santa Cruz Biotechnology, Inc., Santa Cruz, CA) diluted 1:100 in blocking buffer for 1h (RT). For detection of Gsα and Giα₂, membranes were incubated with anti-Gsα or anti-Giα₂ antibodies as previously described (as in Ping et al., J. Clin. Invest. 95: 1271-1280, 1995). Primary antibodies were detected with goat anti-rabbit IgG horseradish peroxidase conjugal (GIBCOBRL Life Technology) in blocking buffer. The antigen then was visualized with chemiluminescent substrates A and B (Kirkegaard and Perry Laboratories, Gaithersburg, MD) and exposed to X-ray film.

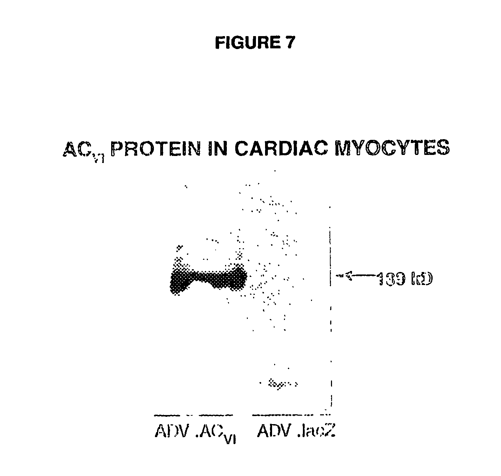

The resulting Western blots revealed that endogenous rat AC_{VI} protein could not be detected in cells after gene transfer with lacZ. However in cardiac myocytes that had received AC_{VI} gene transfer, transgene AC_{VI} protein was easily detectable as a band of appropriate electrophoretic mobility (FIGURE 7). These data document robust transgene AC_{VI} protein expression after gene transfer.

**Forskolin Binding.** [³H]Forskolin-binding assays were conducted using a modification of published methods (Post SR, Biochemical Journal 311: 75-80, 1995). Briefly, myocytes were infected with either adenovirus expressing AC_{VI} or lacZ, and cultured for 48h in 12-well culture plates. Prior to assay, culture medium was aspirated and the cells washed in reverse buffer (100 mM KCl, 20 mM NaCl, 1 mM NaH₂PO₄, 20 mM HEPES, and 1 mM MgSO₄, pH 7.4). Binding assays were initiated by the addition of saponin (20 µg/ml final), 20 nM [³H]forskolin, 1 µM 1,9-dideoxy-forskolin (to reduce association of radiolabel with non-adenylylcyclase molecules) and the additions indicated in the figure legends. Cells were incubated in a final volume of 0.5 ml for 15 min at 25°C. Reactions were terminated by aspiration of media and cells were washed twice with ice-cold washing buffer (50 mM Tris, 10 mM MgCl₂, pH 7.4). The amount of [³H]forskolin associated with cells was determined by extraction of cells in 0.2% Triton X-100 and scintillation counting of the soluble cell extract.

**Cyclic AMP Measurements**. Prior to treatment of cells, growth medium was removed and cells were equilibrated for 30m (RT) in serum- and sodium bicarbonate-free DMEM supplied with 20 mM HEPES pH 7.2. Subsequently, cells were incubated for 10m (RT) in fresh DMEM containing either 10 µM isoproterenol, or 10 µM forskolin in the presence of 0.1 mM ascorbic acid (to prevent oxidization) and 250 µM IBMX, a phosphodiesterase inhibitor. The reaction was terminated by aspiration of medium and addition of 7.5% ice-cold trichloroacetic acid (TCA). TCA extracts were frozen (-20°C) until assayed. Intracellular cAMP levels were determined by radioimmunoassay (Calbiochem, San Diego, CA) of TCA extracts following acetylation according to the protocol provided by the manufacturer. The sensitivity of this assay allowed for large dilution of TCA extracts such that ether extraction of TCA was unnecessary. Production of cAMP was normalized to the amount of acid-insoluble protein assayed by the BioRad protein assay.

**Statistical analysis**. For the forskolin binding and cAMP production studies, data are reported as mean values ± 1 SD. Data were compared using Student's t-test and, where appropriate, analyses of variance. The null hypothesis was rejected when p<0.05.

### Results of studies on forskolin binding and cAMP production.

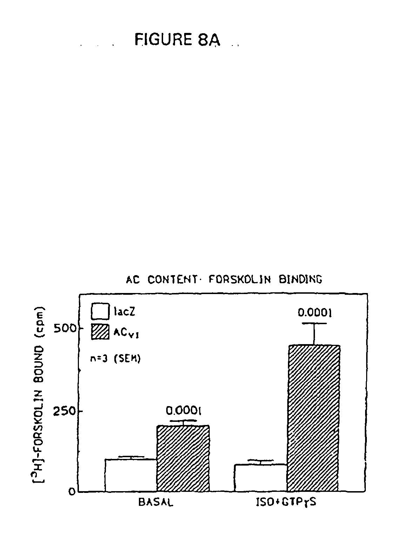

Forskolin binding studies provided a means to evaluate the amount of AC available for activation during hormonally-stimulated signaling. Cardiac myocytes that had received gene transfer on the same day, using the same clone, underwent parallel studies designed to measure forskolin binding as well as hormonally-stimulated cAMP production. The rationale for these studies was to obtain an accurate assessment of the relationship between transgene protein expression and cAMP production. Net GTPγS-stimulated forskolin binding was increased after AC_{VI} gene transfer (lacZ: 81±24 cpm; AC_{VI}: 447±113 cpm; p<0.0001). These are mean values from three experiments, documenting that transgene AC_{VI} is present and responsive to Gs:AC interaction (FIGURE 8A).

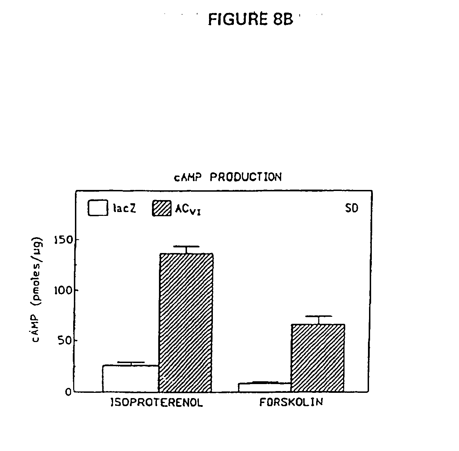

We then measured the responsiveness of cardiac myocytes overexpressing AC_{VI} to hormonal stimulation (FIGURE 8B). AC_{VI} gene transfer was associated with increased cAMP production when stimulated by isoproterenol (lacZ: 26±3 pmoles/µg; AC_{VI}: 136±7 pmoles/µg; p<0.0001) and by forskolin (lacZ: 9±2 pmoles/µg; AC_{VI}: 66±8 pmoles/µg; p<0.0001). These are mean values from three experiments, documenting that cardiac myocytes expressing transgene AC_{VI} have increased adrenergic responsiveness not only to forskolin stimulation, reflecting increased amounts of AC, but to isoproterenol, indicating that newly synthesized AC is functionally coupled and recruitable through βAR stimulation.

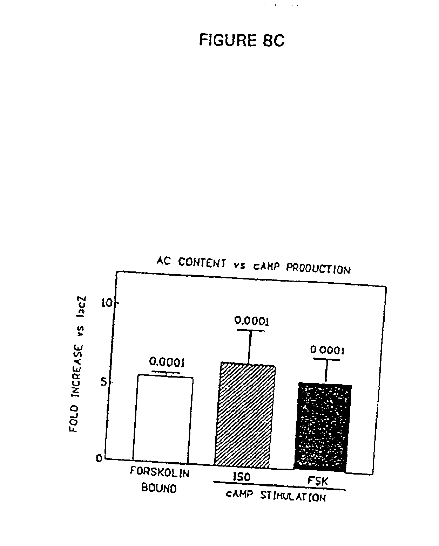

FIGURE 8C displays three measures of altered adrenergic signaling (forskolin binding, and isoproterenol- and forskolin-stimulated cAMP production). These data indicate that a proportional increase in AC content and enhanced adrenergic signaling have occurred.

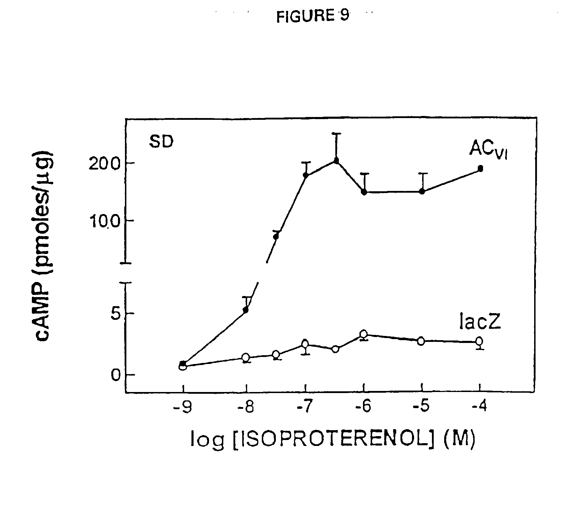

FIGURE 9 shows cAMP production when cardiac myocytes were stimulated by a range of isoproterenol concentrations. After gene transfer with AC_{VI} (vs lacZ), there was an obvious increase in cAMP produced through a wide range of isoproterenol concentrations. The EC50 for isoproterenol-stimulated cAMP production was unchanged (lacZ: 16±13 nM; AC_{VI}: 32±19 nM).

To determine if lacZ has a deleterious effect on cAMP production, we also studied non-transfected cardiac myocytes. These studies showed that untransfected cells were indistinguishable from lacZ infected cells in cAMP production.

**β-Adrenergic Receptor Binding Studies.** βARs were identified in radioligand binding experiments using [¹²⁵I]-iodocyanopindolol (ICYP; 30-240 pM); 10⁻⁴M isoproterenol was used to define nonspecific binding. Transfected cells (lacZ vs AC_{VI}) were lysed and membranes prepared for radioligand binding (as in Ping et al., J. Clin. Invest. 95: 1271-1280, 1995); experiments were performed with triplicate samples. Data are reported as specifically bound ICYP (fmol/mg).

To determine whether increased AC content affected βAR number, we performed radioligand binding assays. These assays identified similar amounts of specifically bound ICYP per mg membrane protein in plates of cardiac myocytes infected with adenovirus expressing lacZ (Bmax: 26 fmol/mg; Kd: 43 pM) or AC_{VI} (Bmax: 29 fmol/mg; Kd: 78 pM). These data indicate that βAR number was unchanged by gene transfer of AC_{VI}.

**Gsα and Giα**_{**2**} **Content**. To determine whether increased AC content affected G protein content, we performed immunoblotting studies with antibodies directed against Gsα and Giα₂. These assays identified similar amounts of Gsα and Giα₂. These data indicate that the content of Gsα and Giα₂ were not changed by gene transfer of AC_{VI},

### Summary of studies involving AC transgenes

The experiments described above demonstrate that AC transgenes (including variant transgenes in which untranslated regions have been altered) can be readily constructed and used to deliver AC gene products to cardiac cells, and further confirm that such transgenes can be used to alter the functional responsiveness of the cells.

Deletion of the 3'-untranslated region in the illustrative AC construct resulted in a substantially smaller transgene that was found to be even more effective in mediating transgene expression and functional response following delivery to the ventricular myocytes. Truncated constructs in which untranslated regions are removed from the transgene (e.g. AC transgenes from which the 3'-untranslated regions are removed) can thus provide alternative, highly functional constructs for use in the present invention. As compared to the "parental-type" construct in which the native 3'-untranslated region was maintained, the altered AC transgene resulted in an approximately 4-fold increase in forskolin-stimulated cAMP production, and an approximately 9-fold increase in isoproterenol-stimulated cAMP production. The ability to employ genes exhibiting differing levels of expression thus provides an added tool that can be used to optimize the present invention in the context of varying therapeutic needs (depending on the desired level of expression in the cells and tissue to be treated). Alternatively, by employing such high expression constructs, one can practice the present invention using correspondingly less vector to achieve an equivalent effect. Indeed, despite unchanged numbers of cell surface receptors in these experiments, we were able to amplify cAMP production through βAR stimulation by approximately 2-100 fold using AC transgenes as described and illustrated above.

Without wishing to be bound by theory, the observed increases in effective expression levels using the altered transgene may be due to an increase in vector packaging efficiency and/or an increase in message stability. In those regards, the substantially reduced size of the transgene construct may allow it to be packaged more efficiently in the viral vector used in these experiments. Our results also suggest that the resulting mRNA was more abundant in transfected cells (approximately 25-fold higher as compared to the parental-type construct), which may be the result of an increase in message stability. With respect to the latter, we have identified a potential mRNA destabilizing element (UUAUUUA(UA)(UA)) in the 3'-untranslated region of the original ACVI construct. Removal of such message destabilizing elements may thus enhance the effective expression of other β-ASP transgenes having such elements.

In addition, our observations that cells overexpressing AC_{VI} showed amplified responsiveness to βAR-mediated stimulation (versus forskolin alone) suggests that newly synthesized AC is functionally coupled and recruitable through stimulation by agonis that are normally present *in vivo.*

### EXAMPLE 9: Demonstration of In Vivo Gene Transfer to the Myocardium in a Porcine Heart Failure Model

In order to examine *in vivo* gene transfer in a large animal model that would be predictive of CHF in humans, we initially demonstrated delivery and expression of a detectable marker transgene to pig heart myocardium using the β-galactosidase-encoding vector generated as described in the examples above.

Briefly, an adenoviral vector was propagated in permissive human 293 cells and purified by CsCl gradient ultracentrifugation (with a final viral titer of 1.5 x 10¹⁰ viral particles), based on the procedures of Example 5.

An anesthetized, ventilated 40 kg pig underwent thoracotomy. A 26-gauge butterfly needle was inserted into the mid left anterior descending (LAD) coronary artery and the vector (1.5 x 10¹⁰ viral particles) was injected in a 2 ml volume. The chest was then closed and the animal allowed to recover. On the fourth day post-injection, the animal was sacrificed. The heart was fixed with glutaraldehyde, then sectioned and incubated with X-gal for 16.5 hours. After imbedding and sectioning, the tissue was counter-stained with eosin.

Microscopic analyses of tissue sections (transmural sections of the LAD bed)) revealed a significant magnitude of gene transfer in cells of the LAD coronary bed with many tissue sections demonstrating greater than 50-60% of the cells staining positively for β-galactosidase. Areas of the myocardium remote from the LAD circulatory bed did not demonstrate X-gal staining and served as a negative control, while diffuse expression of the gene was observed in myocytes and in endothelial cells.

In additional studies using closed-chest intracoronary injection, substantial activity was present 14 days after gene transfer (n=8).

Using these techniques, we thus demonstrated effective *in vivo* gene transfer and expression in a large mammal heart, with no evidence of inflammation or necrosis in areas of gene expression.

In view of our demonstration of the efficacy of this system, the *in vivo* procedure for monitoring delivery and expression of transgenes in the porcine heart failure model can also be readily employed to test other *in vivo* gene delivery vectors according to the present invention.

As described in Examples 10, 11 and 12 below, this *in vivo* procedure for monitoring delivery and expression of transgenes in the porcine heart failure model can also be readily employed to test other *in vivo* gene delivery vectors according to the present invention, including, for example, other *in vivo* delivery vectors (e.g. other viral vectors such as AAV as well as non-viral vectors including lipid-based vectors and various non-viral delivery platforms), vectors in which transgenes are linked to different transcriptional control sequences (such as ventricular-specific promoters), as well as vectors encoding other β-ASP transgenes, as described and illustrated herein.

As described in Example 13 below, we have also demonstrated that delivery of a β-ASP transgene to the myocardium, in accordance with the present invention, can be used to significantly enhance cardiac function in this large animal model of human heart failure.

### EXAMPLE 10: Illustrative In Vivo Gene Transfer to Pig Myocardium Using Other Viral Vectors (AAV)

Other vectors, including various viral vectors (such as adeno-associated virus (AAV)), liposomes and other lipid-containing gene delivery complexes, and other macromolecular complexes (such as multifunctional gene delivery fusion proteins) that are capable of mediating delivery of a polynucleotide to a mammalian host cell *in vivo* can be used to deliver AC transgenes to the myocardium in accordance with present invention. Thus, for example, AAV can be used to deliver one or more AC transgenes to the myocardium *in vivo.* The general principles of the preparation and use of adeno-associated viral vectors have been described in the art (see, e.g., Carter, B., Curr. Opin. Biotechnol., 3: 533-539, 1992; Kotin, R., Human Gene Therapy, 5: 793-801, 1994; and Flotte, T.R., et al., Gene Therapy 2:357-362, 1995; and the other references cited above).

By way of illustration, one or more AC transgenes (preferably comprising less than about 5 kb) as described herein is cloned into a recombinant AAV vector from which some (preferably all) of the AAV coding sequences (i.e. AAV *rep* and *cap* genes) have been deleted, but which retains at least the AAV inverted terminal repeats (ITRs). By placing the AC transgene between the AAV inverted terminal repeats and introducing the vector into a permissive packaging cell line capable of providing or modified to provide the missing AAV packaging functions (i.e. Rep and Cap proteins) *in trans.* The packaging cell line can then be used to replicate and encapsidate the recombinant AAV vector into infective (but replication-defective) AAV particles once the necessary AAV helper virus functions are provided, as described in the art. Alternatively, the recombinant AAV vector can be introduced prior to or coincident with the introduction of the helper virus or helper virus functions. As is known in the art, a variety of helper viruses (or genetic functions derived therefrom) can be used to provide helper activity to AAV, including adenoviruses, herpesviruses and poxviruses such as vaccinia. The most commonly used helper virus is Adenovirus. The deleted AAV packaging functions can be stably introduced into the genome of the packaging cell or they can be provided transiently (by, e.g., transfection with a helper plasmid or by inclusion within the helper virus, such as adenovirus). Recombinant AAV particles are then purified as described in the art (using, e.g., isopycnic ultracentrifugation).

As described in Examples 5-7 above, various β-ASP transgenes are generated for cloning into recombinant vectors, in this case AAV. With first-generation AAV vectors, the transgene or transgenes should comprise less than about 5 kb in order to be efficiently packaged. Of course, different β-ASP transgenes can also be placed into separate vectors.

Methods such as those illustrated in Example 8 can be used to select vector constructs mediating efficient delivery of β-adrenergic signaling proteins to ventricular cells maintained in cell culture; and, as illustrated in Example 9, a detectable marker gene (such as *lacZ*) can be used to select vector constructs mediating efficient delivery of transgenes to the myocardium *in vivo.* Suitable β-ASP vector constructs can then be used (as described in Examples 12-13 below) to deliver β-ASP transgenes to the myocardium in *vivo.*

### EXAMPLE 11: Illustrative In Vivo Gene Transfer to Pig Myocardium Using Non-Viral Vectors

The examples described herein using viral vectors as illustrative gene delivery vehicles can also be readily applied to the use of non-viral vectors, such as liposomes and other lipid-containing gene delivery complexes, and other macromolecular complexes (such as multifunctional gene delivery fusion proteins) that are capable of mediating delivery of a polynucleotide to a mammalian host cell *in vivo.*

By way of illustration, liposomes and other lipid-containing gene delivery complexes can be used to deliver one or more β-ASP transgenes. The principles of the preparation and use of such complexes for gene delivery have been described in the art (see, e.g., Ledley, FD, Human Gene Therapy 6: 1129-1144, 1995; Miller, N., et al., FASEB Journal 9: 190-199, 1995; Chonn, A., et al., Curr. Opin. in Biotech. 6: 698-708, 1995; Schofield, JP, et al., British Med. Bull. 51:56-71,1995; Brigham, K.L., et al., J. Liposome Res. 3: 31-49, 1993; and the other references cited above).

Briefly, one or more β-ASP transgenes as described herein is introduced into a liposome or other lipid-containing gene delivery complex using techniques known in the art. Various β-ASP transgenes can be generated as described in Examples 5-7, and then introduced into liposomes or other lipid-based vectors. Methods such as those illustrated in Example 8 can be used to select vectors mediating efficient delivery of β-adrenergic signaling proteins to ventricular cells maintained in cell culture; and, as illustrated in Example 9, a detectable marker gene (such as *lacZ*) can be used to select vectors mediating efficient delivery of transgenes to the myocardium *in vivo.* Suitable β-ASP vector constructs can then be used (as described in Examples 12-13 below) to deliver β-ASP transgenes to the myocardium *in vivo.*

### EXAMPLE 12: Effect of Gene Transfer of β-ASP Transgenes In vivo in Large Animal Models Predictive of Heart Failure in Humans

### Example 12-1: In Vivo Gene Transfer of an Adenylylcyclase β-ASP Transgene to Myocardium

In view of the foregoing observations, we examined the ability to enhance β-adrenergic responsiveness *in vivo* using gene therapy to deliver a β-ASP transgene to the myocardium of our large animal model.

Animals included 3 domestic pigs (weighing 30-40 kg). A left thoracotomy was performed under sterile conditions for instrumentation (as in Hammond, et al. J Clin Invest **92**:2644-2652, and Roth, et al. J Clin Invest **91**:939-949, 1993).

Catheters were placed in the left atrium and aorta, providing a means to calibrate the left ventricular high fidelity pressure gauge used to measure pressure development, and to monitor pressures. Wires were sutured on the left atrium to permit ECG recording and atrial pacing.

After recovery from surgery (10-14 days), pigs were examined to determine β-adrenergic responsiveness and baseline left ventricular dimension and hemodynamics. The most important element of these studies were heart rate responses to isoproterenol infusion. One of the pigs was also examined for left ventricular dP/dt measurements that were made before and after gene transfer, as described below.

The illustrative adenovirus vector system described above was used to deliver transgenes by *in vivo* gene delivery. As an exemplary β-ASP transgene, we used the AC_{VI} isoform referred to above. The vector material injected *in vivo* was highly purified and contained no wild-type (replication competent) adenovirus. Thus adenovirus infection and inflammatory infiltration in the heart were minimized. The vector preparation was injected into the lumen of the coronary artery by coronary catheters.

Introduction of the vector preparation (4.0 ml containing about 10¹¹ viral particles of adenovirus) was made by injecting 2.0 ml into both the left and right coronary arteries. Animals were anesthetized, and arterial access acquired via the right carotid by cut-down; a 5F Cordis sheath was placed. A 5F Multipurpose (A2) coronary catheter was used to engage the coronary arteries. The catheter tip was then placed 1 cm within the arterial lumen so that minimal material would be lost to the proximal aorta during injection. This procedure was carried out for each of the pigs.

Using these techniques, we have obtained very high efficiency gene delivery to the myocardium with no transgene expression observed in hepatocytes. Moreover, viral RNA could not be detected in the urine at any time after intracoronary injection.

As described in Example 13, such *in vivo* gene delivery of a β-ASP transgene to myocardium was found to substantially enhance cardiac function in our large mammal model.

### Example 12-2: In Vivo Gene Transfer of Other β-ASPs to Myocardium

Vectors comprising other β-ASPs, such as vectors encoding β-ARs or GRK inhibitors as described in Examples 6 and 7, can be used to deliver other preferred β-ASP transgenes using techniques as described in Example 12-1.

As described below, *in vivo* gene transfer of a first exemplary β-ASP transgene (AC) had a substantial and positive impact on cardiac function in our large animal model.

Delivery of other β-ASP transgenes can be used in place of, or in addition to, delivery of an AC transgene. Where a combination of β-ASP transgenes is supplied, the combination can be provided in a single vector (comprising two or more β-ASP transgenes) or in separate vectors (each comprising a β-ASP transgene). With size-constrained vectors such as adenovirus, an additional β-ASP transgene (such as a GRK inhibitor) can be accommodated in the vector by deleting an additional replication gene such as E4, as described above. Moreover, newer generations of such viral vectors are being used in which size-constraints are relieved in additional ways. In addition, a number of available non-viral vectors such as lipid-based vectors (including, e.g., cationic liposome complexes) do not exhibit such restrictive size constraints as observed with viral particle vectors.

Such additional β-ASPs can also be delivered using separate vectors. Where separate vectors are used, the vectors can be introduced together in a single injection (such as illustrated above) or in separate injections. While such separate vectors providing different transgenes are most conveniently analogous vectors (in which one β-ASP transgene is effectively replaced with another), different promoters can be employed as well as different base vectors.

The following example demonstrates the effect of *in vivo* β-ASP transgene delivery on cardiac function in our porcine model of heart failure.

### EXAMPLE 13: Increased Cardiac Function After In Vivo Gene Transfer to Large Animal Models Predictive of CHF in Humans

Pigs that had received an exemplary β-adrenergic signaling protein via in *vivo* gene therapy as described in Example 12-1 above were examined for cardiac function and other criteria, both before and after gene delivery.

Mismatch analyses confirmed that the β-ASP transgene (murine AC_{VI}) was present in the myocardium of animals that had received the gene transfer.

A number of indicators of cardiac function were measured before and after gene transfer. Briefly, conscious animals were suspended in a sling and pressures from the LV (n=1), LA and aorta were monitored, and electrocardiograms were recorded in digital format on-line (at rest and during atrial pacing at 150 bpm). Two-dimensional and M-mode images were obtained using a Hewlett Packard ultrasound imaging system. Images were obtained from a right parasternal approach at the mid-papillary muscle level and recorded on VHS tape. Images were recorded with animals in a basal state and again during right atrial pacing (HR=150 bpm). These studies were performed one day prior to gene transfer and were then repeated at 7 ± 3 days after transfer. Rate-pressure products and left atrial pressures were found to be similar before and after gene transfer, indicating similar myocardial oxygen demands and loading conditions. Echocardiographic measurements were made using standardized criteria (Sahn, et al. Circulation 58:1072,1978). The left ventricular end-diastolic diameter (EDD) and end-systolic diameter (ESD) were measured from 5 continuous beats and averaged.

Left ventricular fractional shortening (%FS) was also examined [(EDD-ESD)/EDD] X 100. This measure was unchanged by gene transfer, indicating no deleterious effects of the intervention, and demonstrating the safety of the procedure. To demonstrate reproducibility of echocardiographic measurements, animals (n=5) were imaged on two consecutive days, showing high correlation (r²=0.90; p=0.005).

After left ventricular dimensions had been measured and baseline hemodynamics recorded, glycopyrrolate (a muscarinic cholinergic antagonist) was given (at 0.14 mg/kg, by i.v.) in order to block the effects of the parasympathetic nervous system on heart rate responses. Isoproterenol was then delivered in bolus doses into the pulmonary artery catheter as heart rate responses were recorded. These studies were conducted one day prior to gene transfer and then again 5-10 days after gene transfer in each pig. Data were then examined by pair d analyses comparing each animal before and after gene transfer.

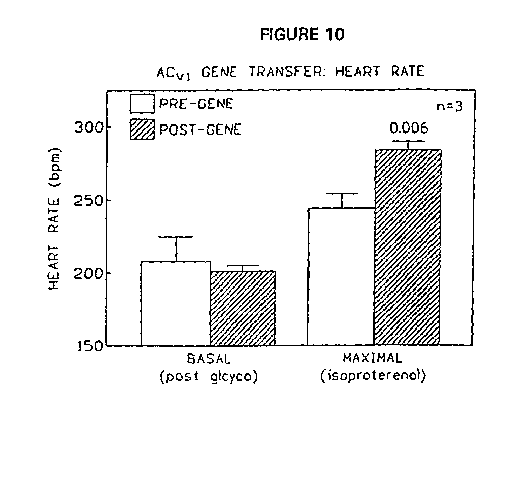

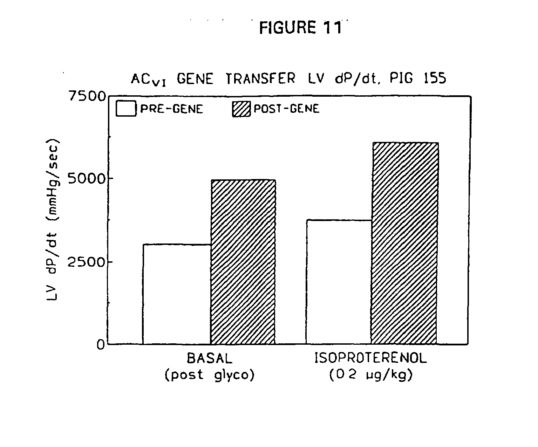

The results, as shown in FIGURES 10 and 11, demonstrated that *in vivo* gene delivery of even a single β-ASP transgene according to the present invention effectively increased β-adrenergic responsiveness in a large animal model heart that is predictive of humans.

FIGURE 10 shows data summarizing the effects of *in vivo* gene transfer of AC_{VI} on heart rate. Animals were studied before and 5-10 days after intracoronary delivery of 10¹¹ viral particles of an adenovirus expressing AC_{VI}. Glycopyrrolate was used to remove parasympathetic influences on heart rate, thereby optimally isolating the myocardial βAR pathway. Basal heart rate was unchanged, but maximal (isoproterenol-stimulated) heart rate was increased significantly by *in vivo* delivery of a β-ASP transgene (i.e. AC_{VI}) according to the present invention. These data demonstrate, for the first time, that *in vivo* gene transfer of a β-ASP transgene can effectively increase β-adrenergic responsiveness in a large mammal heart.

FIGURE 11 shows results of *in vivo* gene transfer of AC_{VI} on LV dP/dt in a normal pig. The animal was studied before and 7 days after intracoronary delivery of 10¹² viral particles of an adenovirus carrying the β-ASP transgene AC_{VI}. Glycopyrrolate was used to remove parasympathetic influences on contractile function, thereby optimally isolating the myocardial βAR pathway.

As shown in FIGURE 11, basal LV dP/dt was substantially increased at the same basal heart rate. Response to isoproterenol was also increased after AC_{VI} gene transfer. These data demonstrate that *in vivo* gene transfer of an illustrative β-ASP transgene can effectively increase contractile function of the intact heart in a large animal model expected to predictive of cardiac function in humans.

In summary, the foregoing studies demonstrated that the use of in *vivo* gene therapy to deliver even a single AC transgene according to the present invention effectively increased endogenous β-adrenergic responsiveness and cardiac function in a mammalian heart that has been observed to mimic cardiac function, and dysfunction, in humans.

As noted previously, AC gene constructs can also be modified to alter the effective levels of expression of the resulting transgene being employed in the context of the present invention. For example, as described above, expression of AC transgenes can be altered by placing the transgene under the control of a heterologous promoter (including, for example, various constitutive or inducible promoters, as well as tissue-specific promoters such as cardiac-specific promoters). As also described above, expression can be altered by changing other regions of the genes, including, for example, the untranslated regions of such genes. By way of illustration, AC_{VI} constructs having deletions in the 3'-untranslated region can be generated (as described in Example 5-3), and tested for their relative ability to affect expression and functional responsiveness in cardiac cells (as illustrated in Example 8-2). The ability to employ genes exhibiting differing levels of expression provides an added tool that can be used to readily tailor and optimize the present invention in the context of varying therapeutic needs (e.g., depending on the desired level of expression in the cells and tissue to be treated), and can also be used to reduce the amount of vector required to generate a given physiological effect. As further described above, it is possible to obtain human isoforms of AC transgenes using techniques such as those illustrated in Example 5-3. The illustrations presented in Examples 9-13 provide additional guidance as to means for testing and using such AC transgenes in the context of the present invention.

In addition, considering these demonstrated effects in conjunction with our observations and others' regarding the effective coupling of various components in the βAR-G₅-AC pathway, the ability to deliver β-ARs, GRK inhibitors or combinations of such β-ASPs (as described above) is expected to provide an even greater enhancement of endogenous β-adrenergic responsiveness and cardiac function in such dysfunctional mammalian hearts. Indeed our data described above support the idea that alterations in β-ARs and proteins affecting β-ARs (such as GRK) are important early contributors to the abnormalities in responsiveness to endogenous β-adrenergic agonists that are observed in association with CHF.

By providing means for effectively enhancing responsiveness to endogenous β-adrenergic agonists, the present invention thus provides greatly-needed alternatives to the use of exogenous pharmacological agonists and other treatments of congestive heart failure in humans.

## Claims

1. Use of a vector comprising a gene encoding an adenylylcyclase (AC) operably linked to a promoter, for the manufacture of a medicament for enhancing cardiac function in a mammal with congestive heart failure by delivering said vector to the heart of said mammal.

2. The use according to claim 1, wherein the vector is introduced into a blood vessel supplying blood to the myocardium of the heart.

3. The use according to claim 1, wherein the vector is delivered to cardiac myocytes.

4. The use according to claim 2, wherein said blood vessel supplying blood to the myocardium of the heart is a coronary artery, a saphenous vein graft or an internal mammary artery graft.

5. The use according to claim 4, wherein the vector is introduced into both the left and right coronary arteries.

6. The use according to any one of the preceding claims, wherein said mammal is a human.

7. The use according to any one of the preceding claims, wherein the vector further comprises a gene encoding a β-adrenergic signalling protein (β-ASP) different from said AC operably linked to a promoter.

8. The use according to any one of claims 1 to 6 wherein a second vector comprising a gene encoding a β-ASP operably linked to a promoter is introduced into said mammal, said β-ASP being different from said AC.

9. The use according to any one of the preceding claims, wherein the AC is AC isoform VI.

10. The use according to claim 9, wherein the AC is human AC isoform VI.

11. The use according to any one of the preceding claims, wherein the gene encoding the AC is operably linked to a heterologous promoter selected from a heterologous constitutive promoter and a heterologous inducible promoter.

12. The use according to claim 11, wherein the promoter is selected from a ventricular myosin light chain 2 promoter and a ventricular myosin heavy chain promoter.

13. The use according to any one of the preceding claims, wherein the gene encoding the AC is a wild-type AC gene comprising a deletion in one or more untranslated regions of said AC gene.

14. The use according to claim 13, wherein the deletion is in the 3'-untranslated region.

15. The use according to claim 13, wherein said deletion removes at least about 100 bp of the 3' -untranslated region.

16. The use according to claim 13, wherein the gene encoding the AC is a truncated AC_{VI} gene having a deletion removing the 3'-untranslated region.

17. The use according to any one of the preceding claims, wherein the or each vector is selected from a viral vector and a lipid-based vector.

18. The use according to any one of claims 1 to 16, wherein the or each vector is a viral particle.

19. The use according to claim 18, wherein the or each viral particle is an adenovirus (Ad) or an adeno-associated virus (AAV).

20. The use according to claim 19, wherein the viral particle is an adenovirus comprising a polynucleotide having a promoter operably linked to a gene encoding an AC, and said adenovirus vector is replication-defective in humans.

21. The use according to claim 20, wherein the AC is AC isoform VI.

22. An isolated polynucleotide comprising a sequence encoding a human adenylcyclase VI (AC_{VI}) polypeptide or a variant thereof having adenylcyclase activity, wherein the polynucleotide comprises:
(i) a sequence of at least 100 nucleotides that has at least 99% overall sequence identity with a nucleotide sequence of comparable length within the sequence shown in Figure 12A; or
(ii) a sequence of at least 1000 nucleotides having at least 95% overall sequence identity with a nucleotide sequence of comparable length within the sequence shown in Figure 12A.

23. An isolated polypeptide encoded by the polynucleotide of claim 22.

24. An isolated polypeptide of claim 23, wherein said polypeptide comprises a sequence of at least 300 amino acid residues that has at least 95% overall amino acid sequence identity with a sequence of comparable length within the sequence shown in Figure 12B.

25. An isolated polypeptide of claim 24, wherein said overall amino acid sequence identity is at least 99%.

26. A vector comprising a polynucleotide of claim 22.

27. A vector of claim 26, which is a viral vector or a lipid-based vector.

28. A vector of claim 26, which is a replication-defective viral vector selected from an adenoviral vector and an adeno-associated viral vector.

29. A recombinant replication-defective viral particle comprising a polynucleotide of claim 22 operably linked to a promoter.

30. The recombinant replication-defective viral particle of claim 29, wherein the viral particle is an adenoviral particle.

## Patentansprüche

1. Verwendung eines Vektors, der ein Gen umfasst, das für eine Adenylylcyclase (AC) codiert und das operativ mit einem Promotor verbunden ist, zur Herstellung eines Medikaments zur Verbesserung der Herzfunktion in einem Säugetier mit kongestiver Herzinsuffizienz durch Abgabe des Vektors an das Herz des Säugetiers.

2. Die Verwendung gemäß Anspruch 1, wobei der Vektor in ein Blutgefäß eingeführt wird, das Blut zum Herzmuskel leitet.

3. Die Verwendung gemäß Anspruch 1, wobei der Vektor an Herzmuskelzellen abgegeben wird.

4. Die Verwendung gemäß Anspruch 2, wobei das Blutgefäß, das Blut zum Herzmuskel leitet, eine Herzkranzarterie, ein Vena-Saphena-Transplantat oder ein Transplantat der inneren Brustarterie ist.

5. Die Verwendung gemäß Anspruch 4, wobei der Vektor sowohl in die linke als auch in die rechte Herzkranzarterie eingeführt wird.

6. Die Verwendung gemäß einem der vorherigen Ansprüche, wobei das Säugetier ein Mensch ist.

7. Die Verwendung gemäß einem der vorherigen Ansprüche, wobei der Vektor außerdem ein Gen umfasst, das für ein β-adrenerges Signalprotein (β-ASP) codiert, das verschieden ist von der AC, und das operativ mit einem Promotor verbunden ist.

8. Die Verwendung gemäß einem der Ansprüche 1 bis 6, wobei ein zweiter Vektor, der ein Gen umfasst, das für ein β-ASP codiert und das operativ mit einem Promotor verbunden ist, in das Säugetier eingeführt wird, wobei das β-ASP verschieden ist von der AC.

9. Die Verwendung gemäß einem der vorherigen Ansprüche, wobei die AC eine AC der Isoform VI ist.

10. Die Verwendung gemäß Anspruch 9, wobei die AC menschliche AC der Isoform VI ist.

11. Die Verwendung gemäß einem der vorherigen Ansprüche, wobei das Gen, das für die AC codiert, operativ mit einem heterologen Promotor verbunden ist, der ausgewählt ist aus einem heterologen konstitutiven Promotor und einem heterologen induzierbaren Promotor.

12. Die Verwendung gemäß Anspruch 11, wobei der Promotor ausgewählt ist aus einem ventrikulären Myosin-leichte Kette 2-Promotor und einem ventrikulären Myosin-schwere Kette-Promotor.

13. Die Verwendung gemäß einem der vorherigen Ansprüche, wobei das Gen, das für die AC codiert, ein Wildtyp-AC-Gen ist, das eine Deletion in einem oder mehreren nicht-translatierten Bereichen des AC-Gens aufweist.

14. Die Verwendung gemäß Anspruch 13, wobei die Deletion sich in dem 3'-nicht-translatierten Bereich befindet.

15. Die Verwendung gemäß Anspruch 13, wobei die Deletion mindestens etwa 100 bp aus dem 3'-nicht-translatierten Bereich entfernt.

16. Die Verwendung gemäß Anspruch 13, wobei das Gen, das für die AC codiert, ein verkürztes AC_{VI}-Gen ist, das eine Deletion aufweist, die den 3'-nicht-translatierten Bereich entfernt.

17. Die Verwendung gemäß einem der vorherigen Ansprüche, wobei der oder jeder Vektor ausgewählt ist aus einem viralen Vektor und einem auf Lipid basierenden Vektor.

18. Die Verwendung gemäß einem der Ansprüche 1 bis 16, wobei der oder jeder Vektor ein virales Partikel ist.

19. Die Verwendung gemäß Anspruch 18, wobei das oder jedes virale Partikel ein Adenovirus (Ad) oder ein Adeno-assozüertes Virus (AAV) ist.

20. Die Verwendung gemäß Anspruch 19, wobei das virale Partikel ein Adenovirus ist, das ein Polynucleotid umfasst, welches einen Promotor aufweist, der operativ mit einem Gen, das für eine AC codiert, verbunden ist, und dieser Adenovirus-Vektor in Menschen replikationsdefekt ist.

21. Die Verwendung gemäß Anspruch 20, wobei die AC eine AC der Isoform VI ist.

22. Ein isoliertes Polynucleotid, umfassend eine Sequenz, die für ein humanes Adenylcyclase VI (AC_{VI})-Polypeptid oder eine Variante dessen, die Adenylcyclase-Aktivität aufweist, codiert, wobei das Polynucleotid umfasst:
(i) eine Sequenz von mindestens 100 Nucleotiden, die mindestens 99% Gesamtsequenzidentität mit einer Nucleotidsequenz vergleichbarer Länge innerhalb der Sequenz aufweist, die in Figur 12A dargestellt ist; oder
(ii) eine Sequenz von mindestens 1000 Nucleotiden, die mindestens 95% Gesamtsequenzidentität mit einer Nucleotidsequenz verleichbarer Länge innerhalb der Sequenz aufweist, die in Figur 12A dargestellt ist.

23. Ein isoliertes Polypeptid, das von dem Polynucleotid des Anspruchs 22 codiert ist.

24. Ein isoliertes Polypeptid gemäß Anspruch 23, wobei das Polypeptid eine Sequenz von mindestens 300 Aminosäureresten umfasst, die mindestens 95% der Gesamtsequenzidentität der Aminosäuren mit einer Sequenz vergleichbarer Länge innerhalb der Sequenz aufweist, die in Figur 12B dargestellt ist.

25. Ein isoliertes Polypeptid gemäß Anspruch 24, wobei die Gesamtsequenzidentität der Aminosäuren mindestens 99% beträgt.

26. Ein Vektor, umfassend ein Polynucleotid gemäß Anspruch 22.

27. Ein Vektor gemäß Anspruch 26, der ein viraler Vektor oder ein auf Lipid basierender Vektor ist.

28. Ein Vektor gemäß Anspruch 26, der ein replikationsdefekter viraler Vektor ist, ausgewählt aus einem adenoviralen Vektor und einem adeno-assozüerten viralen Vektor.

29. Ein rekombinantes replikationsdefektes virales Partikel, umfassend ein Polynucleotid nach Anspruch 22, das operativ mit einem Promotor verbunden ist.

30. Das rekombinante replikationsdefekte virale Partikel nach Anspruch 29, wobei das virale Partikel ein Adenovirus-Partikel ist.

## Revendications

1. Emploi d'un vecteur qui comporte un gène codant une adénylate cyclase (AC), fonctionnellement lié à un promoteur, en vue de la fabrication d'un médicament conçu pour améliorer la fonction cardiaque, chez un mammifère présentant une insuffisance cardiaque, par livraison dudit vecteur dans le coeur dudit mammifère.

2. Emploi conforme à la revendication 1, dans lequel le vecteur est introduit dans un vaisseau sanguin qui amène le sang au myocarde.

3. Emploi conforme à la revendication 1, dans lequel le vecteur est livré à des myocytes cardiaques.

4. Emploi conforme à la revendication 2, dans lequel ledit vaisseau sanguin qui amène le sang au myocarde est une artère coronaire, un greffon de veine saphène ou un greffon d'artère thoracique interne.

5. Emploi conforme à la revendication 4, dans lequel le vecteur est introduit dans les deux artères coronaires, la droite et la gauche.

6. Emploi conforme à l'une des revendications précédentes, ledit mammifère étant un être humain.

7. Emploi conforme à l'une des revendications précédentes, dans lequel le vecteur comporte en outre un gène codant une protéine de signalisation des récepteurs β-adrénergiques (β-ASP), différent dudit gène d'AC fonctionnellement lié à un promoteur.

8. Emploi conforme à l'une des revendications 1 à 6, dans lequel on introduit dans ledit mammifère un deuxième vecteur qui comporte un gène codant une protéine de signalisation des récepteurs β-adrénergiques (β-ASP), laquelle β-ASP est différente de ladite AC.

9. Emploi conforme à l'une des revendications précédentes, dans lequel l'adénylate cyclase est l'isoforme VI d'adénylate cyclase.

10. Emploi conforme à la revendication 9, dans lequel l'adénylate cyclase est l'isoforme VI d'adénylate cyclase humaine.

11. Emploi conforme à l'une des revendications précédentes, dans lequel le gène codant l'AC est fonctionnellement lié à un promoteur hétérologue choisi parmi un promoteur hétérologue constitutif et un promoteur hétérologue inductible.

12. Emploi conforme à la revendication 11, dans lequel le promoteur est choisi parmi un promoteur de chaîne légère 2 de myosine ventriculaire et un promoteur de chaîne lourde de myosine ventriculaire.

13. Emploi conforme à l'une des revendications précédentes, dans lequel le gène codant l'AC est un gène d'AC de type sauvage portant une délétion dans une ou plusieurs de ses régions non-traduites.

14. Emploi conforme à la revendication 13, dans lequel la délétion se situe dans la région 3' non-traduite.

15. Emploi conforme à la revendication 13, dans lequel ladite délétion a supprimé au moins à peu près 100 paires de bases de la région 3' non-traduite.

16. Emploi conforme à la revendication 13, dans lequel le gène codant l'AC est un gène d'AC_{VI} tronqué, portant une délétion qui a supprimé la région 3' non-traduite.

17. Emploi conforme à l'une des revendications précédentes, dans lequel le vecteur ou chacun des vecteurs est choisi parmi un vecteur viral et un vecteur à base de lipides.

18. Emploi conforme à l'une des revendications 1 à 16, dans lequel le vecteur ou chacun des vecteurs est une particule virale.

19. Emploi conforme à la revendication 18, dans lequel la particule virale ou chacune des particules virales est un adénovirus (Ad) ou un virus adéno-associé (AAV).

20. Emploi conforme à la revendication 19, dans lequel la paricule virale est un adénovirus comprenant un polynucléotide qui comporte un promoteur fonctionnellement lié à un gène codant une AC, et ledit vecteur adénoviral est déficient pour la réplication chez les humains.

21. Emploi conforme à la revendication 20, dans lequel l'AC est l'isoforme VI de l'AC.

22. Polynucléotide isolé, comprenant une séquence codant un polypeptide qui est une adénylate cyclase VI (AC_{VI}) humaine, ou un variant de celle-ci qui possède une activité d'adénylate cyclase, lequel polynucléotide comporte :
i) une séquence d'au moins 100 nucléotides qui est identique, pour au moins 99 % de la séquence globale, à une séquence de nucléotides de longueur comparable au sein de la séquence présentée dans la Figure 12A ;
ii) ou une séquence d'au moins 1000 nucléotides qui est identique, pour au moins 95 % de la séquence globale, à une séquence de nucléotides de longueur comparable au sein de la séquence présentée dans la Figure 12A.

23. Polypeptide isolé, codé par un polynucléotide conforme à la revendication 22.

24. Polypeptide isolé conforme à la revendication 23, lequel polypeptide comporte une séquence d'au moins 300 résidus d'acides aminés qui est identique, pour au moins 95 % de la séquence globale d'acides aminés, à une séquence de longueur comparable au sein de la séquence présentée dans la Figure 12B.

25. Polypeptide isolé conforme à la revendication 24, dans lequel ladite relation d'identité concerne au moins 99 % de la séquence globale d'acides aminés.

26. Vecteur comprenant un polynucléotide conforme à la revendication 22.

27. Vecteur conforme à la revendication 26, qui est un vecteur viral ou un vecteur à base de lipides.

28. Vecteur conforme à la revendication 26, qui est un vecteur viral déficient pour la réplication, choisi parmi un vecteur adénoviral ou un vecteur viral adéno-associé.

29. Particule de virus recombiné, déficient pour la réplication, comprenant un polynucléotide conforme à la revendication 22, fonctionnellement lié à un promoteur.

30. Particule de virus recombiné, déficient pour la réplication, conforme à la revendication 29, laquelle particule virale est une particule d'un adénovirus.
